# EUROPEAN PATENT APPLICATION

(11) **EP 4 697 024 A2**
(43) Date of publication of application: **18.02.2026**
(21) Application number: 25208932.1
(22) Date of filing: 05.09.2023
(51) Int. Cl.: G01N 33/68

(54) **DIAGNOSTIC AND PROGNOSTIC BIOMARKER PROFILES IN PATIENTS WITH HEMATOPOIETIC STEM CELL TRANSPLANT-ASSOCIATED THROMBOTIC MICROANGIOPATHY (HSCT-TMA)**

(30) Priority: 06.09.2022 US 202263403942 P; 28.10.2022 US 202263420198 P; 25.01.2023 US 202363440969 P
(62) Divisional of application: 23782345.5
(71) Applicant: ALEXION PHARMACEUTICALS, INC., Boston, MA 02210 (US)
(72) Inventor: COFIELL, Roxanne, Boston 02210 (US); KIM, Sung-Kwon, Boston 02210 (US)
(74) Representative: J A Kemp LLP

(57) **Abstract**

Provided are methods of treating hematopoietic stem cell transplant-associated thrombotic microangiopathy (HSCT-TMA), *e.g*., TMA after HSCT, in human patients having elevated levels of thrombomodulin (TM) and syndecan-1 (SYND1) compared to normal reference ranges of TM and SYND1, using an anti-C5 antibody and/or an anti-CFB antibody . Further provided are methods of treating a patient having HSCT-TMA comprising: (1) obtaining or having obtained a sample (*e.g.*, blood or plasma sample) from the patient, (2) determining or having determined elevated TM and SYND1 levels in the sample compared to normal reference ranges of TM and SYNDI, respectively, and (3) administering to the patient an anti-C5 antibody or an anti-CFB antibody in an amount and with a frequency sufficient to treat HSCT-TMA after elevated TM and SYND1 levels have been determined. In one embodiment, the methods further comprise determining or having determined elevated Ba levels in the sample compared to a normal reference range for Ba. In another embodiment, the methods further comprise determining or having determined an elevated HSPG level in the sample compared to a normal reference range for HSPG.

Further provided are methods of identifying a patient having HSCT-TMA that is suitable for treatment with an anti-C5 antibody or an anti-CFB antibody, as well as methods for monitoring responsiveness of a patient having HSCT-TMA to treatment with an anti-C5 antibody or an anti-CFB antibody.

## Description

### CROSS-REFERENCE TO RELATED APPLICATIONS

This application claims priority to, and the benefit of, U.S. Provisional Application No. 63/403,942 (filed September 6, 2022), U.S. Provisional Application No. 63/420,198 (filed October 28, 2022) and U.S. Provisional Application No. 63/440,969 (filed January 25, 2023). The entire contents of the aforementioned applications is incorporated herein by reference.

### BACKGROUND

Hematopoietic stem cell transplant-associated thrombotic microangiopathy (HSCT-TMA) is a multifactorial disorder caused by systemic vascular endothelial injury that can be triggered by several mechanisms during the transplant process. Patients with HSCT-TMA develop kidney damage, serositis, pulmonary hypertension, and multisystem organ failure. Severe HSCT-TMA is associated with long-term morbidity and mortality rates of approximately 80%. Studies have shown that the large majority of patients die within 6 months (Cho et al., Bone Marrow Transplant. 2008;41(9):813-820; Cho et al., Transplantation. 2010;90(8):918-926; Oran, 2007). Another study showed 9% overall survival of patients with severe HSCT-TMA who did not receive TMA targeted therapy, with all mortality occurring within 10 months of the TMA diagnosis (Jodele, et al., Blood. 2014b;124(4):645-653).

In pediatric patients, HSCT-TMA typically occurs early post allogeneic HSCT, with a median diagnosis at 35 to 47 days post-HSCT, and 88% to 92% occurring before Day +100. However, cases have been reported up to 2 years post-HSCT. Autologous recipients may develop HSCT TMA even earlier, with a median of 18 days post-HSCT (Dvorak et al., Front Pediatr. 2019;7:133).

Endothelial injury is fundamental to the pathogenesis of HSCT-TMA, with dysregulated complement activation likely to be a consequence of the endothelial damage. Risk factors associated with HSCT-TMA development that also initiate endothelial damage include calcineurin inhibitors (CNIs), infections, and conditioning regimens (high dose chemotherapy or total body irradiation) (Khosla, et al., Bone Marrow Transplant. 2018;53(2):129-137; Masias, et al., Blood. 2017;129(21):2857-2863).

Currently, there are no approved therapies for the treatment of HSCT-TMA and the identification of at-risk patients is challenging, leading to delays in diagnosis and dire outcomes. Accordingly, it is an object of the present invention to identify biomarkers associated with HSCT-TMA and provide improved methods for treating and identifying patients (in particular, pediatric patients) with HSCT-TMA.

### SUMMARY

Approximately 10-20% of HSCT patients develop HSCT-TMA. These patients are difficult to identify and have dire outcomes (e.g., transplant rejection and even death). However, by way of the present invention, it has been discovered that patients afflicted with HSCT who have abnormal levels of TM and SYNDI-1, as well as factor Ba and/or heparan sulfate proteoglycan (HSPG), are likely to develop HSCT-TMA. It was further observed that, following administration of an anti-C5 antibody (e.g., eculizumab or ravulizumab) or a complement factor B inhibitor (e.g., an inhibitor of the formation or activity of factor Ba, referred to herein as a "CFB inhibitor"), the concentrations of these proteins changed *(e.g.,* reduced and/or normalized).

Accordingly, in one aspect, the disclosure provides a method for treating a patient *(e.g.,* a pediatric or adult patient) having hematopoietic stem cell transplant-associated thrombotic microangiopathy (HSCT-TMA) who has been determined to have elevated levels *(e.g.,* blood or plasma levels) of a biomarker selected from thrombomodulin (TM) and syndecan-1 (SYND1), or a combination thereof, compared to normal reference ranges of the biomarkers, the method comprising administering to the patient an anti-C5 antibody or an anti-CFB antibody in an amount and with a frequency sufficient to attenuate the biomarker levels in the patient, thereby treating HSCT-TMA. In one embodiment, the patient has further been determined to have an elevated level (e.g., blood or plasma level) of Ba and/or C5b9 compared to a normal reference range for complement factor Ba and/or C5b9. In another embodiment, the patient has been determined to have an elevated level (e.g., blood or plasma level) of HSPG compared to a normal reference range for HSPG.

In another aspect, the disclosure provides a method for treating a patient having HSCT-TMA comprising: (1) obtaining or having obtained a sample *(e.g.,* blood or plasma sample) from the patient, (2) determining or having determined elevated levels of biomarker selected from TM and SYND1 or a combination thereof levels in the sample of the patient compared to normal reference ranges of the biomarkers, and (3) administering to the patient an anti-C5 antibody or an anti-CFB antibody in an amount and with a frequency sufficient to attenuate elevated TM and SYND1 levels in the patient, thereby treating HSCT-TMA. In one embodiment, the method further comprises determining or having determined elevated Ba and/or C5b9 levels in the sample compared to a normal reference range for Ba and/or C5b9. In another embodiment, the method comprises determining or having determined elevated an HSPG level compared to a normal reference range for HSPG.

In another aspect, the disclosure provides a method of identifying a patient having HSCT-TMA that is suitable for treatment with an anti-C5 antibody or an anti-CFB antibody , comprising determining levels of a biomarker selected from TM and SYND1 or a combination thereof in a sample *(e.g.,* blood or plasma sample) from the patient using an *in vitro* assay, wherein elevated levels of the biomarker in the sample compared to normal reference ranges of TM and SYND1, respectively, identifies the patient as being suitable for treatment with an anti-C5 antibody or an anti-CFB antibody . In one embodiment, the method further comprises determining Ba and/or C5b9 levels in the sample, wherein an elevated level of Ba and/or C5b9 compared to a normal reference range for Ba and/or C5b9 identifies the patient as being suitable for treatment with an anti-C5 antibody or an anti-CFB antibody.. In another embodiment, the method comprises determining an HSPG level in the sample, wherein an elevated level of HSPG compared to a normal reference range for HSPG identifies the patient as being suitable for treatment with an anti-C5 antibody or an anti-CFB antibody.In another aspect, the disclosure provides a method for monitoring responsiveness of a patient having HSCT-TMA to treatment with an anti-C5 antibody or an anti-CFB antibody, the method comprising: determining a biomarker selected from TM and SYND1 or a combination thereof in a sample (e.g., blood or plasma sample) from the patient obtained during or after treatment, wherein: decreased biomarker levels in the sample from the patient obtained during or after treatment, as compared to the biomarker levels in a sample from the patient obtained prior to treatment with the anti-C5 antibody or an anti-CFB antibody, indicates that the patient is responsive to treatment with the anti-C5 antibody or an anti-CFB antibody.

In one embodiment, the method further comprises determining Ba and/or C5b9 levels in a sample (e.g., blood or plasma sample) from the patient obtained during or after treatment, wherein: decreased Ba and/or C5b9 levels in the sample from the patient obtained during or after treatment, as compared to the Ba and/or C5b9 levels in a sample from the patient obtained prior to treatment with the anti-C5 antibody or an anti-CFB antibody, indicates that the patient is responsive to treatment with the anti-C5 antibody or an anti-CFB antibody. In another embodiment, the method further comprises determining an HSPG level in a sample *(e.g.,* blood or plasma sample) from the patient obtained during or after treatment, wherein: decreased a decreased HSPG level in the sample from the patient obtained during or after treatment, as compared to the HSPG level in a sample from the patient obtained prior to treatment with the anti-C5 antibody or an anti-CFB antibody, indicates that the patient is responsive to treatment with the anti-C5 antibody or an anti-CFB antibody.

The levels or levels of the biomarkers described herein (e.g., TM, SYND1, /or Ba, and/or HSPG) can be measured by any suitable means or art-recognized technique. In one embodiment, the level or levels are measured by a system or kit that has received regulatory (e.g., USFDA) approval. In another embodiment, the level or levels are measured by using an immunoassay, immunochemistry, immunohistochemistry assay, nucleoprobe assay, in situ hybridization, fluorescent RNA probes, RT-PCR, microarray transcription assay, or RNA transcription assay. In another embodiment, the level or levels are measured by enzyme-linked immunoassay (ELISA).

Levels of the biomarkers described herein (e.g., TM, SYND1, Ba, and/or HSPG) can be measured against a control value. Controls include negative controls and positive controls. In some embodiments, a negative control may include normal reference ranges for each respective biomarker to assess whether the level in an experimental sample *(e.g.,* a sample obtained from a patient who is undergoing or likely to undergo HSCT-TMA) is elevated or decreased comparatively. In one embodiment, the normal reference range for the biomarker is based on a healthy patient *(e.g.,* a patient who does not have HSCT). In another embodiment, the normal reference range for the biomarker is based on a HSCT patient in the absence of TMA.

In some embodiments, a positive control may include reference ranges for each respective biomarkers which permit assessment of whether the level of the biomarker in an experimental sample *(e.g.,* a sample from a patient who is undergoing treatment for HSCT-TMA) is reduced or attenuated comparatively, e.g., before treatment versus after treatment.

In one embodiment, the normal reference range for TM for a healthy patient (e.g., a patient who does not have HSCT) is about 1.8 ng/mL to about 4.8 ng/mL. For example, in one embodiment, a normal TM level for a healthy patient is about 1.8 ng/mL, 1.9 ng/mL, 2.0 ng/mL, 2.1 ng/mL, 2.2 ng/mL, 2.3 ng/mL, 2.4 ng/mL, 2.5 ng/mL, 2.6 ng/mL, 2.7 ng/mL, 2.8 ng/mL, 2.9 ng/mL, 3.0 ng/mL, 3.1 ng/mL, 3.2 ng/mL, 3.3 ng/mL, 3.4 ng/mL, 3.5 ng/mL, 3.6 ng/mL, 3.7 ng/mL, 3.8 ng/mL, 3.9 ng/mL, 4.0 ng/mL, 4.1 ng/mL, 4.2 ng/mL, 4.3 ng/mL, 4.4 ng/mL, 4.5 ng/mL, 4.6 ng/mL, 4.7 ng/mL, or 4.8 ng/mL.

In another embodiment, the normal reference range for TM for a HSCT patient in the absence of TMA is about 3 ng/mL to about 9 ng/mL. For example, in one embodiment, a normal reference range for TM for a HSCT patient in the absence of TMA is about 3 ng/mL, 4 ng/mL, 5 ng/mL, 6 ng/mL, 7 ng/mL, 8 ng/mL, or 9 ng/mL.

In another embodiment, levels of TM are elevated in HSCT patients who have or are likely to develop HSCT-TMA compared to a normal reference range *(e.g.,* a healthy patient who does not have HSCT or an HSCT patient in the absence of TMA). For example, an elevated TM level is greater than about 10 ng/mL, 11 ng/mL, 12 ng/mL, 13 ng/mL, 14 ng/mL, 15 ng/mL, 16 ng/mL, 17 ng/mL, 18 ng/mL, 19 ng/mL, 20 ng/mL, 21 ng/mL, 22 ng/mL, 23 ng/mL, 24 ng/mL, 25 ng/mL, 26 ng/mL, 27 ng/mL, 28 ng/mL, 29 ng/mL, or 30 ng/mL.

In another embodiment, the level of TM in the sample is deemed elevated when it is at least about 10% higher to about 10-fold, *e.g.,* about two-fold greater than the normal reference range for TM *(e.g.,* a healthy patient who does not have HSCT or an HSCT patient in the absence of TMA). In some embodiments, the level of TM in the sample is deemed elevated when it is at least three, four, five, or six-fold greater than the normal reference range for TM.

In one embodiment, the normal reference range for SYND1 for a healthy patient (e.g., a patient who does not have HSCT) is between 15 ng/mL to 70 ng/mL. For example, in one embodiment, a normal SYND1 level for a healthy patient is about 15 ng/mL, 16 ng/mL, 17 ng/mL, 18 ng/mL, 19 ng/mL, 20 ng/mL, 21ng/mL, 22 ng/mL, 23 ng/mL, 24 ng/mL, 25 ng/mL, 26 ng/mL, 27 ng/mL, 28 ng/mL, 29 ng/mL, 30 ng/mL, 31ng/mL, 32 ng/mL, 33 ng/mL, 34 ng/mL, 35 ng/mL, 36 ng/mL, 37 ng/mL, 38 ng/mL, 39 ng/mL, 40 ng/mL, 41ng/mL, 42 ng/mL, 43 ng/mL, 44 ng/mL, 45 ng/mL, 46 ng/mL, 47 ng/mL, 48 ng/mL, 49 ng/mL, 50 ng/mL, 51 ng/mL, 52 ng/mL, 53 ng/mL, 54 ng/mL, 55 ng/mL, 56 ng/mL, 57 ng/mL, 58 ng/mL, 59 ng/mL, 60 ng/mL, 61 ng/mL, 62 ng/mL, 63 ng/mL, 64 ng/mL, 65 ng/mL, 66 ng/mL, 67 ng/mL, 68 ng/mL, 69 ng/mL, or 70 ng/mL.

In another embodiment, the normal reference range for SYND1 for a HSCT patient in the absence of TMA is about 15 ng/mL to 55 ng/mL. For example, in one embodiment, a normal reference range for SYND1 for a HSCT patient in the absence of TMA is about 15 ng/mL, 16 ng/mL, 17 ng/mL, 18 ng/mL, 19 ng/mL, 20 ng/mL, 21ng/mL, 22 ng/mL, 23 ng/mL, 24 ng/mL, 25 ng/mL, 26 ng/mL, 27 ng/mL, 28 ng/mL, 29 ng/mL, 30 ng/mL, 31ng/mL, 32 ng/mL, 33 ng/mL, 34 ng/mL, 35 ng/mL, 36 ng/mL, 37 ng/mL, 38 ng/mL, 39 ng/mL, 40 ng/mL, 41ng/mL, 42 ng/mL, 43 ng/mL, 44 ng/mL, 45 ng/mL, 46 ng/mL, 47 ng/mL, 48 ng/mL, 49 ng/mL, 50 ng/mL, 51ng/mL, 52 ng/mL, 53 ng/mL, 54 ng/mL, or 55 ng/mL.

In another embodiment, levels of SYND1 are elevated in HSCT patients who have or are likely to develop HSCT-TMA compared to a normal reference range *(e.g.,* a healthy patient who does not have HSCT or an HSCT patient in the absence of TMA). For example, an elevated SYND1 level is greater than greater than about 100 ng/mL, 105 ng/mL, 110 ng/mL, 115 ng/mL, 120 ng/mL, 125 ng/mL, 130 ng/mL, 135 ng/mL, 140 ng/mL, 145 ng/mL, 150 ng/mL, 155 ng/mL, 160 ng/mL, 165 ng/mL, 170 ng/mL, 175 ng/mL, 180 ng/mL, 185 ng/mL, 190 ng/mL, 195 ng/ml, 200 mg/mL, 205 ng/mL, 210 ng/mL, 215 ng/mL, 220 ng/mL, 225 ng/mL, 230 ng/mL, 235 ng/mL, 240 ng/mL, 245 ng/mL, or 250 ng/mL.

In another embodiment, the level of SYND1 in the sample is deemed elevated when it is at least about 2-fold to about 17-fold, e.g., about four-fold greater than the normal reference range for SYND1 *(e.g.,* a healthy patient who does not have HSCT or an HSCT patient in the absence of TMA). In some embodiments, the level of SYND1 in the sample is deemed elevated when it is at least three, four, five, six, seven, eight, ten, twelve, or fifteen-fold greater than the normal reference range for SYND1.

In one embodiment, the normal reference range for Factor Ba for a healthy patient *(e.g.,* a patient who does not have HSCT) is less than about 1000 ng/mL. In another embodiment, the normal reference range for Factor Ba for a healthy patient (e.g., a patient who does not have HSCT) is less than about 600 ng/mL. In another embodiment, the normal reference range for Factor Ba for a healthy patient (e.g., a patient who does not have HSCT) is between about 300 ng/mL and 600 ng/mL. For example, the normal reference range for Factor Ba for a healthy patient *(e.g.,* a patient who does not have HSCT) is about 300 ng/mL, 310 ng/mL, 320 ng/mL, 330 ng/mL, 340 ng/mL, 350 ng/mL, 360 ng/mL, 370 ng/mL, 380 ng/mL, 390 ng/mL, 400 ng/mL, 410 ng/mL, 420 ng/mL, 430 ng/mL, 440 ng/mL, 450 ng/mL, 460 ng/mL, 470 ng/mL, 480 ng/mL, 490 ng/mL, 500 ng/mL, 510 ng/mL, 520 ng/mL, 530 ng/mL, 540 ng/mL, 550 ng/mL, 560 ng/mL, 570 ng/mL, 580 ng/mL, 590 ng/mL, or 600 ng/mL.

In another embodiment, the normal reference range for Factor Ba for a HSCT patient in the absence of TMA is about 500 ng/mL to 800. For example, in one embodiment, a normal reference range for Factor Ba for a HSCT patient in the absence of TMA is about 500 ng/mL, 510 ng/mL, 520 ng/mL, 530 ng/mL, 540 ng/mL, 550 ng/mL, 560 ng/mL, 570 ng/mL, 580 ng/mL, 590 ng/mL, 600 ng/mL, 610 ng/mL, 620 ng/mL, 630 ng/mL, 640 ng/mL, 650 ng/mL, 660 ng/mL, 670 ng/mL, 680 ng/mL, 690 ng/mL, 700 ng/mL, 710 ng/mL, 720 ng/mL, 730 ng/mL, 740 ng/mL, 750 ng/mL, 760 ng/mL, 770 ng/mL, 780 ng/mL, 790 ng/mL, or 800 ng/mL.

In another embodiment, levels of Factor Ba are elevated in HSCT patients who have or are likely to develop HSCT-TMA compared to a normal reference range *(e.g.,* a healthy patient who does not have HSCT or an HSCT patient in the absence of TMA). For example, an elevated Ba level is greater than about 900 ng/mL, 910 ng/mL, 920 ng/mL, 930 ng/mL, 940 ng/mL, 950 ng/mL, 960 ng/mL, 970 ng/mL, 980 ng/mL, 990 ng/mL, 1000 ng/mL, 1010 ng/mL, 1020 ng/mL, 1030 ng/mL, 1040 ng/mL, 1050 ng/mL, 1060 ng/mL, 1070 ng/mL, 1080 ng/mL, 1090 ng/mL, 1100 ng/mL, 1110 ng/mL, 1120 ng/mL, 1130 ng/mL, 1140 ng/mL, 1150 ng/mL, 1160 ng/mL, 1170 ng/mL, 1180 ng/mL, 1190 ng/mL, 1200 ng/mL, 1210 ng/mL, 1220 ng/mL, 1230 ng/mL, 1240 ng/mL, 1250 ng/mL, 1260 ng/mL, 1270 ng/mL, 1280 ng/mL, 1290 ng/mL, 1300 ng/mL, 1310 ng/mL, 1320 ng/mL, 1330 ng/mL, 1340 ng/mL, 1350 ng/mL, 1360 ng/mL, 1370 ng/mL, 1380 ng/mL, 1390 ng/mL, 1400 ng/mL, 1410 ng/mL, 1420 ng/mL, 1430 ng/mL, 1440 ng/mL, 1450 ng/mL, 1460 ng/mL, 1470 ng/mL, 1480 ng/mL, 1490 ng/mL, 1500 ng/mL, 1510 ng/mL, 1520 ng/mL, 1530 ng/mL, 1540 ng/mL, 1550 ng/mL, 1560 ng/mL, 1570 ng/mL, 1580 ng/mL, 1590 ng/mL, 1600 ng/mL, 1610 ng/mL, 1620 ng/mL, 1630 ng/mL, 1640 ng/mL, 1650 ng/mL, 1660 ng/mL, 1670 ng/mL, 1680 ng/mL, 1690 ng/mL, 1700 ng/mL, 1710 ng/mL, 1720 ng/mL, 1730 ng/mL, 1740 ng/mL, 1750 ng/mL, 1760 ng/mL, 1770 ng/mL, 1780 ng/mL, 1790 ng/mL, 1800 ng/mL, 1810 ng/mL, 1820 ng/mL, 1830 ng/mL, 1840 ng/mL, 1850 ng/mL, 1860 ng/mL, 1870 ng/mL, 1880 ng/mL, 1890 ng/mL, 1900 ng/mL, 1910 ng/mL, 1920 ng/mL, 1930 ng/mL, 1940 ng/mL, 1950 ng/mL, 1960 ng/mL, 1970 ng/mL, 1980 ng/mL, 1990 ng/mL, 2000 ng/mL, 2010 ng/mL, 2020 ng/mL, 2030 ng/mL, 2040 ng/mL, 2050 ng/mL, 2060 ng/mL, 2070 ng/mL, 2080 ng/mL, 2090 ng/mL, 2100 ng/mL, 2110 ng/mL, 2120 ng/mL, 2130 ng/mL, 2140 ng/mL, 2150 ng/mL, 2160 ng/mL, 2170 ng/mL, 2180 ng/mL, 2190 ng/mL, 2200 ng/mL, 2210 ng/mL, 2220 ng/mL, 2230 ng/mL, 2240 ng/mL, 2250 ng/mL, 2260 ng/mL, 2270 ng/mL, 2280 ng/mL, 2290 ng/mL, 2300 ng/mL, 2310 ng/mL, 2320 ng/mL, 2330 ng/mL, 2340 ng/mL, 2350 ng/mL, 2360 ng/mL, 2370 ng/mL, 2380 ng/mL, 2390 ng/mL, 2400 ng/mL, 2410 ng/mL, 2420 ng/mL, 2430 ng/mL, 2440 ng/mL, 2450 ng/mL, 2460 ng/mL, 2470 ng/mL, 2480 ng/mL, 2490 ng/mL, or 2500 ng/mL.

In another embodiment, the level of Ba in the sample is deemed elevated when it is at least about 12% higher to about 5-fold, e.g., about two-fold greater than the normal reference range for Factor Ba *(e.g.,* a healthy patient who does not have HSCT or an HSCT patient in the absence of TMA). In some embodiments, the level of Ba in the sample is deemed elevated when it is at least three, four, or five-fold greater than the normal reference range for Ba.

In another embodiment, the level of HSPG is elevated in HSCT patients who have or are likely to develop HSCT-TMA compared to a normal reference range *(e.g.,* a healthy patient who does not have HSCT or an HSCT patient in the absence of TMA).

In another embodiment, the level of HSPG in the sample is deemed elevated when it is at least about 20% higher to about 2-fold, e.g., about two-fold, three-fold, four-fold, five-fold, six-fold, seven-fold, eight-fold, nine-fold, ten-fold, eleven-fold, twelve-fold, thirteen-fold, fourteen-fold, fifteen-fold, sixteen-fold, seventeen-fold, eighteen-fold, nineteen-fold, or twenty-fold greater than the normal reference range for HSPGs (e.g., a healthy patient who does not have HSCT or an HSCT patient in the absence of TMA).

Any suitable anti-C5 antibody, or antigen binding fragment thereof, can be used in the methods described herein. In one embodiment, the anti-C5 antibody is a human antibody, a humanized antibody, a bispecific antibody, a chimeric antibody, a Fab, a Fab'2, a scFv, a SMIP, an Affibody^{®}, a nanobody, or a domain antibody which inhibits C5.

Any suitable anti-C5 antibody, or antigen binding fragment thereof, can be used in the methods described herein. An exemplary anti-C5 antibody is eculizumab. Eculizumab (also known as SOLIRIS^{®}) is an anti-C5 antibody comprising heavy chain CDR1, CDR2 and CDR3 domains having the sequences set forth in SEQ ID NOs: 1, 2, and 3, respectively, and light chain CDR1, CDR2 and CDR3 domains having the sequences set forth in SEQ ID NOs: 4, 5, and 6, respectively. Eculizumab comprises a heavy chain variable region having the amino acid sequence set forth in SEQ ID NO: 7 and a light chain variable region having the amino acid sequence set forth in SEQ ID NO: 8. Eculizumab comprises a heavy chain comprising the amino acid sequence set forth in SEQ ID NO:10 and a light chain having the amino acid sequence set forth in SEQ ID NO:11.

Another exemplary anti-C5 antibody is ravulizumab (also known as ULTOMIRIS^{®}, ALXN1210 and antibody BNJ441) comprising the heavy and light chains having the sequences shown in SEQ ID NOs:14 and 11, respectively, or antigen binding fragments and variants thereof. In other embodiments, the antibody comprises the heavy and light chain complementarity determining regions (CDRs) or variable regions (VRs) of ravulizumab. Accordingly, in one embodiment, the antibody comprises the CDR1, CDR2, and CDR3 domains of the heavy chain variable (VH) region of ravulizumab having the sequence shown in SEQ ID NO:12, and the CDR1, CDR2 and CDR3 domains of the light chain variable (VL) region of ravulizumab having the sequence shown in SEQ ID NO:8. In another embodiment, the antibody comprises CDR1, CDR2 and CDR3 heavy chain sequences as set forth in SEQ ID NOs:19, 18, and 3, respectively, and CDR1, CDR2 and CDR3 light chain sequences as set forth in SEQ ID NOs:4, 5, and 6, respectively. In another embodiment, the antibody comprises VH and VL regions having the amino acid sequences set forth in SEQ ID NO:12 and SEQ ID NO:8, respectively. In another embodiment, the antibody comprises a heavy chain constant region as set forth in SEQ ID NO:13.

In another embodiment, the antibody comprises a variant human Fc constant region that binds to human neonatal Fc receptor (FcRn), wherein the variant human Fc CH3 constant region comprises Met-429-Leu and Asn-435-Ser substitutions at residues corresponding to methionine 428 and asparagine 434 of a native human IgG Fc constant region, each in EU numbering.

In another embodiment, the antibody comprises CDR1, CDR2 and CDR3 heavy chain sequences as set forth in SEQ ID NOs:19, 18, and 3, respectively, and CDR1, CDR2 and CDR3 light chain sequences as set forth in SEQ ID NOs:4, 5, and 6, respectively and a variant human Fc constant region that binds to human neonatal Fc receptor (FcRn), wherein the variant human Fc CH3 constant region comprises Met-429-Leu and Asn-435-Ser substitutions at residues corresponding to methionine 428 and asparagine 434 of a native human IgG Fc constant region, each in EU numbering.

In another embodiment, the antibody binds to human C5 at pH 7.4 and 25°C with an affinity dissociation constant (K_{D}) that is in the range 0.1 nM ≤ K_{D} ≤ 1 nM. In another embodiment, the antibody binds to human C5 at pH 6.0 and 25°C with a K_{D} ≥ 10 nM. In yet another embodiment, the [(K_{D} of the antibody or antigen-binding fragment thereof for human C5 at pH 6.0 and at 25°C)/(K_{D} of the antibody or antigen-binding fragment thereof for human C5 at pH 7.4 and at 25°C)] of the antibody is greater than 25.

Another exemplary anti-C5 antibody is described in US Patent Nos. 8,241,628 and 8,883,158. In one embodiment, the antibody, or antigen binding fragment thereof, comprises heavy chain CDR1, CDR2 and CDR3 domains having the sequences set forth in SEQ ID NOs: 21, 22, and 23, respectively, and light chain CDR1, CDR2 and CDR3 domains having the sequences set forth in SEQ ID NOs: 24, 25, and 26, respectively. In another embodiment, the antibody, or antigen binding fragment thereof, comprises the VH region having the sequence set forth in SEQ ID NO:27, and the VL region having the sequence set forth in SEQ ID NO:28.

Another exemplary anti-C5 antibody is also described in US Patent Nos. 8,241,628 and 8,883,158. In one embodiment, the antibody, or antigen binding fragment thereof, comprises heavy chain CDR1, CDR2 and CDR3 domains having the sequences set forth in SEQ ID NOs: 29, 30, and 31, respectively, and light chain CDR1, CDR2 and CDR3 domains having the sequences set forth in SEQ ID NOs: 32, 33, and 34, respectively. In another embodiment, the antibody comprises the VH region having the sequence set forth in SEQ ID NO: 35, and the VL region having the sequence set forth in SEQ ID NO: 36.

Another exemplary anti-C5 antibody is described in US Pat. No. 9,765,135. In one embodiment, the antibody, or antigen binding fragment thereof, comprises heavy chain CDR1, CDR2 and CDR3 domains having the sequences set forth in SEQ ID NOs: 37, 38, and 39, respectively, and light chain CDR1, CDR2 and CDR3 domains having the sequences set forth in SEQ ID NOs: 40, 41, and 42, respectively. In another embodiment, the antibody comprises the VH region having the sequence set forth in SEQ ID NO: 43, and the VL region having the sequence set forth in SEQ ID NO: 44.

Another exemplary anti-C5 antibody is described in Fukuzawa T. et al. (Sci. Rep. 7:1080, 2017). In another embodiment, the antibody, or antigen binding fragment thereof, comprises a heavy chain comprising SEQ ID NO: 45 and a light chain comprising SEQ ID NO: 46.

Another exemplary anti-C5 antibody is described in US Pat. No. 10,633,434. In one embodiment, the antibody comprises a heavy chain variable region comprising SEQ ID NO:47 and a light chain variable region comprising SEQ ID NO:48. In another embodiment, the antibody comprises a heavy chain comprising SEQ ID NO:49 and a light chain comprising SEQ ID NO:50.

In some embodiments, the anti-C5 antibody is a biosimilar of eculizumab (SOLIRIS^{®}). For example, in one embodiment, the anti-C5 antibody is, for example, ABP 959 antibody (eculizumab biosimilar manufactured by Amgen Inc., USA), ELIZARIA^{®} (manufactured by Generium JNC, Russia), SB12 (eculizumab biosimilar manufactured by Samsung Bioepis, Incheon, South Korea), ISU305 (eculizumab biosimilar from ISU Abxis, South Korea), ABLYZE^{®} (eculizumab biosimilar from CinnaGen, Iran), BCD 148 (eculizumab biosimilar from Biocad Medical, Quebec, Canada), tesidolumab (manufactured by Novartis), Crovalimab (manufactured by Roche), CAN106 (manufactured by CanBridge Bio, China), or Pozelimab (manufactured by Regeneron).

In another embodiment, the antibody competes for binding with, and/or binds to the same epitope on C5 as, the above-mentioned antibodies. In another embodiment, the antibody has at least about 90% variable region amino acid sequence identity with the above-mentioned antibodies (e.g., at least about 90%, 91%, 92%, 93%, 94%, 95%, 96%, 97%, 98% or 99% variable region identity).

The anti-C5 antibody can be administered via any suitable means. In one embodiment, the anti-C5 antibody is administered intravenously. In another embodiment, the anti-C5 antibody is administered subcutaneously.

In one embodiment, the anti-C5 antibody (e.g., ravulizumab) is administered:
(a) once on Day 1 at a dose of 600 mg to a patient weighing ≥ 5 to < 10 kg, 600 mg to a patient weighing ≥ 10 to < 20 kg, 900 mg to a patient weighing ≥ 20 to < 30 kg, 1200 mg to a patient weighing ≥ 30 to < 40 kg, 2400 mg to a patient weighing ≥ 40 to < 60 kg, 2700 mg to a patient weighing ≥ 60 to < 100 kg, or 3000 mg to a patient weighing ≥ 100 kg;
(b) once on Day 5 at a dose of 300 mg to a patient weighing ≥ 5 to < 10 kg, 300 mg to a patient weighing ≥ 10 to < 20 kg, 300 mg to a patient weighing ≥ 20 to < 30 kg, 300 mg to a patient weighing ≥ 30 to < 40 kg, 600 mg to a patient weighing ≥ 40 to < 60 kg, 900 mg to a patient weighing ≥ 60 to < 100 kg, or 900 mg to a patient weighing ≥ 100 kg;
(c) once on Day 10 at a dose of 300 mg to a patient weighing ≥ 5 to < 10 kg, 300 mg to a patient weighing ≥ 10 to < 20 kg, 300 mg to a patient weighing ≥ 20 to < 30 kg, 300 mg to a patient weighing ≥ 30 to < 40 kg, 600 mg to a patient weighing ≥ 40 to < 60 kg, 900 mg to a patient weighing ≥ 60 to < 100 kg, or 900 mg to a patient weighing ≥ 100 kg; and
(d) on Day 15 and every four weeks thereafter at a dose of 300 mg to a patient weighing ≥ 5 to < 10 kg or 600 mg to a patient weighing ≥ 10 to < 20 kg; or on Day 15 and every eight weeks thereafter at a dose of 2100 mg to a patient weighing ≥ 20 to < 30 kg, 2700 mg to a patient weighing ≥ 30 to < 40 kg, 3000 mg to a patient weighing ≥ 40 to < 60 kg, 3300 mg to a patient weighing ≥ 60 to < 100 kg, or 3600 mg to a patient weighing ≥ 100 kg.

In another aspect, a an anti-CFB antibody is used in the methods described herein. Any suitable an anti-CFB antibody can be used in the methods described herein. The efficacy of the treatment methods provided herein can be assessed using any suitable means. In one embodiment, the treatment results in a reduction or cessation in microangiopathic hemolytic anemia, thrombocytopenia, endothelial injury, kidney damage, kidney failure, serositis, pulmonary hypertension, and multisystem organ failure compared to baseline. In another embodiment, the treatment results in normalization of LDH, resolution of need for red cell and platelet transfusions, an increase in hemoglobin, and/or a disappearance of schistocytes compared to baseline. In another embodiment, the treatment results in (a) platelet count ≥ 50,000/mm³ without transfusion support during the prior 7 days, (b) LDH <1.5 × ULN, and (c) absence of schistocytes (if there were schistocytes present at baseline), and/or (d) at least 50% reduction of proteinuria from baseline. In another embodiment, the treatment results in results in a favorable hematological response. In another embodiment, the treatment results in hemoglobin ≥ 8 g/dL without transfusion support. In another embodiment, the treatment results in terminal complement inhibition. In another embodiment, the treatment results in a reduction in adverse events. In another embodiment, the treatment results in a change from baseline in quality of life as assessed via a Quality of Life Assessment. In another embodiment, the Quality of Life Assessment is a Quality of Life Inventory (PedsQL) Scale or an EQ-5D-5L questionnaire).

In another aspect, an anti-C5 antibody, or antigen-binding fragment thereof, or an anti-CFB antibody for use in treatment of a patient having hematopoietic stem cell transplant-associated thrombotic microangiopathy (HSCT-TMA) who has been determined to have elevated blood levels of a biomarker selected from thrombomodulin (TM) and syndecan-1 (SYND1), or a combination thereof compared to normal reference ranges of the biomarker is provided, wherein the anti-C5 antibody or an anti-CFB antibody is administered to the patient in an amount and with a frequency sufficient to attenuate the biomarker levels in the patient.

In another aspect, an anti-C5 antibody, or antigen-binding fragment thereof, or an anti-CFB antibody for use in identifying a patient having HSCT-TMA that is suitable for treatment with an anti-C5 antibody or an anti-CFB antibody is provided, comprising determining levels of a biomarker selected from TM and SYND1, or a combination thereof, in a blood sample from the patient using an *in vitro* assay, wherein elevated levels of the biomarker in the blood sample compared to normal reference ranges of TM and SYND1, respectively, identifies the patient as being suitable for treatment with an anti-C5 antibody or an anti-CFB antibody .

In another aspect, an anti-C5 antibody, or antigen-binding fragment thereof, or an anti-CFB antibody for use in monitoring responsiveness of a patient having HSCT-TMA to treatment with an anti-C5 antibody or an anti-CFB antibody is provided, wherein the use comprises: determining a biomarker selected from TM and SYND1 or a combination thereof in a blood sample from the patient obtained during or after treatment, wherein: decreased biomarker levels in the blood sample from the patient obtained during or after treatment, as compared to the biomarker levels in a blood sample from the patient obtained prior to treatment with the anti-C5 antibody or an anti-CFB antibody, indicates that the patient is responsive to treatment with the anti-C5 antibody or an anti-CFB antibody.

In another aspect, the use of an anti-C5, antibody or antigen-binding fragment thereof, or an anti-CFB antibody for treatment of a patient having hematopoietic stem cell transplant-associated thrombotic microangiopathy (HSCT-TMA) who has been determined to have elevated blood levels of a biomarker selected from thrombomodulin (TM) and syndecan-1 (SYND 1), or a combination thereof compared to normal reference ranges of the biomarker is provided, wherein the anti-C5 antibody or an anti-CFB antibody is administered to the patient in an amount and with a frequency sufficient to attenuate the biomarker levels in the patient.

In another aspect, the use of an anti-C5 antibody, or antigen-binding fragment thereof, or an anti-CFB antibody in identifying a patient having HSCT-TMA that is suitable for treatment with an anti-C5 antibody or an anti-CFB antibody, comprising determining levels of a biomarker selected from TM and SYND1, or a combination thereof, in a blood sample from the patient using an *in vitro* assay is provided, wherein elevated levels of the biomarker in the blood sample compared to normal reference ranges of TM and SYND1, respectively, identifies the patient as being suitable for treatment with an anti-C5 antibody or an anti-CFB antibody.

In another aspect, the use of an anti-C5 antibody, or antigen-binding fragment thereof, or an anti-CFB antibody in monitoring responsiveness of a patient having HSCT-TMA to treatment with an anti-C5 antibody or an anti-CFB antibody is provided, the use comprising: determining a biomarker selected from TM and SYND1 or a combination thereof in a blood sample from the patient obtained during or after treatment, wherein: decreased biomarker levels in the blood sample from the patient obtained during or after treatment, as compared to the biomarker levels in a blood sample from the patient obtained prior to treatment with the anti-C5 antibody or an anti-CFB antibody, indicates that the patient is responsive to treatment with the anti-C5 antibody or an anti-CFB antibody.

### BRIEF DESCRIPTION OF THE DRAWINGS

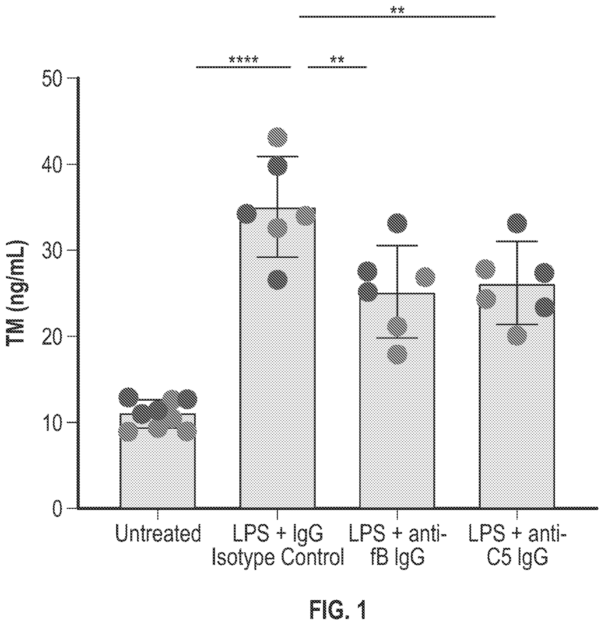

**FIG. 1** is a graph showing plasma TM levels in LPS-injected mice treated with anti-complement agents.

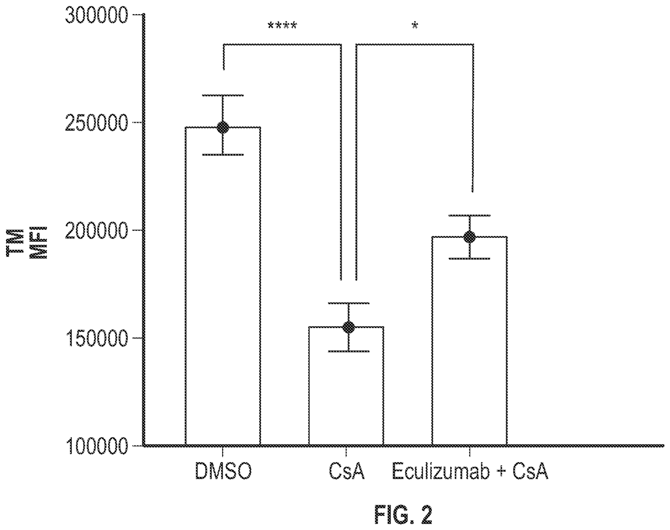

**FIG. 2** is a graph showing cyclosporin-induced TM loss is partially prevented by treatment with eculizumab.

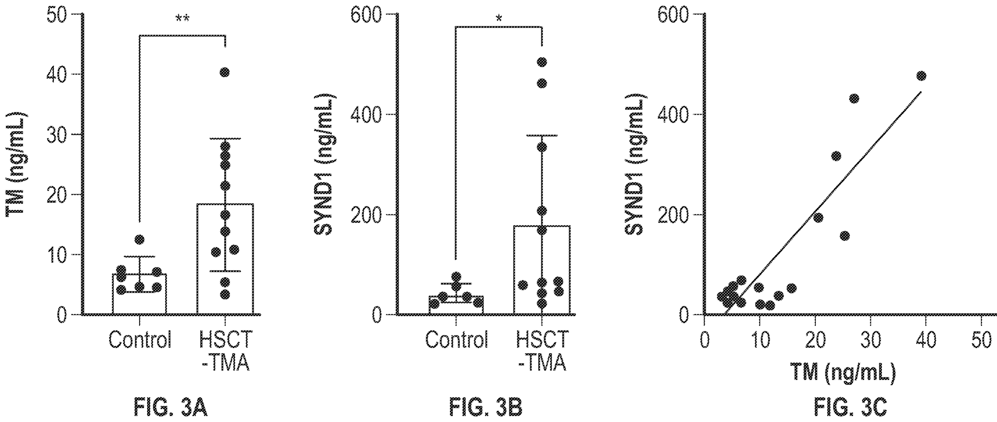

**FIGs. 3A-3C** depict biomarker levels of glyclocalyx components in pediatric patients with and without HSCT-TMA. Specifically, **FIGs. 3A and 3B** show that HSCT-TMA patients (n=11), compared to HSCT control patients (n=7), had significantly elevated levels of biomarkers of glycocalyx damage. **FIG. 3C** shows that a strong positive correlation between TM and SYND1 was observed.

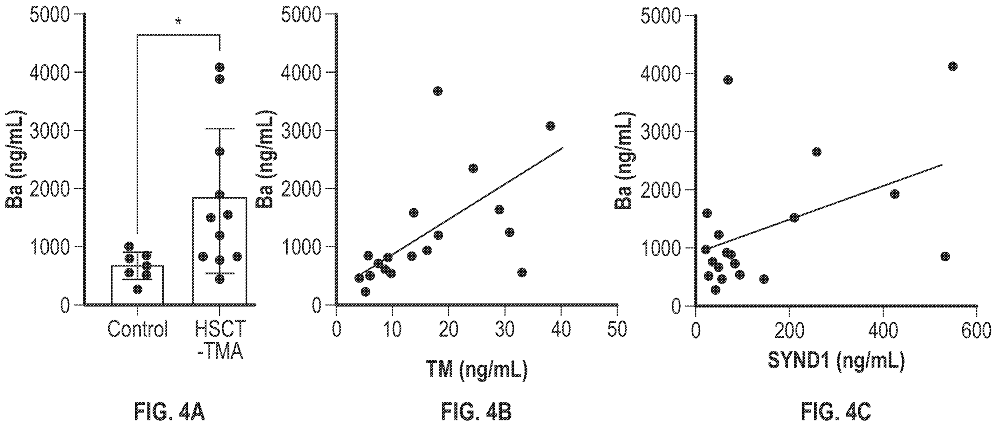

**FIGs. 4A-4C** depict AP activation in pediatric patients with and without HSCT-TMA. Specifically, **FIG. 4A** shows that plasma Ba was significantly elevated in patients with HSCT-TMA compared to those without. In addition, plasma Ba correlated positively with both TM **(****FIG. 4B****)** and SYND1 **(****FIG. 4C****)** levels.

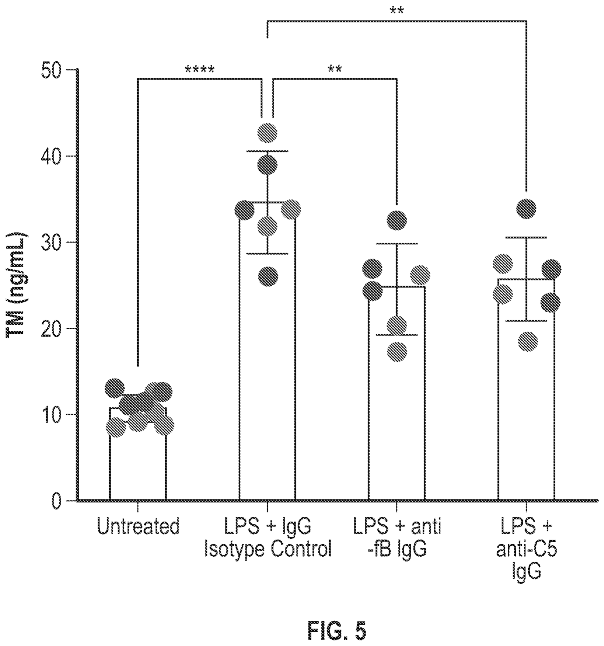

**FIG. 5** depicts plasma TM levels in LPS-injected mice treated with anti-complement agents. As shown in **FIG. 5****,** mice with inflammation-mediated complement activation had elevated circulating TM levels, which were attenuated with anti-complement agent treatment.

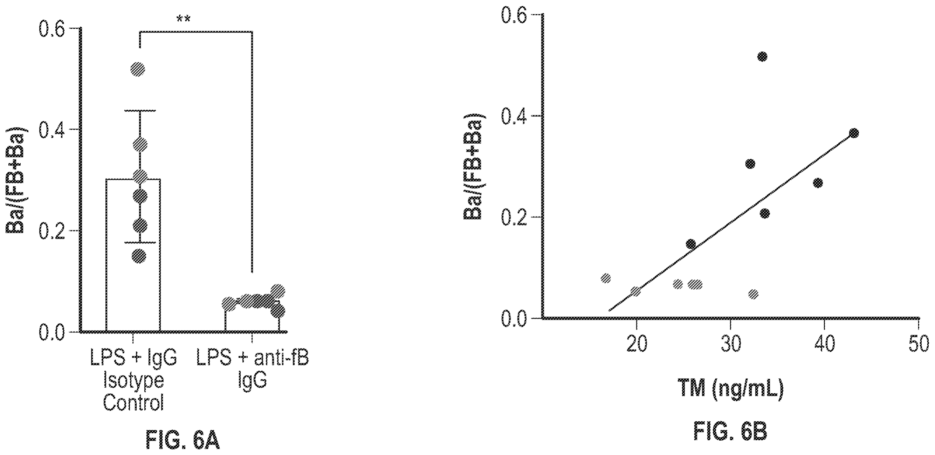

**FIGs. 6A-6B** show the correlation of TM and Ba in LPS-injected mice. TM levels and Ba levels were measured with a commercially available ELISA or by western blotting, respectively **(****FIG. 6A****).** As shown in **FIG. 6B****,** in mice with inflammation-mediated complement activation, plasma Ba correlated positively with TM levels.

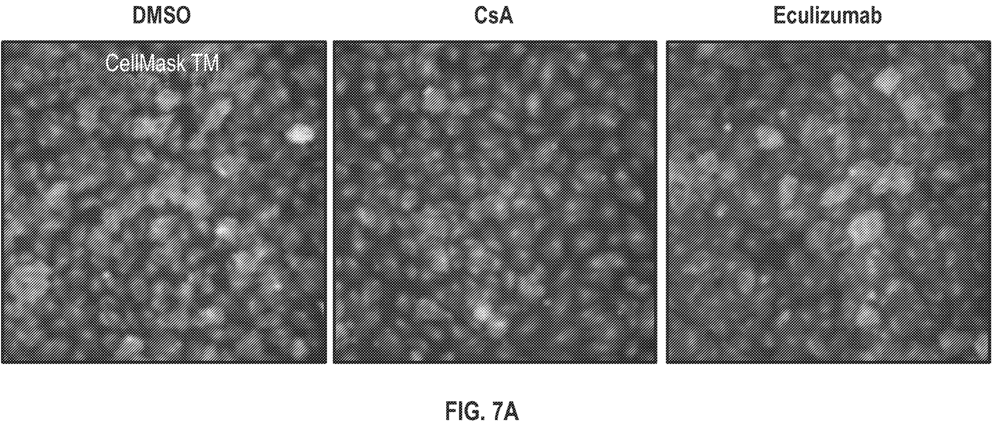

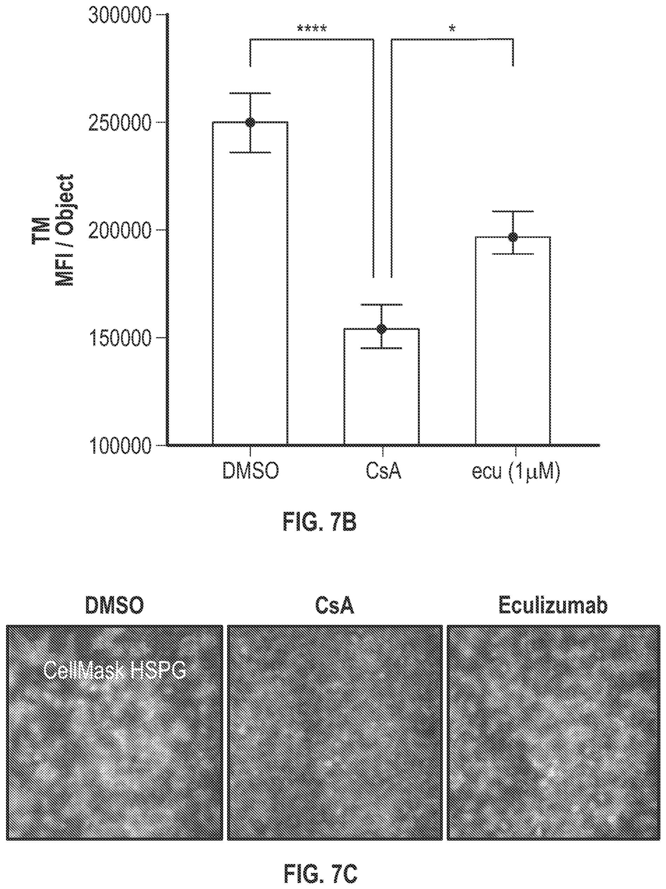

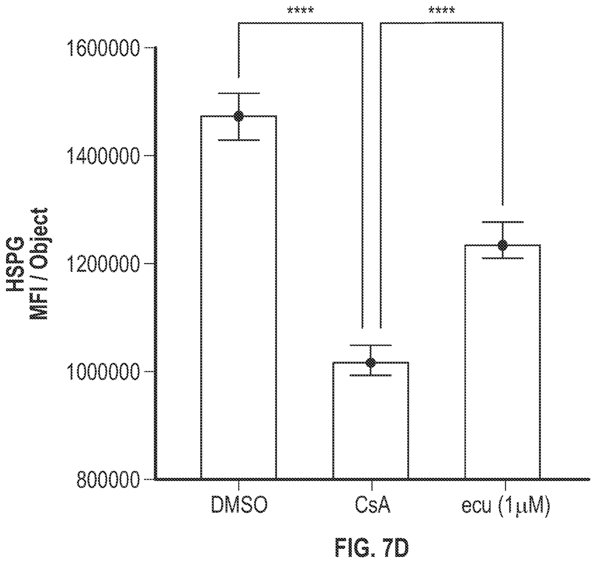

**FIGs. 7A-7D** depict cyclosporin-induced TM loss in HUVECs treated with CsA and 30% NHS in the presence **(****FIGs. 7A-7B****)** or absence of an eculizumab surrogate(**FIGs 7C-****7D)** for 18h. As shown by these figures, TM and heparan sulfate proteoglycan (HSPG) surface expression was reduced on HUVECs treated with CsA and partially restored by the eculizumab surrogate.

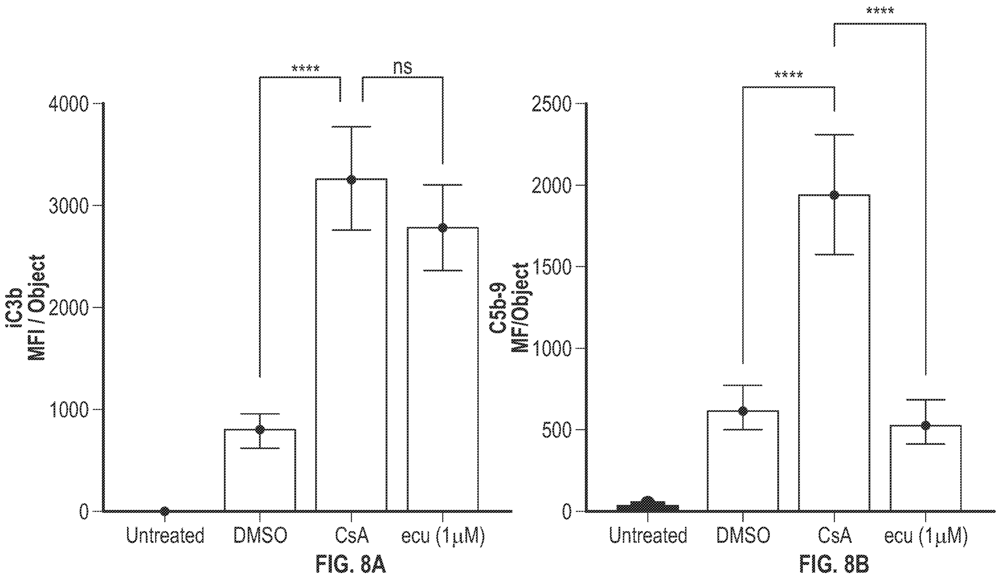

**FIGs. 8A-8B** depict deposition of complement activation products on HMEC-1 cells treated with CsA. As shown in these figures, CsA treatment induced complement deposition on HMEC-1 cells and C5 inhibition reduced C5b-9 **(****FIG. 8B****),** but not iC3b deposits **(****FIG. 8A****).**

### DETAILED DESCRIPTION

As described herein and exemplified in the working Example, particular biomarkers associated with HSCT-TMA have been discovered. Specifically, it has been discovered that HSCT patients with elevated concentration of certain proteins (e.g., TM, SYND1, and/or Ba, referred to herein as "HSCT-TMA biomarker proteins") are likely to develop HSCT-TMA. Similarly, a reduced and/or normalized concentration (or activity) of these proteins in a biological fluid obtained from an HSCT-TMA patient treated with a complement inhibitor *(e.g.,* an anti-C5 antibody *(e.g.,* eculizumab or ravulizumab) or anti-CFB antibody) indicates that the patient is responsive to the treatment. Accordingly, analysis of the concentration and/or activity level of such proteins can be employed to evaluate, among other things, risk that an HSCT patient will develop HSCT-TMA, monitor progression or abatement of HSCT-TMA, and/or monitor treatment response to a complement inhibitor (e.g., such as anti-C5 antibody (e.g., eculizumab or ravulizumab) or anti-CFB antibody).

### I. Definitions

As used herein, the term "subject" or "patient" is a human patient (e.g., a patient having HSCT or HSCT-TMA).

As used herein, the term "pediatric" patient is a human patient that has been classified by a physician or caretaker as belonging to a non-adult category and can include, e.g., newborn (both preterm and of term), infants, children, and adolescents. Typically, pediatric patients are patients under 18 years of age (<18 years of age).

As used herein, the term "adult" patient is a human patient that has been classified by a physician or caretaker as such, *e.g.,* one who is not a newborn, infant, child or adolescent, *e.g.,* based on age, developmental status, physiological features, *etc.* Typically, adult patients are patients who are 18 years of age or older (≥18 years of age).

The term "antibody" describes a polypeptide comprising at least one antibody-derived antigen binding site (e.g., VH/VL region or Fv, or CDR). The term "antibody" is used interchangeably with the term "immunoglobulin." Antibodies include known forms of antibodies, e.g., the antibody can be a human antibody, a humanized antibody, a bispecific antibody, a chimerized or chimeric antibody, a polyclonal antibody, a monoclonal antibody, a primatized antibody, and a deimmunized antibody. The antibody can be made in or derived from any of a variety of species, e.g., mammals such as humans, non-human primates (e.g., orangutan, baboons, or chimpanzees), horses, cattle, pigs, sheep, goats, dogs, cats, rabbits, guinea pigs, gerbils, hamsters, rats, and mice. The antibody can be a purified or a recombinant antibody. The antibody also can be of any of the following isotypes: IgG1, IgG2, IgG3, IgG4, IgM, IgA1, IgA2, IgAsec, IgD, IgE or combinations thereof. The antibody can be a naturally occurring antibody or an antibody that has been altered by a protein engineering technique (e.g., by mutation, deletion, substitution, conjugation to a non-antibody moiety). An antibody can include, for example, one or more variant amino acids (compared to a naturally occurring antibody) that change a property (e.g., a functional property) of the antibody. Numerous such alterations are known in the art that affect, e.g., half-life, effector function, and/or immune responses to the antibody in a patient. The term antibody also includes artificial or engineered polypeptide constructs that comprise at least one antibody-derived antigen binding site.

The term "antibody" includes antigen-binding fragments thereof. The term "antigen-binding fragment" or similar terms are known in the art and can, for example, refer to a fragment of an antibody that retains the ability to bind to a target antigen (e.g., human C5) and inhibit the activity of the target antigen. Such fragments include, e.g., a single chain antibody, a single chain Fv fragment (scFv), an Fd fragment, a Fab fragment, a Fab' fragment, or an F(ab')2 fragment. A scFv fragment is a single polypeptide chain that includes both the heavy and light chain variable regions of the antibody from which the scFv is derived. In addition, intrabodies, minibodies, triabodies, and diabodies are also included in the definition of antibody and are compatible for use in the methods described herein. See, *e.g.*, Todorovska et al. (2001) J Immunol Methods 248(1):47-66; Hudson and Kortt (1999) J Immunol Methods 231(1):177-189; Poljak (1994) Structure 2(12):1121-1123; Rondon and Marasco (1997) Annual Review of Microbiology 51:257-283. An antigen-binding fragment can also include the variable region of a heavy chain polypeptide and the variable region of a light chain polypeptide. An antigen-binding fragment can thus comprise the CDRs of the light chain and heavy chain polypeptide of an antibody.

The term "antibody fragment" also can include, e.g., single domain antibodies such as camelized single domain antibodies. *See, e.g.,* Muyldermans et al. (2001) Trends Biochem Sci 26:230-235; Nuttall et al. (2000) Curr Pharm Biotech 1:253-263; Reichmann et al. (1999) J Immunol Meth 231:25-38; PCT application publication nos. WO 94/04678 and WO 94/25591; and U.S. patent no. 6,005,079. The term "antibody fragment" also includes single domain antibodies comprising two V_{H} domains with modifications such that single domain antibodies are formed.

The terms "polypeptide," "peptide," and "protein" are used interchangeably and are known in the art and can mean any peptide-bond linked chain of amino acids, regardless of length or post-translational modification.

### II. HSCT-TMA Associated Biomarker Proteins

As used herein, the term "biomarker" refers to a measurable substance in a subject whose presence is indicative of some phenomenon such as disease, infection, or environmental exposure. By way of the present disclosure, it has been discovered that certain biomarkers are associated with and indicative of HSCT-TMA. These HSCT-TMA associated biomarker proteins include, e.g., thrombomodulin (TM), syndecan-1 (SYND1), and a proteolytic fragment of complement component factor B (*e.g.*, Ba).

### A. Thrombomodulin

Thrombomodulin (also known as "TM", "THBD", "AHUS6", "BDCA3", "CD141", "THPH12", "THRM", and "BDCA-3") is a transmembrane glycoprotein located on the luminal surface of endothelial cells in most normal blood vessels that regulates coagulation and inflammation (see, e.g., Califano, et al., Eur. Rev. Med. Pharmacol. Sci. 2000 May-Jun;4(3):59-66). Thrombomodulin is also expressed on human mesothelial cell, monocyte, and a dendritic cell subset. Thrombomodulin can stimulate endothelial cell growth. This characteristic of TM depends from the molecular substrate of the sequences, known as EGF-like (epidermal growth factor-like). One of the main roles of TM is to bind thrombin. When bound to TM, thrombin loses its procoagulant, proinflammatory, and profibrogenic properties and instead acquires the ability to activate protein C (APC). APC limits further thrombin generation, counteracts thrombin's adverse effects, and has additional anti-inflammatory and cytoprotective properties. TM itself also has intrinsic anti-inflammatory actions by binding to and inhibiting the proinflammatory protein HMGB1. Thrombomodulin is present in the body in two forms (see, *e.g.,* Doggen et al., Thromb. Haemost. 1998; 80: 743-748). The first type has a higher molecular weight, and it is bound to the cytoplasmatic membrane of endothelial cells (see, e.g., Boff MC, Haemostasis 1996; 26 (Suppl 4): 233-243). The second form has a lower molecular weight and represents the soluble or plasmatic form. The heavy form weighs 150 kDa and the light form 69 kDa. The molecular sequence of thrombomodulin contains an N-terminal lectin-like element (1-154 residues), an hydrophobic region (155-222 residues), six EGF-like modules (223-462 residues), one dominium rich in Ser/Thr (463-497 residues), a transmembrane moiety composed by 23 amino acids (498-521 residues) and a tail of 35 cytoplasmic amino acids (522-557 residues) (see, *e.g.,* Califano, et al., Eur. Rev. Med. Pharmacol. Sci. 2000 May-Jun;4(3):59-66).

Different pathological situations increase soluble circulating TM (see, *e.g.,* Califano, et al., Eur. Rev. Med. Pharmacol. Sci. 2000 May-Jun;4(3):59-66). The endothelial cells more exposed to hemodynamic turbulence (such as those on the bifurcation of major arteries) liberate a great quantity of TM (see, e.g., Salomaa et al., Lancet 1999; 353: 1729-1734). Thrombomodulin levels range from 3 to 300 ng/ml. It is believed that normal levels are 3.1 ± 1.3 ng/ml, with slightly higher levels in males (see, e.g., Doggen et al., Thromb. Haemost. 1998; 80: 743-748). It seems that in the women TM levels rise during menopause. Women with surgical induced menopause have soluble TM levels well above normal. After six weeks of hormone replacement therapy a significant reduction of TM levels takes place (see, e.g., Neumann et al., Circulation 1995; 92: 748-755). Thrombomodulin levels vary according to race and Blacks seem to have lower levels (see, *e.g.,* Tohda et al., Arterioscler. Thromb. Vasc. Biol. 1998; 18: 1861-1869. Thrombomodulin levels are usually measured with ELISA method.

In one embodiment, the normal reference range for TM for a healthy patient (e.g., a patient who does not have HSCT) is about 1.8 ng/mL to about 4.8 ng/mL. For example, in one embodiment, a normal TM level for a healthy patient is about 1.8 ng/mL, 1.9 ng/mL, 2.0 ng/mL, 2.1 ng/mL, 2.2 ng/mL, 2.3 ng/mL, 2.4 ng/mL, 2.5 ng/mL, 2.6 ng/mL, 2.7 ng/mL, 2.8 ng/mL, 2.9 ng/mL, 3.0 ng/mL, 3.1 ng/mL, 3.2 ng/mL, 3.3 ng/mL, 3.4 ng/mL, 3.5 ng/mL, 3.6 ng/mL, 3.7 ng/mL, 3.8 ng/mL, 3.9 ng/mL, 4.0 ng/mL, 4.1 ng/mL, 4.2 ng/mL, 4.3 ng/mL, 4.4 ng/mL, 4.5 ng/mL, 4.6 ng/mL, 4.7 ng/mL, or 4.8 ng/mL.

In another embodiment, the normal reference range for TM for a HSCT patient in the absence of TMA is about 3 ng/mL to about 9 ng/mL. For example, in one embodiment, a normal reference range for TM for a HSCT patient in the absence of TMA is about 3 ng/mL, 4 ng/mL, 5 ng/mL, 6 ng/mL, 7 ng/mL, 8 ng/mL, or 9 ng/mL.

In another embodiment, levels of TM are elevated in HSCT patients who have or are likely to develop HSCT-TMA compared to a normal reference range *(e.g.,* a healthy patient who does not have HSCT or an HSCT patient in the absence of TMA). For example, an elevated TM level is greater than about 10 ng/mL, 11 ng/mL, 12 ng/mL, 13 ng/mL, 14 ng/mL, 15 ng/mL, 16 ng/mL, 17 ng/mL, 18 ng/mL, 19 ng/mL, 20 ng/mL, 21 ng/mL, 22 ng/mL, 23 ng/mL, 24 ng/mL, 25 ng/mL, 26 ng/mL, 27 ng/mL, 28 ng/mL, 29 ng/mL, or 30 ng/mL.

In another embodiment, the level of TM in the sample is deemed elevated when it is at least about 10% higher to about 10-fold, e.g., about two-fold greater than the normal reference range for TM *(e.g.,* a healthy patient who does not have HSCT or an HSCT patient in the absence of TMA). In some embodiments, the level of TM in the sample is deemed elevated when it is at least three, four, five, or six-fold greater than the normal reference range for TM.

### B_{.} Syndecan-1

Syndecan-1 (also known as "SDC", "Syndecan proteoglycan 1", "CD138", "SDC1", "SYND1" "syndecan", "heparan sulfate proteoglycan fibroblast growth factor receptor," SYND1 is a protein which in humans is encoded by the SDC1 gene (see, e.g., Mali M, et al., (April 1990), The Journal of Biological Chemistry, 265 (12): 6884-9; and Ala-Kapee M, et al. (September 1990), Somatic Cell and Molecular Genetics, 16 (5): 501-5). The protein is a transmembrane (type I) heparan sulfate proteoglycan and is a member of the syndecan proteoglycan family. The SYND1 protein functions as an integral membrane protein and participates in cell proliferation, cell migration and cell-matrix interactions via its receptor for extracellular matrix proteins. SYND1 is a sponge for growth factors and chemokines, with binding largely via heparan sulfate chains (see, *e.g.,* Götte M (April 2003), FASEB Journal. 17 (6): 575-91). The syndecans mediate cell binding, cell signaling, and cytoskeletal organization and syndecan receptors are required for internalization of the HIV-1 tat protein. The SYND1 core protein consists of an extracellular domain which can be substituted with heparan sulfate and chondroitin sulfate glycosaminoglycan chains, a highly conserved transmembrane domain, and a highly conserved cytoplasmic domain, which contains two constant regions that are separated by a variable region (see, e.g., Bemfield M, (1999), Annual Review of Biochemistry. 68: 729-77). The extracellular domain can be cleaved (shed) from the cell surface at a juxtamembrane site, converting the membrane-bound proteoglycan into a paracrine effector molecule with roles in wound repair, and invasive growth of cancer cells (see, e.g., Wang Z, Götte M, Bemfield M, Reizes O (September 2005), Biochemistry. 44 (37): 12355-61, Elenius V, et al., The Journal of Biological Chemistry. 279 (40): 41928-35, and Piperigkou Z, (September 2016), Cell and Tissue Research. 365 (3): 643-55).

In one embodiment, the normal reference range for SYND1 for a healthy patient *(e.g.,* a patient who does not have HSCT) is between 15 ng/mL to 70 ng/mL. For example, in one embodiment, a normal SYND1 level for a healthy patient is about 15 ng/mL, 16 ng/mL, 17 ng/mL, 18 ng/mL, 19 ng/mL, 20 ng/mL, 21ng/mL, 22 ng/mL, 23 ng/mL, 24 ng/mL, 25 ng/mL, 26 ng/mL, 27 ng/mL, 28 ng/mL, 29 ng/mL, 30 ng/mL, 31ng/mL, 32 ng/mL, 33 ng/mL, 34 ng/mL, 35 ng/mL, 36 ng/mL, 37 ng/mL, 38 ng/mL, 39 ng/mL, 40 ng/mL, 41ng/mL, 42 ng/mL, 43 ng/mL, 44 ng/mL, 45 ng/mL, 46 ng/mL, 47 ng/mL, 48 ng/mL, 49 ng/mL, 50 ng/mL, 51 ng/mL, 52 ng/mL, 53 ng/mL, 54 ng/mL, 55 ng/mL, 56 ng/mL, 57 ng/mL, 58 ng/mL, 59 ng/mL, 60 ng/mL, 61 ng/mL, 62 ng/mL, 63 ng/mL, 64 ng/mL, 65 ng/mL, 66 ng/mL, 67 ng/mL, 68 ng/mL, 69 ng/mL, or 70 ng/mL.

In another embodiment, the normal reference range for SYND1 for a HSCT patient in the absence of TMA is about 15 ng/mL to 55 ng/mL. For example, in one embodiment, a normal reference range for SYND1 for a HSCT patient in the absence of TMA is about 15 ng/mL, 16 ng/mL, 17 ng/mL, 18 ng/mL, 19 ng/mL, 20 ng/mL, 21ng/mL, 22 ng/mL, 23 ng/mL, 24 ng/mL, 25 ng/mL, 26 ng/mL, 27 ng/mL, 28 ng/mL, 29 ng/mL, 30 ng/mL, 31ng/mL, 32 ng/mL, 33 ng/mL, 34 ng/mL, 35 ng/mL, 36 ng/mL, 37 ng/mL, 38 ng/mL, 39 ng/mL, 40 ng/mL, 41ng/mL, 42 ng/mL, 43 ng/mL, 44 ng/mL, 45 ng/mL, 46 ng/mL, 47 ng/mL, 48 ng/mL, 49 ng/mL, 50 ng/mL, 51ng/mL, 52 ng/mL, 53 ng/mL, 54 ng/mL, or 55 ng/mL.

In another embodiment, levels of SYND1 are elevated in HSCT patients who have or are likely to develop HSCT-TMA compared to a normal reference range (e.g., a healthy patient who does not have HSCT or an HSCT patient in the absence of TMA). For example, an elevated SYND1 level is greater than greater than about 100 ng/mL, 105 ng/mL, 110 ng/mL, 115 ng/mL, 120 ng/mL, 125 ng/mL, 130 ng/mL, 135 ng/mL, 140 ng/mL, 145 ng/mL, 150 ng/mL, 155 ng/mL, 160 ng/mL, 165 ng/mL, 170 ng/mL, 175 ng/mL, 180 ng/mL, 185 ng/mL, 190 ng/mL, 195 ng/ml, 200 mg/mL, 205 ng/mL, 210 ng/mL, 215 ng/mL, 220 ng/mL, 225 ng/mL, 230 ng/mL, 235 ng/mL, 240 ng/mL, 245 ng/mL, or 250 ng/mL.

In another embodiment, the level of SYND1 in the sample is deemed elevated when it is at least about 2-fold to about 17-fold, e.g., about four-fold greater than the normal reference range for SYND1 (e.g., a healthy patient who does not have HSCT or an HSCT patient in the absence of TMA). In some embodiments, the level of SYND1 in the sample is deemed elevated when it is at least three, four, five, six, seven, eight, ten, twelve, or fifteen-fold greater than the normal reference range for SYND1.

### C. Factor Ba

Factor Ba is the fragment of complement factor B. Factor B, a glycosylated protein composed of a single 93,000 Da polypeptide chain, is an essential component of the alternative pathway of complement activation. In the presence of magnesium, factor B binds to C3b. The C3b/B complex can be activated by factor D, a serine protease that circulates as an active trypsin-like serine protease. Cleavage of factor B by factor D causes the release of the Ba fragment (33,000 Da) and leaves the 60,000 Bb fragment bound to C3b. This Ba fragment comes from the N-terminal of factor B and it contains three CCP domains which interact with C3b. The isolated fragment Ba has been reported to have a weak affinity for C3b and to inhibit the interaction of factor B with C3b thus inhibiting the activation of the alternative pathway.

In one embodiment, the normal reference range for Factor Ba for a healthy patient *(e.g.,* a patient who does not have HSCT) is less than about 1000 ng/mL. In another embodiment, the normal reference range for Factor Ba for a healthy patient *(e.g.,* a patient who does not have HSCT) is less than about 600 ng/mL. In another embodiment, the normal reference range for Factor Ba for a healthy patient *(e.g.,* a patient who does not have HSCT) is between about 300 ng/mL and 600 ng/mL. For example, the normal reference range for Factor Ba for a healthy patient *(e.g.,* a patient who does not have HSCT) is about 300 ng/mL, 310 ng/mL, 320 ng/mL, 330 ng/mL, 340 ng/mL, 350 ng/mL, 360 ng/mL, 370 ng/mL, 380 ng/mL, 390 ng/mL, 400 ng/mL, 410 ng/mL, 420 ng/mL, 430 ng/mL, 440 ng/mL, 450 ng/mL, 460 ng/mL, 470 ng/mL, 480 ng/mL, 490 ng/mL, 500 ng/mL, 510 ng/mL, 520 ng/mL, 530 ng/mL, 540 ng/mL, 550 ng/mL, 560 ng/mL, 570 ng/mL, 580 ng/mL, 590 ng/mL, or 600 ng/mL.

In another embodiment, the normal reference range for Factor Ba for a HSCT patient in the absence of TMA is about 500 ng/mL to 800. For example, in one embodiment, a normal reference range for Factor Ba for a HSCT patient in the absence of TMA is about 500 ng/mL, 510 ng/mL, 520 ng/mL, 530 ng/mL, 540 ng/mL, 550 ng/mL, 560 ng/mL, 570 ng/mL, 580 ng/mL, 590 ng/mL, 600 ng/mL, 610 ng/mL, 620 ng/mL, 630 ng/mL, 640 ng/mL, 650 ng/mL, 660 ng/mL, 670 ng/mL, 680 ng/mL, 690 ng/mL, 700 ng/mL, 710 ng/mL, 720 ng/mL, 730 ng/mL, 740 ng/mL, 750 ng/mL, 760 ng/mL, 770 ng/mL, 780 ng/mL, 790 ng/mL, or 800 ng/mL.

In another embodiment, levels of Factor Ba are elevated in HSCT patients who have or are likely to develop HSCT-TMA compared to a normal reference range *(e.g.,* a healthy patient who does not have HSCT or an HSCT patient in the absence of TMA). For example, an elevated Ba level is greater than about 900 ng/mL, 910 ng/mL, 920 ng/mL, 930 ng/mL, 940 ng/mL, 950 ng/mL, 960 ng/mL, 970 ng/mL, 980 ng/mL, 990 ng/mL, 1000 ng/mL, 1010 ng/mL, 1020 ng/mL, 1030 ng/mL, 1040 ng/mL, 1050 ng/mL, 1060 ng/mL, 1070 ng/mL, 1080 ng/mL, 1090 ng/mL, 1100 ng/mL, 1110 ng/mL, 1120 ng/mL, 1130 ng/mL, 1140 ng/mL, 1150 ng/mL, 1160 ng/mL, 1170 ng/mL, 1180 ng/mL, 1190 ng/mL, 1200 ng/mL, 1210 ng/mL, 1220 ng/mL, 1230 ng/mL, 1240 ng/mL, 1250 ng/mL, 1260 ng/mL, 1270 ng/mL, 1280 ng/mL, 1290 ng/mL, 1300 ng/mL, 1310 ng/mL, 1320 ng/mL, 1330 ng/mL, 1340 ng/mL, 1350 ng/mL, 1360 ng/mL, 1370 ng/mL, 1380 ng/mL, 1390 ng/mL, 1400 ng/mL, 1410 ng/mL, 1420 ng/mL, 1430 ng/mL, 1440 ng/mL, 1450 ng/mL, 1460 ng/mL, 1470 ng/mL, 1480 ng/mL, 1490 ng/mL, 1500 ng/mL, 1510 ng/mL, 1520 ng/mL, 1530 ng/mL, 1540 ng/mL, 1550 ng/mL, 1560 ng/mL, 1570 ng/mL, 1580 ng/mL, 1590 ng/mL, 1600 ng/mL, 1610 ng/mL, 1620 ng/mL, 1630 ng/mL, 1640 ng/mL, 1650 ng/mL, 1660 ng/mL, 1670 ng/mL, 1680 ng/mL, 1690 ng/mL, 1700 ng/mL, 1710 ng/mL, 1720 ng/mL, 1730 ng/mL, 1740 ng/mL, 1750 ng/mL, 1760 ng/mL, 1770 ng/mL, 1780 ng/mL, 1790 ng/mL, 1800 ng/mL, 1810 ng/mL, 1820 ng/mL, 1830 ng/mL, 1840 ng/mL, 1850 ng/mL, 1860 ng/mL, 1870 ng/mL, 1880 ng/mL, 1890 ng/mL, 1900 ng/mL, 1910 ng/mL, 1920 ng/mL, 1930 ng/mL, 1940 ng/mL, 1950 ng/mL, 1960 ng/mL, 1970 ng/mL, 1980 ng/mL, 1990 ng/mL, 2000 ng/mL, 2010 ng/mL, 2020 ng/mL, 2030 ng/mL, 2040 ng/mL, 2050 ng/mL, 2060 ng/mL, 2070 ng/mL, 2080 ng/mL, 2090 ng/mL, 2100 ng/mL, 2110 ng/mL, 2120 ng/mL, 2130 ng/mL, 2140 ng/mL, 2150 ng/mL, 2160 ng/mL, 2170 ng/mL, 2180 ng/mL, 2190 ng/mL, 2200 ng/mL, 2210 ng/mL, 2220 ng/mL, 2230 ng/mL, 2240 ng/mL, 2250 ng/mL, 2260 ng/mL, 2270 ng/mL, 2280 ng/mL, 2290 ng/mL, 2300 ng/mL, 2310 ng/mL, 2320 ng/mL, 2330 ng/mL, 2340 ng/mL, 2350 ng/mL, 2360 ng/mL, 2370 ng/mL, 2380 ng/mL, 2390 ng/mL, 2400 ng/mL, 2410 ng/mL, 2420 ng/mL, 2430 ng/mL, 2440 ng/mL, 2450 ng/mL, 2460 ng/mL, 2470 ng/mL, 2480 ng/mL, 2490 ng/mL, or 2500 ng/mL.

In one embodiment, the level of Ba in the sample is deemed elevated when it is at least about 12% higher to about 5-fold, e.g., about two-fold greater than the normal reference range for Factor Ba *(e.g.,* a healthy patient who does not have HSCT or an HSCT patient in the absence of TMA). In some embodiments, the level of Ba in the sample is deemed elevated when it is at least two, three, four, or five-fold greater than the normal reference range for Ba.

### D. Heparan Sulfate Proteoglycan (HSPG)

Heparan sulfate proteoglycans (HSPGs) are glycoproteins, with the common characteristic of containing one or more covalently attached heparan sulfate (HS) chains, a type of glycosaminoglycan (GAG) (see, e.g., Esko et al., Proteoglycans and Sulfated Glycosaminoglycans. In Essentials of glycobiology (ed. Varki A, et al.), pp. 229-248 Cold Spring Harbor Laboratory Press, Cold Spring Harbor, NY). Heparan sulfate proteoglycans are found at the cell surface and in the extracellular matrix, where they interact with a plethora of ligands (see, *e.g.,* Sarrazin et al., Cold Spring Harb Perspect Biol. 2011 Jul; 3(7): a004952). Although few in number, heparan sulfate proteoglycans have profound effects at the cellular, tissue, and organismal level. Cells have a relatively small set of HSPGs (approximately 17) that fall into three groups according to their location: membrane HSPGs, such as syndecans and glycosylphosphatidylinositol-anchored proteoglycans (glypicans), the secreted extracellular matrix HSPGs (agrin, perlecan, type XVIII collagen), and the secretory vesicle proteoglycan, serglycin (see, *e.g.,* Sarrazin *et al.,* 2011).

In one embodiment, the level of HSPG are elevated in HSCT patients who have or are likely to develop HSCT-TMA compared to a normal reference range *(e.g.,* a healthy patient who does not have HSCT or an HSCT patient in the absence of TMA).

In another embodiment, the level of HSPG in the sample is deemed elevated when it is at least about 20% higher to about 2-fold, e.g., about two-fold, three-fold, four-fold, five-fold, six-fold, seven-fold, eight-fold, nine-fold, ten-fold, eleven-fold, twelve-fold, thirteen-fold, fourteen-fold, fifteen-fold, sixteen-fold, seventeen-fold, eighteen-fold, nineteen-fold, or twenty-fold greater than the normal reference range for HSPGs (e.g., a healthy patient who does not have HSCT or an HSCT patient in the absence of TMA).

### E. Biomarker Combination

Partly owing to the enhanced prognostic and diagnostic significance of biomarkers when used in combination, a plurality (e.g., at least 2, 3, or more) biomarkers may be detected and measured in accordance with the disclosure. Such biomarker combinations may be referred herein as signatures.

In an embodiment, the detection step may comprise detecting a biomarker signature comprising the following biomarkers: (a) TM + Ba, optionally together with C5b9; (b) TM + SYND1, optionally together with C5b9; (c) SYND1 + Ba, optionally together with C5b9; (d) TM + Ba, optionally together with HSPG; or (e) TM + SYND1, optionally together with HSPG; (f) SYND1 + Ba, optionally together with HSPG.

In an embodiment, the detection step may comprise detecting a biomarker signature comprising the following biomarkers: TM + SYND1 + Ba, optionally together with C5b9 and/or HSPG.

In an embodiment, the detection step may comprise detecting a biomarker signature comprising TM + SYND1 + Ba + C5b9 + HSPG

### III. Anti-C5 Antibodies

Any suitable anti-C5 antibody, or antigen binding fragment thereof, can be used in the methods described herein. Anti-C5 antibodies (or VH/VL domains derived therefrom) suitable for use in the methods described herein can be generated using methods known in the art. Alternatively, art recognized anti-C5 antibodies can be used. Antibodies that compete for binding to C5 with any of these art recognized antibodies or antibodies described herein can also be used. In some embodiments, anti-C5 antibodies described herein bind to complement component C5 (e.g., human C5) and inhibit the cleavage of C5 into fragments C5a and C5b.

An exemplary anti-C5 antibody is eculizumab. Eculizumab (also known as SOLIRIS^{®}) is an anti-C5 antibody comprising heavy chain CDR1, CDR2 and CDR3 domains having the sequences set forth in SEQ ID NOs: 1, 2, and 3, respectively, and light chain CDR1, CDR2 and CDR3 domains having the sequences set forth in SEQ ID NOs: 4, 5, and 6, respectively. Eculizumab comprises a heavy chain variable region having the amino acid sequence set forth in SEQ ID NO: 7 and a light chain variable region having the amino acid sequence set forth in SEQ ID NO: 8. Eculizumab comprises a heavy chain comprising the amino acid sequence set forth in SEQ ID NO:10 and a light chain having the amino acid sequence set forth in SEQ ID NO:11.

An exemplary anti-C5 antibody is ravulizumab comprising heavy and light chains having the sequences shown in SEQ ID NOs:14 and 11, respectively, or antigen binding fragments and variants thereof. Ravulizumab (also known as ULTOMIRIS^{®}, BNJ441 and ALXN1210) is described in WO2015134894 and US Patent No: 9,079,949, the entire teachings of which are hereby incorporated by reference. The terms ravulizumab, BNJ441, and ALXN1210 may be used interchangeably throughout this document, but all refer to the same antibody. Ravulizumab selectively binds to human complement protein C5, inhibiting its cleavage to C5a and C5b during complement activation. This inhibition prevents the release of the proinflammatory mediator C5a and the formation of the cytolytic pore-forming membrane attack complex (MAC) C5b-9 while preserving the proximal or early components of complement activation (e.g., C3 and C3b) essential for the opsonization of microorganisms and clearance of immune complexes.

The polypeptide sequence of ravulizumab, as accessioned in KEGG DRUG Database (https://www.kegg.jp/entry/D11054), provides that the N-terminal amino acid of variable heavy chain is "X", but the database does not state what X is. The Chemical Abstracts (CAS) for ravulizumab (CAS 1803171-55-2) also provides that N-terminal X is pyroglutamic acid (designated as "chain 1 pyroglutamic acid-1" in the CAS report). Although this information may seem different from the VH sequence of ravulizumab, e.g., a heavy chain variable region polypeptide comprising the amino acid sequence depicted in SEQ ID NO: 12 and/or a heavy chain polypeptide comprising the amino acid sequence depicted in SEQ ID NO:14, there is alignment between patented sequences and the drug database/CAS sequence because it was recognized in the art that N-Terminal Q in polypeptide and/or antibody sequence cyclizes during process development to yield drug product conversion to pyroglutamate (Pryo-Q) near 100%, as disclosed in Liu et al. (J Pharm Sci . 2019 Oct;108(10):3194-3200) https://pubmed.ncbi.nlm.nih.gov/31145921/ and Nguyen et al. (Int. J. Mol. Sci. 2017 Jul 20;18(7):1575) https://www.researchgate.net/figure/Cyclization-reactions-of-N-terminal-glutamine-and-glutamate-residues-in-a-polypeptide_fig4_318926365. Additional information is provided in page 7 and Table 4 of Xu et al. (MAbs, 2019 Feb/Mar;11(2):239-264) and the following referenced publications: (1) Yu et al., "Investigation of N-terminal glutamatecyclization of recombinant monoclonal antibody in formulation development", J. Pharm. Biomed. Anal., 2006, 42, 455-463 and Dick et al., "Determination of the origin of the N-terminal pyro-glutamatevariation in monoclonal antibodies using model peptides", Biotechnol. Bioeng., 2007, 97, 544-553, the disclosures in which are incorporated by reference in their entirety.

In other embodiments, the antibody comprises the heavy and light chain CDRs or variable regions of ravulizumab. Accordingly, in one embodiment, the antibody comprises the CDR1, CDR2 and CDR3 domains of the VH region of ravulizumab having the sequence set forth in SEQ ID NO:12, and the CDR1, CDR2 and CDR3 domains of the VL region of ravulizumab having the sequence set forth in SEQ ID NO:8. In another embodiment, the antibody comprises heavy chain CDR1, CDR2 and CDR3 domains having the sequences set forth in SEQ ID NOs:19, 18 and 3, respectively, and light chain CDR1, CDR2 and CDR3 domains having the sequences set forth in SEQ ID NOs:4, 5 and 6, respectively. In another embodiment, the antibody comprises VH and VL regions having the amino acid sequences set forth in SEQ ID NO:12 and SEQ ID NO:8, respectively.

Another exemplary anti-C5 antibody is antibody BNJ421 comprising heavy and light chains having the sequences shown in SEQ ID NOs:20 and 11, respectively, or antigen binding fragments and variants thereof. BNJ421 (also known as ALXN1211) is described in WO2015134894 and US Patent No.9,079,949, the entire teachings of which are hereby incorporated by reference.

In other embodiments, the antibody comprises the heavy and light chain CDRs or variable regions of BNJ421. Accordingly, in one embodiment, the antibody comprises the CDR1, CDR2 and CDR3 domains of the VH region of BNJ421 having the sequence set forth in SEQ ID NO:12, and the CDR1, CDR2 and CDR3 domains of the VL region of BNJ421 having the sequence set forth in SEQ ID NO:8. In another embodiment, the antibody comprises heavy chain CDR1, CDR2 and CDR3 domains having the sequences set forth in SEQ ID NOs:19, 18 and 3, respectively, and light chain CDR1, CDR2 and CDR3 domains having the sequences set forth in SEQ ID NOs:4, 5 and 6, respectively. In another embodiment, the antibody comprises VH and VL regions having the amino acid sequences set forth in SEQ ID NO:12 and SEQ ID NO:8, respectively.

The exact boundaries of CDRs are defined differently according to different methods. In some embodiments, the positions of the CDRs or framework regions within a light or heavy chain variable domain are as defined by Kabat et al. [(1991) "Sequences of Proteins of Immunological Interest." NIH Publication No. 91-3242, U.S. Department of Health and Human Services, Bethesda, MD]. In such cases, the CDRs can be referred to as "Kabat CDRs" *(e.g.,* "Kabat LCDR2" or "Kabat HCDR1"). In some embodiments, the positions of the CDRs of a light or heavy chain variable region are as defined by Chothia et al. (Nature, 342:877-83, 1989). Accordingly, these regions can be referred to as "Chothia CDRs" *(e.g.,* "Chothia LCDR2" or "Chothia HCDR3"). In some embodiments, the positions of the CDRs of the light and heavy chain variable regions can be defined by a Kabat-Chothia combined definition. In such embodiments, these regions can be referred to as "combined Kabat-Chothia CDRs." Thomas, C. et al. (Mol. Immunol., 33:1389-401, 1996) exemplifies the identification of CDR boundaries according to Kabat and Chothia numbering schemes.

Another exemplary anti-C5 antibody is the 7086 antibody described in US Patent Nos. 8,241,628 and 8,883,158. In one embodiment, the antibody comprises the heavy and light chain CDRs or variable regions of the 7086 antibody (*see* US Patent Nos. 8,241,628 and 8,883,158). In another embodiment, the antibody, or antigen binding fragment thereof, comprises heavy chain CDR1, CDR2 and CDR3 domains having the sequences set forth in SEQ ID NOs:21, 22 and 23, respectively, and light chain CDR1, CDR2 and CDR3 domains having the sequences set forth in SEQ ID NOs:24, 25 and 26, respectively. In another embodiment, the antibody, or antigen binding fragment thereof, comprises the VH region of the 7086 antibody having the sequence set forth in SEQ ID NO:27, and the VL region of the 7086 antibody having the sequence set forth in SEQ ID NO:28.

Another exemplary anti-C5 antibody is the 8110 antibody also described in US Patent Nos. 8,241,628 and 8,883,158. In one embodiment, the antibody comprises the heavy and light chain CDRs or variable regions of the 8110 antibody. In another embodiment, the antibody, or antigen binding fragment thereof, comprises heavy chain CDR1, CDR2 and CDR3 domains having the sequences set forth in SEQ ID NOs:29, 30 and 31, respectively, and light chain CDR1, CDR2 and CDR3 domains having the sequences set forth in SEQ ID NOs:32, 33 and 34, respectively. In another embodiment, the antibody comprises the VH region of the 8110 antibody having the sequence set forth in SEQ ID NO:35, and the VL region of the 8110 antibody having the sequence set forth in SEQ ID NO:36.

Another exemplary anti-C5 antibody is the 305LO5 antibody described in US Patent No. 9,765,135. In one embodiment, the antibody comprises the heavy and light chain CDRs or variable regions of the 305LO5 antibody. In another embodiment, the antibody, or antigen binding fragment thereof, comprises heavy chain CDR1, CDR2 and CDR3 domains having the sequences set forth in SEQ ID NOs:37, 38 and 39, respectively, and light chain CDR1, CDR2 and CDR3 domains having the sequences set forth in SEQ ID NOs:40, 41 and 42, respectively. In another embodiment, the antibody comprises the VH region of the 305LO5 antibody having the sequence set forth in SEQ ID NO:43, and the VL region of the 305LO5 antibody having the sequence set forth in SEQ ID NO:44.

Another exemplary anti-C5 antibody is the SKY59 antibody (Fukuzawa, T. et al., Sci. Rep., 7:1080, 2017). In one embodiment, the antibody comprises the heavy and light chain CDRs or variable regions of the SKY59 antibody. In another embodiment, the antibody, or antigen binding fragment thereof, comprises a heavy chain comprising SEQ ID NO:45 and a light chain comprising SEQ ID NO:46.

In some embodiments, the anti-C5 antibody comprises the heavy and light chain variable regions or heavy and light chains of the REGN3918 antibody (see US Patent No. 10,633,434). In some embodiments, the anti-C5 antibody, or antigen-binding fragment thereof, comprises a heavy chain variable region sequence set forth in SEQ ID NO: 47 and a light chain variable region comprising the sequence set forth in SEQ ID NO: 48. In some embodiments, the anti-C5 antibody, or antigen-binding fragment thereof, comprises a heavy chain sequence set forth in SEQ ID NO: 49 and a light chain sequence set forth in SEQ ID NO: 50.

In some embodiments, the anti-C5 antibody is a biosimilar of eculizumab (SOLIRIS^{®}). For example, in one embodiment, the anti-C5 antibody is, for example, ABP 959 antibody (eculizumab biosimilar manufactured by Amgen Inc., USA), ELIZARIA^{®} (eculizumab biosimilar manufactured by Generium JNC, Russia), SB12 (eculizumab biosimilar manufactured by Samsung Bioepis, Incheon, South Korea), ISU305 (eculizumab biosimilar from ISU Abxis, South Korea), ABLYZE^{®} (eculizumab biosimilar from CinnaGen, Iran), BCD 148 (eculizumab biosimilar from Biocad Medical, Canada), tesidolumab (manufactured by Novartis), Crovalimab (manufactured by Roche), CAN106 (manufactured by CANBridge Pharmaceuticals, China) or Pozelimab (manufactured by Regeneron).

In some embodiments, an anti-C5 antibody described herein comprises a heavy chain CDR1 comprising, or consisting of, the following amino acid sequence: GHIFSNYWIQ (SEQ ID NO: 19). In some embodiments, an anti-C5 antibody described herein comprises a heavy chain CDR2 comprising, or consisting of, the following amino acid sequence: EILPGSGHTEYTENFKD (SEQ ID NO:18). In some embodiments, an anti-C5 antibody described herein comprises a heavy chain variable region comprising the following amino acid sequence:



 (SEQ ID NO:12).

In some embodiments, an anti-C5 antibody described herein comprises a light chain variable region comprising the following amino acid sequence:



 (SEQ ID NO:8).

An anti-C5 antibody described herein can, in some embodiments, comprise a variant human Fc constant region that binds to human neonatal Fc receptor (FcRn) with greater affinity than that of the native human Fc constant region from which the variant human Fc constant region was derived. The Fc constant region can, for example, comprise one or more (e.g., two, three, four, five, six, seven, or eight or more) amino acid substitutions relative to the native human Fc constant region from which the variant human Fc constant region was derived. The substitutions can increase the binding affinity of an IgG antibody containing the variant Fc constant region to FcRn at pH 6.0, while maintaining the pH dependence of the interaction. Methods for testing whether one or more substitutions in the Fc constant region of an antibody increase the affinity of the Fc constant region for FcRn at pH 6.0 (while maintaining pH dependence of the interaction) are known in the art and exemplified in the working examples. See, e.g., WO2015134894 and US Patent No.9,079949 the disclosures of each of which are incorporated herein by reference in their entirety.

Substitutions that enhance the binding affinity of an antibody Fc constant region for FcRn are known in the art and include, *e.g.,* (1) the M252Y/S254T/T256E triple substitution (Dall'Acqua, W. et al., J. Biol. Chem., 281:23514-24, 2006); (2) the M428L or T250Q/M428L substitutions (Hinton, P. et al., J. Biol. Chem., 279:6213-6, 2004; Hinton, P. et al., J. Immunol., 176:346-56, 2006); and (3) the N434A or T307/E380A/N434A substitutions (Petkova, S. et al., Int. Immunol., 18:1759-69, 2006). The additional substitution pairings: P257I/Q311I, P257I/N434H and D376V/N434H (Datta-Mannan, A. et al., J. Biol. Chem., 282:1709-17, 2007), the disclosures of each of which are incorporated herein by reference in their entirety.

In some embodiments, the variant constant region has a substitution at EU amino acid posaition 255 for valine. In some embodiments, the variant constant region has a substitution at EU amino acid position 309 for asparagine. In some embodiments, the variant constant region has a substitution at EU amino acid position 312 for isoleucine. In some embodiments, the variant constant region has a substitution at EU amino acid position 386.

In some embodiments, the variant Fc constant region comprises no more than 30 *(e.g.,* no more than 29, 28, 27, 26, 25, 24, 23, 22, 21, 20, 19, 18, 17, 16, 15, 14, 13, 12, 11, 10, 9, 8, 7, 6, 5, 4, 3 or 2) amino acid substitutions, insertions, or deletions relative to the native constant region from which it was derived. In some embodiments, the variant Fc constant region comprises one or more amino acid substitutions selected from the group consisting of: M252Y, S254T, T256E, N434S, M428L, V259I, T250I and V308F. In some embodiments, the variant human Fc constant region comprises a methionine at position 428 and an asparagine at position 434 of a native human IgG Fc constant region, each in EU numbering. In some embodiments, the variant Fc constant region comprises a 428L/434S double substitution as described in, e.g., U.S. Patent No. 8,088,376.

In some embodiments the precise location of these mutations may be shifted from the native human Fc constant region position due to antibody engineering. For example, the 428L/434S double substitution when used in a IgG2/4 chimeric Fc may correspond to 429L and 435S as in the M429L and N435S variants found in ravulizumab and described in US Patent Number 9,079,949 the disclosure of which is incorporated herein by reference in its entirety.

In some embodiments, the variant constant region comprises a substitution at amino acid position 237, 238, 239, 248, 250, 252, 254, 255, 256, 257, 258, 265, 270, 286, 289, 297, 298, 303, 305, 307, 308, 309, 311, 312, 314, 315, 317, 325, 332, 334, 360, 376, 380, 382, 384, 385, 386, 387, 389, 424, 428, 433, 434 or 436 (EU numbering) relative to the native human Fc constant region. In some embodiments, the substitution is selected from the group consisting of: methionine for glycine at position 237; alanine for proline at position 238; lysine for serine at position 239; isoleucine for lysine at position 248; alanine, phenylalanine, isoleucine, methionine, glutamine, serine, valine, tryptophan, or tyrosine for threonine at position 250; phenylalanine, tryptophan, or tyrosine for methionine at position 252; threonine for serine at position 254; glutamic acid for arginine at position 255; aspartic acid, glutamic acid, or glutamine for threonine at position 256; alanine, glycine, isoleucine, leucine, methionine, asparagine, serine, threonine, or valine for proline at position 257; histidine for glutamic acid at position 258; alanine for aspartic acid at position 265; phenylalanine for aspartic acid at position 270; alanine, or glutamic acid for asparagine at position 286; histidine for threonine at position 289; alanine for asparagine at position 297; glycine for serine at position 298; alanine for valine at position 303; alanine for valine at position 305; alanine, aspartic acid, phenylalanine, glycine, histidine, isoleucine, lysine, leucine, methionine, asparagine, proline, glutamine, arginine, serine, valine, tryptophan, or tyrosine for threonine at position 307; alanine, phenylalanine, isoleucine, leucine, methionine, proline, glutamine, or threonine for valine at position 308; alanine, aspartic acid, glutamic acid, proline, or arginine for leucine or valine at position 309; alanine, histidine, or isoleucine for glutamine at position 311; alanine or histidine for aspartic acid at position 312;lysine or arginine for leucine at position 314; alanine or histidine for asparagine at position 315; alanine for lysine at position 317; glycine for asparagine at position 325; valine for isoleucine at position 332; leucine for lysine at position 334; histidine for lysine at position 360; alanine for aspartic acid at position 376; alanine for glutamic acid at position 380; alanine for glutamic acid at position 382; alanine for asparagine or serine at position 384; aspartic acid or histidine for glycine at position 385; proline for glutamine at position 386; glutamic acid for proline at position 387; alanine or serine for asparagine at position 389; alanine for serine at position 424; alanine, aspartic acid, phenylalanine, glycine, histidine, isoleucine, lysine, leucine, asparagine, proline, glutamine, serine, threonine, valine, tryptophan, or tyrosine for methionine at position 428; lysine for histidine at position 433; alanine, phenylalanine, histidine, serine, tryptophan, or tyrosine for asparagine at position 434; and histidine for tyrosine or phenylalanine at position 436, all in EU numbering.

Suitable anti-C5 antibodies for use in the methods described herein, in some embodiments, comprise a heavy chain polypeptide comprising the amino acid sequence set forth in SEQ ID NO:14 and/or a light chain polypeptide comprising the amino acid sequence set forth in SEQ ID NO:11. Alternatively, the anti-C5 antibodies for use in the methods described herein, in some embodiments, comprise a heavy chain polypeptide comprising the amino acid sequence set forth in SEQ ID NO:20 and/or a light chain polypeptide comprising the amino acid sequence set forth in SEQ ID NO:11.

In one embodiment, the antibody binds to C5 at pH 7.4 and 25°C (and, otherwise, under physiologic conditions) with an affinity dissociation constant (K_{D}) that is at least 0.1 *(e.g.,* at least 0.15, 0.175, 0.2, 0.25, 0.275, 0.3, 0.325, 0.35, 0.375, 0.4, 0.425, 0.45, 0.475, 0.5, 0.525, 0.55, 0.575, 0.6, 0.625, 0.65, 0.675, 0.7, 0.725, 0.75, 0.775, 0.8, 0.825, 0.85, 0.875, 0.9, 0.925, 0.95, or 0.975) nM. In one embodiment, the antibody binds to C5 at pH 7.4 and 25°C (and, otherwise, under physiologic conditions) with an affinity dissociation constant (Ko) that is about 0.5 nM. In some embodiments, the K_{D} of the anti-C5 antibody, or antigen binding fragment thereof, is no greater than 1 *(e.g.,* no greater than 0.9, 0.8, 0.7, 0.6, 0.5, 0.4, 0.3, or 0.2) nM. In some embodiments, the antibody binds to C5 at pH 6.0 and 25°C (and, otherwise, under physiologic conditions) with a K_{D} that is about 22 nM.

In other embodiments, the [(K_{D} of the antibody for C5 at pH 6.0 at 25°C)/(K_{D} of the antibody for C5 at pH 7.4 at 25C)] is greater than 21 (e.g., greater than 22, 23, 24, 25, 26, 27, 28, 29, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 350, 400, 450, 500, 600, 700, 800, 900, 1000, 1500, 2000, 2500, 3000, 3500, 4000, 4500, 5000, 5500, 6000, 6500, 7000, 7500 or 8000)

Methods for determining whether an antibody binds to a protein antigen and/or the affinity for an antibody to a protein antigen are known in the art. The binding of an antibody to a protein antigen, for example, can be detected and/or quantified using a variety of techniques such as, but not limited to, Western blot, dot blot, surface plasmon resonance (SPR) detection (e.g., BIAcore system; Pharmacia Biosensor AB, Uppsala, Sweden and Piscataway, N.J.), or enzyme-linked immunosorbent assay (ELISA; Benny K. C. Lo (2004) "Antibody Engineering: Methods and Protocols," Humana Press (ISBN: 1588290921); Johne, B. et al., J. Immunol. Meth., 160:191-8, 1993; Jönsson, U. et al., Ann. Biol. Clin., 51:19-26, 1993; Jönsson, U. et al., Biotechniques, 11:620-7, 1991). In addition, methods for measuring the affinity (e.g., dissociation and association constants) are set forth in the working examples.

As used herein, the term "kₐ" refers to the rate constant for association of an antibody to an antigen. The term "k_{d}" refers to the rate constant for dissociation of an antibody from the antibody/antigen complex. And the term "K_{D}" refers to the equilibrium dissociation constant of an antibody-antigen interaction. The equilibrium dissociation constant is deduced from the ratio of the kinetic rate constants, K_{D} = kₐ/k_{d}. Such determinations can be measured, for example, at 25C or 37C (see the working examples). The kinetics of antibody binding to human C5 can be determined, for example, at pH 8.0, 7.4, 7.0, 6.5 and 6.0 via SPR on a BIAcore 3000 instrument using an anti-Fc capture method to immobilize the antibody.

In one embodiment, the anti-C5 antibody, or antigen binding fragment thereof, blocks the cleavage of C5 into C5a and C5b. Through this blocking effect, for example, the pro-inflammatory effects of C5a and the generation of the C5b-9 membrane attack complex (MAC) at the surface of a cell are inhibited.

Methods for determining whether a particular antibody described herein inhibits C5 cleavage are known in the art. Inhibition of human complement component C5 can reduce the cell-lysing ability of complement in a subject's body fluids. Such reductions of the cell-lysing ability of complement present in the body fluid(s) can be measured by methods known in the art such as, for example, by a conventional hemolytic assay such as the hemolysis assay (Kabat and Mayer (eds.), "Experimental Immunochemistry, 2nd Edition," 135-240, Springfield, IL, CC Thomas (1961), pages 135-139), or a conventional variation of that assay such as the chicken erythrocyte hemolysis method (Hillmen, P. et al., N. Engl. J. Med. , 350:552-9, 2004). Methods for determining whether a candidate compound inhibits the cleavage of human C5 into forms C5a and C5b are known in the art (Evans, M. et al., Mol. Immunol., 32:1183-95, 1995). The concentration and/or physiologic activity of C5a and C5b in a body fluid can be measured, for example, by methods known in the art. For C5b, hemolytic assays or assays for soluble C5b-9 as discussed herein can be used. Other assays known in the art can also be used. Using assays of these or other suitable types, candidate agents capable of inhibiting human complement component C5 can be screened.

Immunological techniques such as, but not limited to, ELISA can be used to measure the protein concentration of C5 and/or its split products to determine the ability of an anti-C5 antibody, or antigen binding fragment thereof, to inhibit conversion of C5 into biologically active products. In some embodiments, C5a generation is measured. In some embodiments, C5b-9 neoepitope-specific antibodies are used to detect MAC formation.

Hemolytic assays can be used to determine the inhibitory activity of an anti-C5 antibody, or antigen binding fragment thereof, on complement activation. To determine the effect of an anti-C5 antibody, or antigen binding fragment thereof, on classical complement pathway-mediated hemolysis in a serum test solution *in vitro,* for example, sheep erythrocytes coated with hemolysin or chicken erythrocytes sensitized with anti-chicken erythrocyte antibody are used as target cells. The percentage of lysis is normalized by considering 100% lysis equal to the lysis occurring in the absence of the inhibitor. In some embodiments, the classical complement pathway is activated by a human IgM antibody, for example, as utilized in the Wieslab^{®} Classical Pathway Complement Kit (Wieslab^{®} COMPL CP310, Euro-Diagnostica, Sweden). Briefly, the test serum is incubated with an anti-C5 antibody, or antigen binding fragment thereof, in the presence of a human IgM antibody. The amount of C5b-9 that is generated is measured by contacting the mixture with an enzyme conjugated anti-C5b-9 antibody and a fluorogenic substrate and measuring the absorbance at the appropriate wavelength. As a control, the test serum is incubated in the absence of the anti-C5 antibody, or antigen binding fragment thereof. In some embodiments, the test serum is a C5-deficient serum reconstituted with a C5 polypeptide.

To determine the effect of an anti-C5 antibody, or antigen binding fragment thereof, on alternative pathway-mediated hemolysis, unsensitized rabbit or guinea pig erythrocytes can be used as the target cells. In some embodiments, the serum test solution is a C5-deficient serum reconstituted with a C5 polypeptide. The percentage of lysis is normalized by considering 100% lysis equal to the lysis occurring in the absence of the inhibitor. In some embodiments, the alternative complement pathway is activated by lipopolysaccharide molecules, for example, as utilized in the Wieslab^{®} Alternative Pathway Complement Kit (Wieslab^{®} COMPL AP330, Euro-Diagnostica, Sweden). Briefly, the test serum is incubated with an anti-C5 antibody, or antigen binding fragment thereof, in the presence of lipopolysaccharide. The amount of C5b-9 that is generated is measured by contacting the mixture with an enzyme conjugated anti-C5b-9 antibody and a fluorogenic substrate and measuring the fluorescence at the appropriate wavelength. As a control, the test serum is incubated in the absence of the anti-C5 antibody, or antigen binding fragment thereof.

In some embodiments, C5 activity, or inhibition thereof, is quantified using a CH50eq assay. The CH50eq assay is a method for measuring the total classical complement activity in serum. This test is a lytic assay, which uses antibody-sensitized erythrocytes as the activator of the classical complement pathway and various dilutions of the test serum to determine the amount required to give 50% lysis (CH50). The percent hemolysis can be determined, for example, using a spectrophotometer. The CH50eq assay provides an indirect measure of terminal complement complex (TCC) formation, since the TCC themselves are directly responsible for the hemolysis that is measured. The assay is known and commonly practiced by those of skill in the art. Briefly, to activate the classical complement pathway, undiluted serum samples (e.g., reconstituted human serum samples) are added to microassay wells containing the antibody-sensitized erythrocytes to thereby generate TCC. Next, the activated sera are diluted in microassay wells, which are coated with a capture reagent (e.g., an antibody that binds to one or more components of the TCC). The TCC present in the activated samples bind to the monoclonal antibodies coating the surface of the microassay wells. The wells are washed and to each well is added a detection reagent that is detectably labeled and recognizes the bound TCC. The detectable label can be, e.g., a fluorescent label or an enzymatic label. The assay results are expressed in CH50 unit equivalents per milliliter (CH50 U Eq/mL).

Inhibition, e.g., as it pertains to terminal complement activity, includes at least a 5 *(e.g.,* at least a 6, 7, 8, 9, 10, 15, 20, 25, 30, 35, 40, 45, 50, 55 or 60) % decrease in the activity of terminal complement in, *e.g.,* a hemolytic assay or CH50eq assay as compared to the effect of a control antibody (or antigen-binding fragment thereof) under similar conditions and at an equimolar concentration. Substantial inhibition, as used herein, refers to inhibition of a given activity *(e.g.,* terminal complement activity) of at least 40 *(e.g.,* at least 45, 50, 55, 60, 65, 70, 75, 80, 85, 90, or 95 or greater) %. In some embodiments, an anti-C5 antibody described herein contains one or more amino acid substitutions relative to the CDRs of eculizumab *(i.e.,* SEQ ID NOs:1-6), yet retains at least 30 *(e.g.,* at least 31, 32, 33, 34, 35, 36, 37, 38, 39, 40, 41, 42, 43, 44, 45, 46, 47, 48, 49, 50, 55, 60, 65, 70, 75, 80, 85, 90 or 95) % of the complement inhibitory activity of eculizumab in a hemolytic assay or CH50eq assay.

An anti-C5 antibody described herein has a serum half-life in humans that is at least *20 (e.g.,* at least 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54 or 55) days. In another embodiment, the anti-C5 antibody described herein has a serum half-life in humans that is at least 40 days. In another embodiment, the anti-C5 antibody described herein has a serum half-life in humans that is approximately 43 days. In another embodiment, the anti-C5 antibody described herein has a serum half-life in humans that is between 39-48 days. Methods for measuring the serum half-life of an antibody are known in the art. In some embodiments, an anti-C5 antibody, or antigen binding fragment thereof, described herein has a serum half-life that is at least 20 *(e.g.,* at least 30, 35, 40, 45, 50, 55, 60, 65, 70, 75, 80, 85, 90, 95, 100, 125, 150, 175, 200, 250, 300, 400 or 500) % greater than the serum half-life of eculizumab, e.g., as measured in one of the mouse model systems described in the working examples (e.g., the C5-deficient/NOD/scid mouse or hFcRn transgenic mouse model system).

In one embodiment, the antibody competes for binding with, and/or binds to the same epitope on C5 as an antibody described herein. The term "binds to the same epitope" with reference to two or more antibodies means that the antibodies bind to the same segment of amino acid residues, as determined by a given method. Techniques for determining whether antibodies bind to the same epitope on C5 with an antibody described herein include, for example, epitope mapping methods, such as, x-ray analyses of crystals of antigen: antibody complexes, and hydrogen/deuterium exchange mass spectrometry (HDX-MS). Other methods monitor the binding of the antibody to peptide antigen fragments or mutated variations of the antigen where loss of binding due to a modification of an amino acid residue within the antigen sequence is often considered an indication of an epitope component. In addition, computational combinatorial methods for epitope mapping can also be used. These methods rely on the ability of the antibody of interest to affinity isolate specific short peptides from combinatorial phage display peptide libraries. Antibodies having the same VH and VL or the same CDR1, CDR2 and CDR3 sequences are expected to bind to the same epitope.

Antibodies that "compete with another antibody for binding to a target" refer to antibodies that inhibit (partially or completely) the binding of the other antibody to the target. Whether two antibodies compete with each other for binding to a target, *i.e.,* whether and to what extent one antibody inhibits the binding of the other antibody to a target, may be determined using known competition experiments. In certain embodiments, an antibody competes with, and inhibits binding of another antibody to a target by at least 10%, 20%, 30%, 40%, 50%, 60%, 70%, 80%, 90% or 100%. The level of inhibition or competition may be different depending on which antibody is the "blocking antibody" *(i.e.,* the antibody that is incubated first with the target). Competing antibodies can bind to, for example, the same epitope, an overlapping epitope or to adjacent epitopes (e.g., as evidenced by steric hindrance).

The antibody or antigen-binding fragment thereof may include a humanized antibody, a recombinant antibody, a diabody, a chimerized or chimeric antibody, a monoclonal antibody, a deimmunized antibody, a fully human antibody, a single chain antibody, an Fv fragment, an Fd fragment, an Fab fragment, an Fab' fragment, an F(ab')₂ fragment, or a combination thereof.

The monoclonal antibodies disclosed herein can be of any isotype. The monoclonal antibody can be, for example, an IgM or an IgG antibody, such as IgG1 or an IgG2. The class of an antibody that immunospecifically binds C5b-9 can be switched with another (for example, IgG can be switched to IgM), according to well-known procedures. Class switching can also be used to convert one IgG subclass to another, such as from IgG1 to IgG2.

The antibodies of the present invention may be monovalent, bivalent, trivalent or multivalent. For example, monovalent scFvs can be multimerized either chemically or by association with another protein or substance. An scFv that is fused to a hexahistidine tag (SEQ ID NO: 51) or a Flag tag can be multimerized using Ni-NTA agarose (Qiagen) or using anti-Flag antibodies (Stratagene, Inc.).

The antibodies of the present invention may be monospecific, bispecific, trispecific or of greater multispecificity. Multispecific antibodies may be specific for different epitopes of C5b-9, or fragment thereof, and a heterologous epitope, such as a heterologous polypeptide or solid support material. *See, e.g.,* WO 93/17715; WO 92/08802; WO 91/00360; WO 92/05793; Tutt et al, J. Immunol. 147:60-69 (1991); U.S. Patent Nos. 4,474,893; 4,714,681; 4,925,648; 5,573,920; 5,601,819; Kostelny et al. J. Immunol. 148:1547-1553 (1992).

Anti-C5 antibodies, or antigen-binding fragments thereof described herein, used in the methods described herein can be generated using a variety of art-recognized techniques. Monoclonal antibodies can be obtained by various techniques familiar to those skilled in the art. Briefly, spleen cells from an animal immunized with a desired antigen are immortalized, commonly by fusion with a myeloma cell (Köhler, G. & Milstein, C., Eur. J. Immunol., 6:511-9, 1976)). Methods of immortalization include transformation with Epstein Barr Virus, oncogenes, or retroviruses or other methods known in the art. Colonies arising from single immortalized cells are screened for production of antibodies of the desired specificity and affinity for the antigen, and yield of the monoclonal antibodies produced by such cells may be enhanced by various techniques, including injection into the peritoneal cavity of a vertebrate host. Alternatively, one may isolate DNA sequences that encode a monoclonal antibody or a binding fragment thereof by screening a DNA library from human B cells (Huse, W. et al., Science, 246:1275-81, 1989).

Antibodies that may be used in the methods described herein (including scFvs and other molecules comprising, or alternatively consisting of antibody fragments or variants of the invention) can be produced by any method known in the art for the synthesis of antibodies, in particular, by chemical synthesis or preferably, by recombinant expression techniques. Greenfield (Ed.) (2014) "Antibodies: A Laboratory Manual", Cold Spring Harbor Laboratory: Cold Spring Harbor, N.Y.

Single chain Fvs (scFvs) that immunospecifically bind to the biomarkers of the disclosure (e.g., TM, SYND1, Ba, or fragments thereof) may be generated using phage display methods known in the art. In phage display methods, functional antibody domains are displayed on the surface of phage particles which carry the polynucleotide sequences encoding them. Examples of phage display methods that can be used to make the antibodies of the present invention include, but are not limited to, those disclosed in Brinkman et al., J. Immunol. Methods 182:41-50 (1995); Ames et al., J. Immunol. Methods 184:177-186 (1995); Kettleborough et al. Eur. J. Immunol. 24:952-958 (1994); Persic et al., Gene 187 9-18 (1997); Burton et al. Advances in Immunology 57:191-280(1994); WO 91/10737; WO 92/01047; WO 92/18619; WO 93/11236; WO 95/15982; WO 95/20401; WO97/13844; and U.S. Patent Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108.

As described in the above references, after phage selection, the antibody coding regions from the phage can be isolated and used to generate whole antibodies, including human or humanized antibodies, or any other desired antigen binding fragment, and expressed in any desired host, including mammalian cells, insect cells, plant cells, yeast, and bacteria, e.g., as described below. Techniques to recombinantly produce Fab, Fab' and F(ab')2 fragments can also be employed using methods known in the art such as those disclosed in WO 92/22324; Mullinax et al., BioTechniques 12(6):864-869 (1992); and Sawai et al., AJRI 34:26-34 (1995).

To generate whole antibodies, PCR primers including VH or VL nucleotide sequences, a restriction site, and a flanking sequence to protect the restriction site can be used to amplify the VH or VL sequences in scFv clones. Utilizing cloning techniques known to those of skill in the art, the PCR amplified VH domains can be cloned into vectors expressing a VH constant region, e.g., the human gamma 4 constant region, and the PCR amplified VL domains can be cloned into vectors expressing a VL constant region, e.g., human kappa or lambda constant regions.

Once an antibody that may be used in the methods described herein has been synthesized or recombinantly expressed, it may be purified by any method known in the art for purification of an immunoglobulin molecule, or more generally, a protein molecule, such as, for example, by chromatography (e.g., ion exchange, affinity, particularly by affinity for the specific antigen after Protein A, and sizing column chromatography), centrifugation, differential solubility, or by any other standard technique for the purification of proteins.

Vectors comprise elements that facilitate manipulation for the expression of a foreign protein within the target host cell. Conveniently, manipulation of sequences and production of DNA for transformation is first performed in a bacterial host (*e.g., E. coli*) and usually vectors will include sequences to facilitate such manipulations, including a bacterial origin of replication and appropriate bacterial selection marker. Selection markers encode proteins necessary for the survival or growth of transformed host cells grown in a selective culture medium. Host cells not transformed with the vector containing the selection gene will not survive in the culture medium.

The host cells used to express the anti-sC5b-9 antibodies may be either a bacterial cell such as *E. coli,* yeast (*e.g., S. cerevisiae*)*,* or a eukaryotic cell *(e.g.,* a mammalian cell line). Well-defined cell types for this purpose, such as a myeloma cell, 3T3, HeLa, C6A2780, Vero, MOCK II, Chinese hamster ovary (CHO), Sf9, Sf21, COS, NSO, or HEK293, may be used.

Antibodies binding to biomarkers may be screened using any known methods, e.g., binding assays. In a representative method, target biomarkers or antigenic epitope thereof are expressed in standard cells and antibodies are panned using selection techniques known in the art. Antibodies may be ranked, e.g., based on binding affinities, for example, a dissociation constant (K_{d}) of at least 10⁻⁶ M; preferably 10⁻⁸ M; and especially 10⁻¹⁰ M. Here, K_{d} values may be determined using standard binding assays.

The various embodiments of the present disclosure are described in detail in the following non-limiting and representative examples.

In the Examples section and elsewhere, representative types of antibodies which are useful in carrying out various embodiments of the disclosure are provided, e.g., with information on the particular vendor and/or catalog number. It should be understood that the disclosure is not limited to the exemplary embodiments which utilize antibody detection regents from a particular vendor/manufacturer. Antibodies against the biomarkers/analytes of the disclosure can be obtained from any manufacturer, including, e.g., Thermo Fisher Catalog # MA5-24214 for anti-human TM antibody; Catalog# 12-1389-42 for anti-human SYND1 antibody; and MA5-28083 for anti-human complement Factor Ba antibody (all from Thermo Scientific, Waltham, MA). Antibodies can also be purchased from Biolegend (San Diego, CA), Southern Biotech (Birmingham, AL), United States Biological (USB; Salem, MA), Lifespan Biosciences (LSBIO; Seattle, WA), Abeam (Cambridge, United Kingdom), Cell Signaling Technology (Danvers, MA), and Sigma-Aldrich (St. Louis, MO). Conventional techniques, e.g., immunization of a mammal such as a mouse or rabbit and/or hybridoma technology, may also be used to generate antibodies or anti-sera of interest.

Also provided herein are compositions comprising an anti-C5 antibody (or antigen binding fragment thereof). The compositions can be formulated as a pharmaceutical solution, *e.g.,* for administration to a subject for the treatment of HSCT-TMA. The pharmaceutical compositions generally include a pharmaceutically acceptable carrier. As used herein, a "pharmaceutically acceptable carrier" refers to, and includes, any and all solvents, dispersion media, coatings, antibacterial and antifungal agents, isotonic and absorption delaying agents, and the like that are physiologically compatible. The compositions can include a pharmaceutically acceptable salt, *e.g.,* an acid addition salt or a base addition salt, sugars, carbohydrates, polyols and/or tonicity modifiers.

The compositions can be formulated according to standard methods. Pharmaceutical formulation is an established art (see, for example, Gennaro (2000) "Remington: The Science and Practice of Pharmacy," 20th Edition, Lippincott, Williams & Wilkins (ISBN: 0683306472); Ansel et al. (1999) "Pharmaceutical Dosage Forms and Drug Delivery Systems," 7th Edition, Lippincott Williams & Wilkins Publishers (ISBN: 0683305727); and Kibbe (2000) "Handbook of Pharmaceutical Excipients American Pharmaceutical Association," 3rd Edition (ISBN: 091733096X)). In some embodiments, a composition can be formulated, for example, as a buffered solution at a suitable concentration and suitable for storage at 2-8C *(e.g.,* 4C). In some embodiments, a composition can be formulated for storage at a temperature below 0C *(e.g.,* -20C or -80C). In some embodiments, the composition can be formulated for storage for up to 2 years *(e.g.,* 1 month, 2 months, 3 months, 4 months, 5 months, 6 months, 7 months, 8 months, 9 months, 10 months, 11 months, 1 year, 1½ years or 2 years) at 2-8C *(e.g.,* 4C). Thus, in some embodiments, the compositions described herein are stable in storage for at least 1 year at 2-8C *(e.g.,* 4C).

The pharmaceutical compositions can be in a variety of forms. These forms include, *e.g.,* liquid, semi-solid and solid dosage forms, such as liquid solutions *(e.g.,* injectable and infusible solutions), dispersions or suspensions, tablets, pills, powders, liposomes and suppositories. The preferred form depends, in part, on the intended mode of administration and therapeutic application. Compositions containing a composition intended for systemic or local delivery, for example, can be in the form of injectable or infusible solutions. Accordingly, the compositions can be formulated for administration by a parenteral mode (e.g., intravenous, subcutaneous, intraperitoneal, or intramuscular injection). "Parenteral administration," "administered parenterally" and other grammatically equivalent phrases, as used herein, refer to modes of administration other than enteral and topical administration, usually by injection, and include, without limitation, intravenous, intranasal, intraocular, pulmonary, intramuscular, intraarterial, intrathecal, intracapsular, intraorbital, intracardiac, intradermal, intrapulmonary, intraperitoneal, transtracheal, subcutaneous, subcuticular, intraarticular, subcapsular, subarachnoid, intraspinal, epidural, intracerebral, intracranial, intracarotid and intrasternal injection and infusion.

### IV. Anti-Complement Factor B Antibodies

In another aspect, an anticomplement Factor B antibody is used in the methods described herein. An anti-CFB antibody refers to an antibody that inhibits: (i) the expression, or proper intracellular trafficking or secretion by a cell, of a complement factor B protein; (ii) the activity of factor B cleavage fragments Ba or Bb *(e.g.,* the binding of Bb to its complement factor C3b); (iii) the proper intracellular trafficking of, or secretion by a cell, of a complement factor B; or (v) the stability of factor B protein or the mRNA encoding factor B protein. Inhibition of complement factor B protein expression includes: inhibition of transcription of a gene encoding a human complement factor B protein; increased degradation of an mRNA encoding a complement factor B protein; inhibition of translation of an mRNA encoding a human complement factor B; increased degradation of a human complement factor B protein; inhibition of proper processing of a pre-pro human complement factor B protein; or inhibition of proper trafficking or secretion by a cell of a human complement factor B protein. Methods for determining whether a candidate antibody is an inhibitor of human complement factor B are known in the art and described herein.

### V. Biological Samples

Suitable biological samples for use in the methods described herein include, *e.g.,* any biological fluid. A biological sample can be, for example, a specimen obtained from a subject *(e.g.,* a mammal, such as a human) or can be derived from such a subject. A biological sample can also be a biological fluid such as urine, whole blood, or a fraction thereof (e.g., plasma or serum), saliva, semen, sputum, cerebrospinal fluid, tears, or mucus. A biological sample can be further fractionated, if desired, to a fraction containing particular analytes (e.g., proteins) of interest. For example, a whole blood sample can be fractionated into serum or into fractions containing particular types of proteins. If desired, a biological sample can be a combination of different biological samples from a subject such as a combination of two different fluids.

Biological samples suitable for the invention may be fresh or frozen samples collected from a subject, or archival samples with known diagnosis, treatment and/or outcome history. The biological samples can be obtained from a subject, *e.g.,* a subject having, suspected of having, or at risk of developing, a complement-associated disorder such as HSCT-TMA. Any suitable methods for obtaining the biological samples can be employed, although exemplary methods include, e.g., phlebotomy, swab (e.g., buccal swab), lavage, or fine needle aspirate biopsy procedure. Biological samples can also be obtained from bone marrow.

In some embodiments, a protein extract may be prepared from a biological sample. In some embodiments, a protein extract contains the total protein content. Methods of protein extraction are well known in the art. See, *e.g.,* Roe (2001) "Protein Purification Techniques: A Practical Approach", 2nd Edition, Oxford University Press. Numerous different and versatile kits can be used to extract proteins from bodily fluids and tissues, and are commercially available from, for example, BioRad Laboratories (Hercules, CA), BD Biosciences Clontech (Mountain View, CA), Chemicon International, Inc. (Temecula, CA), Calbiochem (San Diego, CA), Pierce Biotechnology (Rockford, IL), and Invitrogen Corp. (Carlsbad, CA).

Methods for obtaining and/or storing samples that preserve the activity or integrity of cells in the biological sample are well known to those skilled in the art. For example, a biological sample can be further contacted with one or more additional agents, such as appropriate buffers and/or inhibitors, including protease inhibitors, the agents meant to preserve or minimize changes (e.g., changes in osmolarity or pH) in protein structure. Such inhibitors include, for example, chelators such as ethylenediamine tetraacetic acid (EDTA), ethylene glycol tetraacetic acid (EGTA), protease inhibitors such as phenylmethylsulfonyl fluoride (PMSF), aprotinin, and leupeptin. Appropriate buffers and conditions for storing or otherwise manipulating whole cells are described in, e.g., Pollard and Walker (1997), "Basic Cell Culture Protocols," volume 75 of Methods in molecular biology, Humana Press; Masters (2000) "Animal cell culture: a practical approach," volume 232 of Practical approach series, Oxford University Press; and Jones (1996) "Human cell culture protocols," volume 2 of Methods in molecular medicine, Humana Press.

A sample also can be processed to eliminate or minimize the presence of interfering substances. For example, a biological sample can be fractionated or purified to remove one or more materials (e.g., cells) that are not of interest. Methods of fractionating or purifying a biological sample include, but are not limited to, flow cytometry, fluorescence activated cell sorting, and sedimentation.

In embodiments of the disclosure, the biomarkers may be detected using an array. For example, the array may be a protein chip where each address of the array is a well of an assay plate. Each address of the array may be a particle (e.g., a bead) having immobilized thereupon a binding agent.

Measuring protein expression levels in a biological sample may be performed by any suitable method. *See, e.g.,* Greenfield (Ed.) (2014) "Antibodies: A Laboratory Manual", Cold Spring Harbor Laboratory: Cold Spring Harbor, N.Y. In general, protein levels are determined by contacting a biological sample obtained from a subject with binding agents for the biomarker proteins; detecting, in the sample (e.g., the biological fluid), the levels of one or more of the biomarker proteins that bind to the binding agents; and comparing the levels of one or more of the biomarker proteins in the sample with the levels of the corresponding protein biomarkers in a control sample (e.g., a normal sample).

In certain embodiments, the biomarkers/analytes of the disclosure may be detected via binding to a suitable binding agent such as ribosome (with or without a peptide component), an RNA molecule, or a polypeptide (e.g., a polypeptide that comprises a polypeptide sequence of a protein marker, a peptide variant thereof, or a non-peptide mimetic of such a sequence). Suitable binding agents also include an antibody specific for a biomarker protein described herein. Suitable antibodies for use in the methods of the present invention include monoclonal and polyclonal antibodies and antigen-binding fragments (e.g., Fab fragments or scFvs) of antibodies. Antibodies, including monoclonal and polyclonal antibodies, fragments and chimeras, may be prepared using methods known in the art. Antibodies to be used in the methods of the invention can be purified by methods well-known in the art. Greenfield (Ed.) (2014) "Antibodies: A Laboratory Manual", Cold Spring Harbor Laboratory: Cold Spring Harbor, N.Y. Antibodies may also be obtained from commercial sources.

The binding agent is directly or indirectly labeled with a detectable moiety. The role of a detectable agent (binding agent labeled with a detectable moiety) is to facilitate the detection step of the diagnostic method by allowing visualization of the complex formed by binding of the binding agent to the protein marker (or fragment thereof). The detectable agent can be selected such that it generates a signal that can be measured and whose intensity is related (preferably proportional) to the amount of protein marker present in the sample being analyzed. Methods for labeling biological molecules, such as polypeptides and antibodies, are well-known in the art. Any of a wide variety of detectable agents can be used in the practice of the present invention. Suitable detectable agents include, but are not limited to: various ligands, radionuclides, fluorescent dyes, chemiluminescent agents, microparticles (e.g., quantum dots, nanocrystals, phosphors), enzymes *(e.g.,* those used in an ELISA, *e.g.,* horseradish peroxidase, beta-galactosidase, luciferase, alkaline phosphatase), colorimetric labels, magnetic labels, and biotin, digoxigenin or other haptens and proteins for which antisera or monoclonal antibodies are available.

The binding agents *(e.g.,* antibodies) may be immobilized on a carrier or support *(e.g.,* a bead, a magnetic particle, a latex particle, a microtiter plate well, a cuvette, or other reaction vessel). Examples of suitable carrier or support materials include agarose, cellulose, nitrocellulose, dextran, Sephadex^{®}, Sepharose^{®}, liposomes, carboxymethyl cellulose, polyacrylamides, polystyrene, gabbros, filter paper, magnetite, ion-exchange resin, plastic film, plastic tube, glass, polyamine-methyl vinyl-ether-maleic acid copolymer, amino acid copolymer, ethylene-maleic acid copolymer, nylon, silk, or combinations thereof. Binding agents may be indirectly immobilized using second binding agents specific for the first binding agents (e.g., mouse antibodies specific for the protein markers may be immobilized using sheep anti-mouse IgG Fc fragment specific antibody coated on the carrier or support).

Protein levels in a biological sample may be determined using immunoassays. Examples of such assays include time resolved fluorescence immunoassays (TR-FIA), radioimmunoassays, enzyme immunoassays (e.g., ELISA), immunofluorescence immunoprecipitation, latex agglutination, hemagglutination, Western blot, and histochemical tests, which are conventional methods well-known in the art. Methods of detection and quantification of the signal generated by the complex formed by binding of the binding agent with the protein marker will depend on the nature of the assay and of the detectable moiety (e.g., fluorescent moiety).

In an example, the presence or amount of protein expression of a gene *(e.g.,* TM or SYND1 or combination thereof) can be determined using a Western blotting technique. For example, a lysate can be prepared from a biological sample, or the biological sample (e.g., biological fluid) itself, can be contacted with Laemmli buffer and subjected to sodium-dodecyl sulfate polyacrylamide gel electrophoresis (SDS-PAGE). SDS-PAGE-resolved proteins, separated by size, can then be transferred to a filter membrane (e.g., nitrocellulose) and subjected to immunoblotting techniques using a detectably-labeled antibody specific to the protein of interest. The presence or amount of bound detectably-labeled antibody indicates the presence or amount of protein in the biological sample.

In an example, an immunoassay can be used for detecting and/or measuring the protein expression of a biomarker protein (e.g., TM or SYND1, Ba, or fragments thereof). As above, for the purposes of detection, an immunoassay can be performed with an antibody that bears a detection moiety (e.g., a fluorescent agent or enzyme). Proteins from a biological sample can be conjugated directly to a solid-phase matrix (e.g., a multi-well assay plate, nitrocellulose, agarose, Sepharose^{®}, encoded particles, or magnetic beads) or it can be conjugated to a first member of a specific binding pair (e.g., biotin or streptavidin) that attaches to a solid-phase matrix upon binding to a second member of the specific binding pair (e.g., streptavidin or biotin). Such attachment to a solid-phase matrix allows the proteins to be purified away from other interfering or irrelevant components of the biological sample prior to contact with the detection antibody and also allows for subsequent washing of unbound antibody. Here, as above, the presence or amount of bound detectably-labeled antibody indicates the presence or amount of protein in the biological sample.

Alternatively, the protein expression levels may be determined using mass spectrometry based methods or image-based methods known in the art for the detection of proteins. Other suitable methods include 2D-gel electrophoresis, proteomics-based methods such as the identification of individual proteins recovered from the gel *(e.g.,* by mass spectrometry or N-terminal sequencing) and/or bioinformatics.

Methods for detecting or measuring protein expression can, optionally, be performed in formats that allow for rapid preparation, processing, and analysis of multiple samples. This can be, for example, in multi-well assay plates *(e.g.,* 96 wells or 386 wells) or arrays *(e.g.,* protein chips). Stock solutions for various reagents can be provided manually or robotically, and subsequent sample preparation, pipetting, diluting, mixing, distribution, washing, incubating (e.g., hybridization), sample readout, data collection (optical data) and/or analysis (computer aided image analysis) can be done robotically using commercially available analysis software, robotics, and detection instrumentation capable of detecting the signal generated from the assay. Examples of such detectors include, but are not limited to, spectrophotometers, luminometers, fluorimeters, and devices that measure radioisotope decay.

### VI. Methods of Treatment

Also provided herein are methods for treating HSCT-TMA in a subject. In one embodiment, a method for treating a patient *(e.g.,* a pediatric or adult patient) having HSCT-TMA who has been determined to have elevated levels *(e.g.,* blood or plasma levels) of a biomarker selected from TM and SYND1 or a combination thereof compared to normal reference ranges of the biomarker is provided, the method comprising administering to the patient an anti-C5 antibody or an anti-CFB antibody in an amount and with a frequency sufficient to attenuate the biomarker levels in the patient, thereby treating HSCT-TMA. In another embodiment, a method for treating a patient (e.g., a pediatric or adult patient) having HSCT-TMA who has been determined to have elevated levels (e.g., blood or plasma levels) of TM, SYND1, and Ba and/or C5b9 and/or HSPG compared to normal reference ranges of TM, SYND1, and Ba and/or C5b9 and/or HSPG respectively, is provided, the method comprising administering to the patient an anti-C5 antibody or an anti-CFB antibody in an amount and with a frequency sufficient to treat HSCT-TMA.

Further provided are methods for treating a patient having HSCT-TMA comprising: (1) obtaining or having obtained a sample (e.g., blood or plasma sample) from the patient, (2) determining or having determined elevated levels of the biomarker selected from TM and SYND1 or a combination thereof in the sample compared to normal reference ranges of the biomarkers and (3) administering to the patient an anti-C5 antibody or an anti-CFB antibody in an amount and with a frequency sufficient to attenuate elevated TM and SYND1 levels in the patient, thereby treating HSCT-TMA. In another embodiment, methods for treating a patient having HSCT-TMA comprising: (1) obtaining or having obtained a sample *(e.g.,* blood or plasma sample) from the patient, (2) determining or having determined elevated levels of biomarkers selected from TM, SYND1, and Ba levels in the sample compared to normal reference ranges of the biomarkers and (3) administering to the patient an anti-C5 antibody or an anti-CFB antibody in an amount and with a frequency sufficient to attenuate TM, SYND 1, and Ba levels, thereby treating HSCT. In another embodiment, methods for treating a patient having HSCT-TMA comprising: (1) obtaining or having obtained a sample *(e.g.,* blood or plasma sample) from the patient, (2) determining or having determined elevated levels of biomarkers selected from TM, SYND1, Ba, and HSPG levels in the sample compared to normal reference ranges of the biomarkers and (3) administering to the patient an anti-C5 antibody or an anti-CFB antibody in an amount and with a frequency sufficient to attenuate TM, SYND1, Ba, HSPG levels, thereby treating HSCT.

As used herein, the term "treating" includes prophylactic and/or therapeutic treatments. The term "prophylactic or therapeutic" treatment is art-recognized and includes administration to a patient one or more of therapeutic agents as described herein (e.g., an anti-C5 antibody (e.g., eculizumab or ravulizumab) or an anti-anti-CFB antibody). If the one or more therapeutic agents are administered prior to clinical manifestation of the unwanted condition *(e.g.,* development of HSCT-TMA) then the treatment is prophylactic, *(i.e.,* it protects the patient against developing the unwanted condition), whereas if it is administered after manifestation of the unwanted condition, the treatment is therapeutic, *(i.e.,* it is intended to diminish, ameliorate, or stabilize the existing unwanted condition or side effects thereof). Preferably, it is intended that the severity of the subject's condition is reduced or at least partially improved or modified and that some alleviation, mitigation, reversal or decrease in at least one clinical symptom is achieved.

As used herein, a patient "in need of prevention," "in need of treatment," or "in need thereof," refers to one, who by the judgment of an appropriate medical practitioner (e.g., a doctor, a nurse, or a nurse practitioner), would reasonably benefit from a given treatment (such as treatment with an anti-C5 antibody or an anti-CFB antibody).

The therapeutic agent or agents (e.g., anti-C5 antibody or an anti-CFB antibody) can be administered to a patient, e.g., a human subject, using a variety of methods that depend, in part, on the route of administration. The route can be, e.g., intravenous injection or infusion (IV), subcutaneous injection (SC), intraperitoneal (IP) injection, or intramuscular injection.

Administration can be achieved by, *e.g.,* local infusion, injection, or by means of an implant. The implant can be of a porous, non-porous, or gelatinous material, including membranes, such as sialastic membranes, or fibers. The implant can be configured for sustained or periodic release of the composition to the subject. See, *e.g.,* U.S. patent publication no. 20080241223; U.S. Patent Nos. 5,501,856; 4,863,457; and 3,710,795; and European Patent Nos. EP488401 and EP430539, the disclosures of each of which are incorporated herein by reference in their entirety. The composition can be delivered to the subject by way of an implantable device based on, e.g., diffusive, erodible or convective systems, e.g., osmotic pumps, biodegradable implants, electrodiffusion systems, electroosmosis systems, vapor pressure pumps, electrolytic pumps, effervescent pumps, piezoelectric pumps, erosion-based systems, or electromechanical systems.

As used herein, the terms "therapeutically effective amount" or "therapeutically effective dose," refer to an amount of an agent (e.g., an anti-C5 antibody (e.g., eculizumab or ravulizumab) or an anti-CFB antibody) that will elicit the desired biological or medical response (e.g., prevention of or an improvement in one or more symptoms of HSCT-TMA). A suitable dose of a therapeutic agent as described herein (e.g., anti-C5 antibody and/or an anti-CFB antibody), which is capable of treating or preventing HSCT-TMA in a subject, can depend on a variety of factors including, e.g., the age, sex, and weight of a subject to be treated and the particular inhibitor compound used. For example, the dose can depend on the severity of the HSCT-TMA. Other factors can include, e.g., other medical disorders concurrently or previously affecting the subject, the general health of the subject, the genetic disposition of the subject, diet, time of administration, rate of excretion, drug combination, and any other additional therapeutics that are administered to the subject. It should also be understood that a specific dosage and treatment regimen for any particular subject depends upon the judgment of the treating medical practitioner (e.g., doctor or nurse). A therapeutically effective amount is also one in which any toxic or detrimental effects of the composition are outweighed by the therapeutically beneficial effects.

The one or more therapeutic agents (e.g., anti-C5 antibody and/or an anti-CFB antibody ) can be administered as a fixed dose, or in a milligram per kilogram "mg/kg" dose. In some embodiments, the dose can also be chosen to reduce or avoid production of antibodies or other host immune responses against one or more active agents in the composition.

While in no way intended to be limiting, exemplary dosages of an inhibitor, such as an anti-C5 antibody, include, e.g., 1-100 mg/kg, 0.5-50 mg/kg, 0.1-100 mg/kg, 0.5-25 mg/kg, 1-20 mg/kg, and 1-10 mg/kg of body weight.

In some embodiments, an anti-C5 antibody *(e.g.,* ravulizumab) is administered *(e.g.,* intravenously):
(a) once on Day 1 at a dose of 600 mg to a patient weighing ≥ 5 to < 10 kg, 600 mg to a patient weighing ≥ 10 to < 20 kg, 900 mg to a patient weighing ≥ 20 to < 30 kg, 1200 mg to a patient weighing ≥ 30 to < 40 kg, 2400 mg to a patient weighing ≥ 40 to < 60 kg, 2700 mg to a patient weighing ≥ 60 to < 100 kg, or 3000 mg to a patient weighing ≥ 100 kg;
(b) once on Day 5 at a dose of 300 mg to a patient weighing ≥ 5 to < 10 kg, 300 mg to a patient weighing ≥ 10 to < 20 kg, 300 mg to a patient weighing ≥ 20 to < 30 kg, 300 mg to a patient weighing ≥ 30 to < 40 kg, 600 mg to a patient weighing ≥ 40 to < 60 kg, 900 mg to a patient weighing ≥ 60 to < 100 kg, or 900 mg to a patient weighing ≥ 100 kg;
(c) once on Day 10 at a dose of 300 mg to a patient weighing ≥ 5 to < 10 kg, 300 mg to a patient weighing ≥ 10 to < 20 kg, 300 mg to a patient weighing ≥ 20 to < 30 kg, 300 mg to a patient weighing ≥ 30 to < 40 kg, 600 mg to a patient weighing ≥ 40 to < 60 kg, 900 mg to a patient weighing ≥ 60 to < 100 kg, or 900 mg to a patient weighing ≥ 100 kg; and
(d) on Day 15 and every four weeks thereafter at a dose of 300 mg to a patient weighing ≥ 5 to < 10 kg or 600 mg to a patient weighing ≥ 10 to < 20 kg; or on Day 15 and every eight weeks thereafter at a dose of 2100 mg to a patient weighing ≥ 20 to < 30 kg, 2700 mg to a patient weighing ≥ 30 to < 40 kg, 3000 mg to a patient weighing ≥ 40 to < 60 kg, 3300 mg to a patient weighing ≥ 60 to < 100 kg, or 3600 mg to a patient weighing ≥ 100 kg.

In some embodiments, a human can be intravenously administered an anti-C5 antibody (e.g., eculizumab) at a dose of about 900 mg about every 12 (e.g., about every 10, 11, 13, 14, 15, 16, 17, 18, 19, 20, 21, 28, 30, 42, or 49 or more) days. See, *e.g.,* Hill et al. (2005) Blood 106(7):2559.

In some embodiments, a human can be intravenously administered an anti-C5 antibody (e.g., eculizumab) at a dose of about 600 (e.g., about 625, 650, 700, 725, 750, 800, 825, 850, 875, 900, 925, 950, or 1,000 or more) mg every week, optionally, for two or more (e.g., three, four, five, six, seven, or eight or more) weeks. Following the initial treatment, the human can be administered the antibody at a dose of about 900 mg about every 14 (e.g., about every 11, 12, 13, 14, 15, 16, 17, 18, 19, 20, 21, 28, 30, 42, or 49 or more) days, *e.g.,* as a maintenance dose. See, e.g., Hillmen et al. (2004) N Engl J Med. 350(6):552-9 and Dmytrijuk et al. (2008) The Oncologist 13(9):993.

In some embodiments, a human can be intravenously administered an anti-C5 antibody *(e.g.,* eculizumab) at a dose of about 900 *(e.g.,* 925, 950, 975, 1000, 1100, or 1200 or more) mg every week, optionally, for two or more (e.g., three, four, five, six, seven, or eight or more) weeks. Following the initial treatment, the human can be administered the antibody at a dose of about 1200 mg about every 14 *(e.g.,* about every 11, 12, 13, 14, 15, 16, 17, 18, 19, 20, 21, 28, 30, 42, or 49 or more) days, *e.g.,* as a maintenance dose. See, for example, International patent Application Publication No. WO 2010/054403.

Toxicity and therapeutic efficacy of such compositions can be determined by known pharmaceutical procedures in cell cultures or experimental animals (animal models of HSCT-TMA). These procedures can be used, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Therapeutic agents that exhibit high therapeutic indices are preferred. While compositions that exhibit toxic side effects may be used, care should be taken to design a delivery system that targets such compounds to the site of affected tissue and to minimize potential damage to normal cells and, thereby, reduce side effects.

In some embodiments, the therapeutic agents described herein *(e.g.,* an anti-C5 antibody or an anti-CFB antibody) are administered to a patient as a monotherapy. Alternatively, the therapeutic agents can be administered to a patient as a combination therapy with another treatment, e.g., another therapeutic agent and/or treatment for HSCT-TMA. For example, the combination therapy can include administering to the patient one or more additional agents that provide a therapeutic benefit to the subject who has, or is at risk of developing, HSCT-TMA. In some embodiments, the agent(s) described herein (*e.g.*, an anti-C5 antibody or an anti-CFB antibody) and one or more additional active agents are administered at the same time. In other embodiments, the agent(s) described herein *(e.g.,* an anti-C5 antibody or an anti-CFB antibody) is administered first in time and the one or more additional active agents are administered second in time.

Methods of treating HSCT-TMA with eculizumab (SOLIRIS^{®}) is described in US Pat. No. 10,815,296 (including corresponding WO/2015/39126), the disclosure in which is incorporated by reference herein. Methods of treating HSCT-TMA with ravulizumab (AXLN1210 or ULTOMIRIS^{®}) is described in WO/2022/36151, the disclosure in which is incorporated by reference herein. Methods of treating HSCT-TMA in patients transfused with red blood cells (RBC) during the maintenance phase of the dosing cycle with ravulizumab (AXLN1210 or ULTOMIRIS^{®}) is described in US Prov. App. No. , entitled "SUPPLEMENTAL DOSAGE AND ADMINISTRATION OF ANTI-C5 ANTIBODIES FOR TREATING HEMATOPOIETIC STEM CELL TRANSPLANT-ASSOCIATED THROMBOTIC MICROANGIOPATHY (HSCT-TMA)" (Attorney Ref. AXJ-306-1), filed: SEP 6, 2022), the disclosure in which is incorporated by reference herein.

### VII. Treatment Outcomes

The efficacy of the treatment methods provided herein can be assessed using any suitable means. In one embodiment, the treatment results in a reduction or cessation in microangiopathic hemolytic anemia, thrombocytopenia, endothelial injury, kidney damage, kidney failure, serositis, pulmonary hypertension, and multisystem organ failure compared to baseline.

In another embodiment, the treatment results in normalization of LDH, resolution of need for red cell and platelet transfusions, an increase in hemoglobin, and/or a disappearance of schistocytes compared to baseline.

In another embodiment, the treatment results in (a) platelet count ≥ 50,000/mm³ without transfusion support during the prior 7 days, (b) LDH <1.5 × ULN, and (c) absence of schistocytes (if there were schistocytes present at baseline), and/or (d) at least 50% reduction of proteinuria from baseline.

In another embodiment, the treatment results in results in a favorable hematological response.

In another embodiment, the treatment results in hemoglobin ≥ 8 g/dL without transfusion support.

In another embodiment, the treatment results in terminal complement inhibition. the treatment results in a reduction in adverse events.

In another embodiment, the treatment results in a change from baseline in quality of life as assessed via a Quality of Life Assessment. In one embodiment, the Quality of Life Assessment is a Quality of Life Inventory (PedsQL) Scale. The Pediatric Quality of Life Inventory (PedsQL) 4.0 Generic Core Scales are multidimensional child self-reports and parent proxy-report standardized instruments to measure health related quality of life (QoL) in children and adolescents 2-18 years of age. In another embodiment, the Quality of Life Assessment is an EuroQoL 5-Dimensions 5-Level (EQ-5D-5L) questionnaire. The EQ-5D-5L is a self-assessed, standardized instrument to measure health related quality of life (QoL) and has been used in a wide range of health conditions.

### VIII. Methods of Identifying Patients

In another aspect, the disclosure provides a method of identifying a patient having HSCT-TMA that is suitable for treatment with an anti-C5 antibody or an anti-CFB antibody, comprising determining levels of a biomarker selected from TM and SYND1 or a combination thereof in a sample (*e.g.,* blood or plasma sample) from the patient using an *in vitro* assay, wherein elevated levels of the biomarker in the sample compared to normal reference ranges of the biomarker identifies the patient as being suitable for treatment with an anti-C5 antibody or an anti-CFB antibody. In another embodiment, a method of identifying a patient having HSCT-TMA that is suitable for treatment with an anti-C5 antibody or an anti-CFB antibody is provided, comprising determining levels of a biomarker selected from TM, SYND1, Ba, and HSPG levels in a sample (*e.g.,* blood or plasma sample) from the patient using an *in vitro* assay, wherein elevated levels of the biomarker in the sample compared to normal reference ranges of the biomarker, identifies the patient as being suitable for treatment with an anti-C5 antibody or an anti-CFB antibody.

In another aspect, the methods include identifying a patient as one having, suspected of having, or at risk for developing HSCT-TMA. In addition to use of the HSCT-TMA biomarker profiling described herein, laboratory tests can be performed to determine whether a human subject has symptoms of HSCT-TMA. For example, thrombocytopenia can be diagnosed by a medical professional as one or more of: (i) a platelet count that is less than 150,000/mm³ (e.g., less than 60,000/mm³); (ii) a reduction in platelet survival time that is reduced, reflecting enhanced platelet disruption in the circulation; and (iii) giant platelets observed in a peripheral smear, which is consistent with secondary activation of thrombocytopoiesis.

As used herein, a patient "at risk for developing HSCT-TMA" is a patient having one or more (*e.g.,* two, three, four, five, six, seven, or eight or more) risk factors for developing the disorder. Risk factors for HSCT-TMA include, *e.g.,* calcineurin inhibitors (CNIs), infections, and conditioning regimens (high dose chemotherapy or total body irradiation) (Khosla, et al., Bone Marrow Transplant. 2018;53(2):129-137; Masias, et al., Blood. 2017;129(21):2857-2863).

As used herein, a patient "suspected of having HSCT-TMA" is a patient who has one or more symptoms of the condition. Symptoms of this condition are well-known to those of skill in the art of medicine and include, *e.g.,* microangiopathic hemolytic anemia, thrombocytopenia, endothelial injury, kidney damage, kidney failure, serositis, pulmonary hypertension, and multisystem organ failure.

### IX. Methods For Monitoring Patient Responsiveness to Treatment

In another aspect, the disclosure provides a method for monitoring responsiveness of a patient having HSCT-TMA to treatment with an anti-C5 antibody or an anti-CFB antibody, the method comprising: determining levels of a biomarker selected from TM and SYND1 or a combination thereof in a sample (*e.g.,* blood or plasma sample) from the patient obtained during or after treatment, wherein: decreased biomarker levels in the sample from the patient obtained during or after treatment, as compared to the biomarker levels in a sample from the patient obtained prior to treatment with the anti-C5 antibody or an anti-CFB antibody, indicates that the patient is responsive to treatment with the anti-C5 antibody or CFB inhibitor. Also provided are methods for monitoring responsiveness of a patient having HSCT-TMA to treatment with an anti-C5 antibody or an anti-CFB antibody, the method comprising: determining levels of a biomarker selected from TM, SYND1, Ba, and HSPG in a sample (*e.g.,* blood or plasma sample) from the patient obtained during or after treatment, wherein: a decreased biomarker level in the sample from the patient obtained during or after treatment, as compared to the biomarker level in a sample from the patient obtained prior to treatment with the anti-C5 antibody or an anti-CFB antibody, indicates that the patient is responsive to treatment with the anti-C5 antibody or an anti-CFB antibody.

Monitoring a patient (*e.g.,* a human patient) for an improvement in HSCT-TMA, as defined herein, means evaluating the subject for a change in a disease parameter, *e.g.,* an improvement in one or more symptoms of the disease. Such symptoms include any of the symptoms of HSCT-TMA described herein. In some embodiments, the evaluation is performed at least 1 hour, e.g., at least 2, 4, 6, 8, 12, 24, or 48 hours, or at least 1 day, 2 days, 4 days, 10 days, 13 days, 20 days or more, or at least 1 week, 2 weeks, 4 weeks, 10 weeks, 13 weeks, 20 weeks or more, after treatment begins. The subject can be evaluated in one or more of the following periods: prior to beginning of treatment; during the treatment; or after one or more elements of the treatment have been administered. Evaluating can include evaluating the need for further treatment, *e.g.,* evaluating whether a dosage, frequency of administration, or duration of treatment should be altered. It can also include evaluating the need to add or drop a selected therapeutic modality, *e.g.,* adding or dropping any of the treatments for HSCT-TMA described herein.

### X. Kits

Also provided are kits that include a pharmaceutical composition containing an anti-C5 antibody or an anti-CFB antibodyin a therapeutically effective amount adapted for use in the methods described herein. In addition, the kits can comprise various reagents and materials useful for carrying out the methods described herein. The procedures for measuring, diagnosing, evaluating, and/or assessing described herein may be performed by diagnostic laboratories, experimental laboratories, or individual practitioners. The invention provides kits which can be used in any or all of these settings.

In some embodiments, the kits described herein comprise materials and reagents for, among other things, characterizing or processing biological samples (*e.g.*, biological fluids), measuring biomarker levels (*e.g.*, protein or nucleic acid levels), diagnosing HSCT-TMA in a subject, or monitoring treatment response in a subject according to the methods provided herein. In certain embodiments, an inventive kit comprises at least one or more reagents that specifically detect protein levels of one or more HSCT-TMA biomarker proteins (*e.g.,* TM, SYND-1, factor Ba, and/or HSPG) and, optionally, instructions for using the kit. The kit can include, *e.g.,* any of the arrays described herein.

Exemplary arrays and chips of the disclosure include antibodies binding to the signatures described previously. In embodiments, such arrays comprise a plurality (*e.g.,* at least 2, 3, or more) antibodies that a capable of detecting the signatures. The antibodies may be monospecific or multi-specific (*e.g.,* binding to more than one biomarker), for example, a bispecific antibody.

In an embodiment, the chips or arrays comprise one or more antibodies for detecting a biomarker signature comprising the following biomarkers: (a) TM + Ba; (b) TM + SYND1; (c) SYND1 + Ba. The chips or arrays may further comprise an antibody for detecting C5b9, in which case, the biomarker signature includes (a) TM + Ba + C5b9; (b) TM + SYND1 + C5b9; (c) SYND1 + Ba + C5b9.

In an embodiment, the chips or arrays comprise one or more antibodies for detecting a biomarker signature comprising the following biomarkers: TM + SYND1 + Ba + HSPG. Such chips or arrays may further comprise an antibody for detecting C5b9, in which case, the biomarker signature includes TM + SYND1 + Ba + C5b9 + HSPG.

In some embodiments, the kits may include suitable control samples (*e.g.,* biological fluids from normal healthy individuals or individuals with HSCT (in the absence of TMA) or a solution comprising a known, control amount of a particular analyte of interest). In some embodiments, kits of the invention may include instructions for using the kit according to one or more methods described herein and may comprise instructions for processing the biological sample (*e.g.,* a biological fluid) obtained from the subject and/or for performing the test or instructions for interpreting the results.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. All publications, patent applications, patents, sequences, database entries (e.g., PUBMED, NCBI or UNIPROT accession numbers), and other references mentioned herein are incorporated by reference in their entirety. In case of conflict, the present specification, including definitions, will control.

While particular embodiments of the present invention have been illustrated and described, it would be obvious to those skilled in the art that various other changes and modifications may be made without departing from the spirit and scope of the invention. It is therefore intended to cover in the appended claims all such changes and modifications that are within the scope of this invention.

The following example is merely illustrative and should not be construed as limiting the scope of this disclosure in any way as many variations and equivalents will become apparent to those skilled in the art upon reading the present disclosure.

### EXAMPLES:

### Example 1: Analysis of the Link Between Excessive AP Activation and Endothelial Damage in the Context of HSCT-TMA

Biomarker levels of soluble TM, SYND1, and Ba were measured in plasma of pediatric HSCT patients with and without (controls) TMA. Biomarkers were also measured in a pre-clinical *in vivo* model of inflammation-mediated complement activation similar to HSCT-TMA, in which C57BL/6J mice were injected with lipopolysaccharide (LPS) to induce complement activation, followed by treatment with AP (anti-fB IgG) and terminal complement pathway (anti-C5 IgG) inhibitors or an IgG isotype control. As the immunosuppressant cyclosporin A (CsA) is a known trigger of HSCT-TMA, the role of complement in CsA-mediated damage in human ECs (HMEC-1 cells and HUVECs) was assessed *in vitro.* ECs were incubated with CsA followed by normal human serum (NHS) in the presence or absence of the C5 complement inhibitor eculizumab (Alexion Pharmaceuticals). TM was then measured by fluorescent staining on the cell surface and quantified using mean fluorescence intensity (MFI).

HSCT-TMA patients (n=11), compared to HSCT control patients (n=7), showed significantly elevated levels of biomarkers of glycocalyx damage (TM: 18.3 ± 10.9 ng/mL and 6.6 ± 2.9 ng/mL, respectively; P<0.01; SYND1: 175.3 ± 177.0 ng/mL and 35.8 ± 18.9, respectively; P<0.05). A strong positive correlation between TM and SYND1 was observed (Pearson r=0.89). Plasma Ba was also significantly elevated in patients with TMA compared to those without (1790.0 ± 1262.0 ng/mL and 652.1 ± 233.5 ng/mL, respectively; P<0.05), and correlated positively with both TM and SYND1 levels (Pearson r=0.72 and 0.50, respectively).
It has previously been shown in an *in vivo* model of inflammation-mediated complement activation that LPS-injected mice, compared to saline-injected control mice, had significantly increased plasma Ba levels. As demonstrated here, LPS-injected mice (which also received IgG isotype control) had higher circulating levels of TM than untreated mice (35.1 ± 5.8 ng/mL [n=6] and 10.9 ± 1.6 ng/mL [n=6], respectively; P<0.0001). Inhibition of alternative (anti-fB IgG) and terminal (anti-C5 IgG) pathways of complement significantly attenuated TM levels (compared to isotype control [35.1 ± 5.8 ng/mL]; anti-FB: 25.2 ± 5.3 ng/mL, P<0.01; anti-C5: 26.2 ± 4.9 ng/mL, P<0.05, **FIG. 1**). TM was positively correlated with Ba (Pearson r=0.68).

*In vitro* treatment of HMEC-1 cells with CsA resulted in deposition of iC3b and C5b-9, and C5 inhibition with eculizumab significantly reduced C5b-9 deposition (P< 0.0001) but had no effect on iC3b deposition. Treatment of HUVECs with CsA also reduced surface expression of TM (P<0.0001), and TM loss was partially restored by eculizumab (P<0.05, **FIG 2**).

In summary, plasma biomarkers of endothelial damage were elevated in patients with HSCT-TMA and in mice with inflammatory-mediated complement activation. Furthermore, AP activation positively correlated with biomarkers of endothelial damage. Blockade of complement activation significantly reduced markers of endothelial damage in both *in vivo* and *in vitro* models. These results provide further evidence linking complement activation and endothelial damage in HSCT-TMA and that the use of these biomarkers aid in the diagnosis of HSCT-TMA.

### Example 2: Complement Activation is Associated with Endothelial Damage in HSCT-TMA

The objective of this study is to explore the link between excessive alternative complement pathway (AP) activation and endothelial damage in the context of HSCT-TMA and to potentially identify biomarkers to aid in its diagnosis.

Levels of soluble TM, SYND1, and Ba were measured in plasma of pediatric HSCT patients with and without TMA. In addition, levels of TM, SYND1, and Ba were measured in a pre-clinical in vivo mouse model of inflammation-mediated complement activation similar to HSCT-TMA. Finally, the role of complement in CsA-mediated damage, TM shedding, and HSPG expression was assessed in human ECs (HMEC-1 cells and HUVECs).

**FIGs. 3A-3C** depict biomarker levels of glyclocalyx components in pediatric patients with and without HSCT-TMA. As shown in these figures, HSCT patients had elevated levels of TM and SYND1. Specifically, **FIGs. 3A and 3B** show that HSCT-TMA patients (n=11), compared to HSCT control patients (n=7), had significantly elevated levels of biomarkers of glycocalyx damage. Mean±SD was calculated, and significance determined with Welch's t-test (*P<0.05; **P<0.01). **FIG. 3C** shows that a strong positive correlation between TM and SYND1 was observed (Pearsons r=0.89).

**FIGs. 4A-4C** depict AP activation in pediatric patients with and without HSCT-TMA. As shown in these figures, HSCT patients had elevated Ba levels which positively correlated with TM and SYND1. Specifically, **FIG. 4A** shows that plasma Ba was significantly elevated in patients with HSCT-TMA compared to those without (Mean±SD was calculated, and significance determined with Welch's t-test. *P<0.05). In addition, plasma Ba correlated positively with both TM **(****FIG. 4B****)** and SYND1 **(****FIG. 4C****)** levels (Pearsons r=0.89 and 0.50, respectively).

**FIG. 5** depicts plasma TM levels in LPS-injected mice treated with anti-complement agents. C57BL/6J mice (12-17 weeks old) were intraperitoneally (IP) injected with LPS (5 mg/kg) to induce complement activation. Three hours after LPS injection, mice were IP injected with IgG isotype control, anti-fB IgG, or anti-C5 IgG (40 mg/kg each). Twenty-four hours later, citrated plasma was collected, and TM levels were measured with a commercially available ELISA (R&D Systems). Untreated mice did not receive LPS or IgG (Blue=male, pink=female). Mean±SD was calculated, and significance determined with ordinary one-way ANOVA (** = P<0.01 **** = P<0.0001). As shown in **FIG. 5****,** mice with inflammation-mediated complement activation had elevated circulating TM levels, which were attenuated with anti-complement agent treatment.

**FIGs. 6A-6B** show the correlation of TM and Ba in LPS-injected mice. C57BL/6J mice (12-17 weeks old) were intraperitoneally **(IP)** injected with LPS (5 mg/kg) to induce complement activation. Three hours after LPS injection, mice were IP injected with IgG isotype control or anti-fB IgG (40 mg/kg each). Twenty-four hours later, citrated plasma was collected, and TM levels and Ba levels were measured with a commercially available ELISA (R&D Systems) or by western blotting, respectively **(****FIG. 6A****;** Blue=male, pink=female). Mean±SD was calculated, and significance determined with Welch's t-test. As shown in **FIG. 6B****,** in mice with inflammation-mediated complement activation, plasma Ba correlated positively with TM levels (Pearsons r=0.68. Grey=LPS+IgG isotype control, Black=LPS+anti-fB IgG).

**FIGs. 7A-7D** depicts cyclosporin-induced TM loss in HUVECs. HUVECs were treated with CsA (28.9µM) and 30% NHS in the presence or absence of an eculizumab surrogate (ecu, 1 µM) for 18h. The cells were then fixed with 4% PFA and incubated with mouse anti-human TM antibody followed by goat anti-mouse AF488 secondary antibody or rabbit anti-human HSPG antibody followed by donkey anti-rabbit AF488 secondary antibody. TM and HSPG expression were imaged and analyzed by CX7 high-content screening platform. MFI±SEM was calculated and significance determined by ordinary one-way ANOVA (*P<0.05 **** = P<0.0001). As shown by these figures, TM and HSPG surface expression was reduced on HUVECs treated with CsA and partially restored by the eculizumab surrogate.

**FIGs.** 8A-8B depict deposition of complement activation products on HMEC-1 cells treated with CsA. HMEC-1 cells were treated with CsA (28.9 µM) for 18 hrs followed by 30 min with 30% NHS in the presence or absence of an eculizumab surrogate (ecu, 1 uM). Cells were fixed with 4% PFA and stained with mouse antibody to iC3b followed by goat anti-mouse AF647 antibody or rabbit anti-human C5b-9 antibody followed by goat anti-rabbit AF647 secondary antibody. Deposition was imaged and analyzed by CX7 high-content screening platform. MFI+SEM was calculated and significance determined by ordinary one-way ANOVA (**** = P<0.0001, ns = not significant). As shown in these figures, CsA treatment induced complement deposition on HMEC-1 cells and C5 inhibition reduced C5b-9 **(****FIG. 8B****),** but not iC3b deposits **(****FIG. 8A****).**

In sum, plasma markers of endothelial damage (TM and SYND1) were elevated in patients with HSCT-TMA and in mice with inflammatory-mediated complement activation. AP activation positively correlated with biomarkers of endothelial damage. Blockade of complement activation significantly reduced markers of endothelial damage in both *in vivo* and *in vitro* models. These results provide further evidence linking complement activation and endothelial damage in HSCT-TMA. Moreover, the use of biomarkers TM, SYND1, and Ba may aid in the diagnosis of HSCT-TMA.

### SEQUENCE SUMMARY

| |
|---|
| **SEQ ID NO:1** |
| GYIFSNYWIQ |
| **SEQ ID NO:2** |
| EILPGSGSTEYTENFKD |
| **SEQ ID NO:3** |
| YFFGSSPNWYFDV |
| **SEQ ID NO:4** |
| GASENIYGALN |
| **SEQ ID NO:5** |
| GATNLAD |
| **SEQ ID NO:6** |
| QNVLNTPLT |
| **SEQ ID NO:7** |
| |
| **SEQ ID NO:8** |
| |
| **SEQ ID NO:9** |
| ASTKGPSVFP LAPCSRSTSE STAALGCLVK DYFPEPVTVS WNSGALTSGV HTFPAVLQSS GLYSLSSVVT VPSSNFGTQT YTCNVDHKPS NTKVDKTVER KCCVECPPCP APPVAGPSVF LFPPKPKDTL MISRTPEVTC VVVDVSQEDP EVQFNWYVDG VEVHNAKTKP REEQFNSTYR VVSVLTVLHQ DWLNGKEYKC KVSNKGLPSS IEKTISKAKG QPREPQVYTL PPSQEEMTKN QVSLTCLVKG FYPSDIAVEW ESNGQPENNY KTTPPVLDSD GSFFLYSRLT VDKSRWQEGN VFSCSVMHEA LHNHYTQKSL SLSLGK |
| **SEQ ID NO:10** |
| QVQLVQSGAE VKKPGASVKV SCKASGYIFS NYWIQWVRQA PGQGLEWMGE ILPGSGSTEY TENFKDRVTM TRDTSTSTVY MELSSLRSED TAVYYCARYF FGSSPNWYFD VWGQGTLVTV SSASTKGPSV FPLAPCSRST SESTAALGCL VKDYFPEPVT VSWNSGALTS GVHTFPAVLQ SSGLYSLSSV VTVPSSNFGT QTYTCNVDHK PSNTKVDKTV ERKCCVECPP CPAPPVAGPS VFLFPPKPKD TLMISRTPEV TCVVVDVSQE DPEVQFNWYV DGVEVHNAKT KPREEQFNST YRVVSVLTVL HQDWLNGKEY KCKVSNKGLP SSIEKTISKA KGQPREPQVY TLPPSQEEMT NQVSLTCLVK GFYPSDIAVE WESNGQPENN YKTTPPVLDS DGSFFLYSRL TVDKSRWQEG NVFSCSVMHE ALHNHYTQKS LSLSLGK |
| **SEQ ID NO:11** |
| |
| **SEQ ID NO:12** |
| |
| |
| **SEQ ID NO:13** |
| ASTKGPSVFP LAPCSRSTSE STAALGCLVK DYFPEPVTVS WNSGALTSGV HTFPAVLQSS GLYSLSSVVT VPSSNFGTQT YTCNVDHKPS NTKVDKTVER KCCVECPPCP APPVAGPSVF LFPPKPKDTL MISRTPEVTC VVVDVSQEDP EVQFNWYVDG VEVHNAKTKP REEQFNSTYR VVSVLTVLHQ DWLNGKEYKC KVSNKGLPSS IEKTISKAKG QPREPQVYTL PPSQEEMTKN QVSLTCLVKG FYPSDIAVEW ESNGQPENNY KTTPPVLDSD GSFFLYSRLT VDKSRWQEGN VFSCSVLHEA LHSHYTQKSL SLSLGK |
| **SEQ ID NO:14** |
| QVQLVQSGAE VKKPGASVKV SCKASGHIFS NYWIQWVRQA PGQGLEWMGE ILPGSGHTEY TENFKDRVTM TRDTSTSTVY MELSSLRSED TAVYYCARYF FGSSPNWYFD VWGQGTLVTV SSASTKGPSV FPLAPCSRST SESTAALGCL VKDYFPEPVT VSWNSGALTS GVHTFPAVLQ SSGLYSLSSV VTVPSSNFGT QTYTCNVDHK PSNTKVDKTV ERKCCVECPP CPAPPVAGPS VFLFPPKPKD TLMISRTPEV TCVVVDVSQE DPEVQFNWYV DGVEVHNAKT KPREEQFNST YRVVSVLTVL HQDWLNGKEY KCKVSNKGLP SSIEKTISKA KGQPREPQVY TLPPSQEEMT KNQVSLTCLV KGFYPSDIAV EWESNGQPEN NYKTTPPVLD SDGSFFLYSR LTVDKSRWQE GNVFSCSVLH EALHSHYTQK SLSLSLGK |
| **SEQ ID NO:15** |
| ASTKGPSVFP LAPCSRSTSE STAALGCLVK DYFPEPVTVS WNSGALTSGV HTFPAVLQSS GLYSLSSVVT VTSSNFGTQT YTCNVDHKPS NTKVDKTVER KCCVECPPCP APPVAGPSVF LFPPKPKDTL YITREPEVTC VVVDVSHEDP EVQFNWYVDG MEVHNAKTKP REEQFNSTFR VVSVLTVVHQ DWLNGKEYKC KVSNKGLPAP IEKTISKTKG QPREPQVYTL PPSREEMTKN QVSLTCLVKG FYPSDIAVEW ESNGQPENNY KTTPPMLDSD GSFFLYSKLT VDKSRWQQGN VFSCSVMHEA LHNHYTQKSL SLSPGK |
| **SEQ ID NO:16** |
| QVQLVQSGAE VKKPGASVKV SCKASGYIFS NYWIQWVRQA PGQGLEWMGE ILPGSGSTEY TENFKDRVTM TRDTSTSTVY MELSSLRSED TAVYYCARYF FGSSPNWYFD VWGQGTLVTV SSASTKGPSV FPLAPCSRST SESTAALGCL VKDYFPEPVT VSWNSGALTS GVHTFPAVLQ SSGLYSLSSV VTVTSSNFGT QTYTCNVDHK PSNTKVDKTV ERKCCVECPP CPAPPVAGPS VFLFPPKPKD TLYITREPEV TCVVVDVSHE DPEVQFNWYV DGMEVHNAKT KPREEQFNST FRVVSVLTVV HQDWLNGKEY KCKVSNKGLP APIEKTISKT KGQPREPQVY TLPPSREEMT KNQVSLTCLV KGFYPSDIAV EWESNGQPEN NYKTTPPMLD SDGSFFLYSK LTVDKSRWQQ GNVFSCSVMH EALHNHYTQK SLSLSPGK |
| **SEQ ID NO:17** |
| GASENIYHALN |
| **SEQ ID NO:18** |
| EILPGSGHTEYTENFKD |
| **SEQ ID NO:19** |
| GHIFSNYWIQ |
| **SEQ ID NO:20** |
| QVQLVQSGAE VKKPGASVKV SCKASGHIFS NYWIQWVRQA PGQGLEWMGE ILPGSGHTEY TENFKDRVTM TRDTSTSTVY MELSSLRSED TAVYYCARYF FGSSPNWYFD VWGQGTLVTV SSASTKGPSV FPLAPCSRST SESTAALGCL VKDYFPEPVT VSWNSGALTS GVHTFPAVLQ SSGLYSLSSV VTVPSSNFGT QTYTCNVDHK PSNTKVDKTV ERKCCVECPP CPAPPVAGPS VFLFPPKPKD |
| TLMISRTPEV TCVVVDVSQE DPEVQFNWYV DGVEVHNAKT KPREEQFNST YRVVSVLTVL HQDWLNGKEY KCKVSNKGLP SSIEKTISKA KGQPREPQVY TLPPSQEEMT KNQVSLTCLV KGFYPSDIAV EWESNGQPEN NYKTTPPVLD SDGSFFLYSR LTVDKSRWQE GNVFSCSVMH EALHNHYTQK SLSLSLGK |
| **SEQ ID NO:21** |
| SYAIS |
| **SEQ ID NO:22** |
| GIGPFFGTANYAQKFQG |
| **SEQ ID NO:23** |
| DTPYFDY |
| **SEQ ID NO:24** |
| SGDSIPNYYVY |
| **SEQ ID NO:25** |
| DDSNRPS |
| **SEQ ID NO:26** |
| QSFDSSLNAEV |
| **SEQ ID NO:27** |
| QVQLVQSGAE VKKPGSSVKV SCKASGGTFS SYAISWVRQA PGQGLEWMGG IGPFFGTANY AQKFQGRVTI TADESTSTAY MELSSLRSED TAVYYCARDT PYFDYWGQGT LVTVSS |
| **SEQ ID NO:28** |
| DIELTQPPSV SVAPGQTARI SCSGDSIPNY YVYWYQQKPG QAPVLVIYDD SNRPSGIPER FSGSNSGNTA TLTISGTQAE DEADYYCQSF DSSLNAEVFG GGTKLTVL |
| **SEQ ID NO:29** |
| NYIS |
| **SEQ ID NO:30** |
| IIDPDDSYTEYSPSFQG |
| **SEQ ID NO:31** |
| YEYGGFDI |
| **SEQ ID NO:32** |
| SGDNIGNSYVH |
| **SEQ ID NO:33** |
| KDNDRPS |
| **SEQ ID NO:34** |
| GTYDIESYV |
| **SEQ ID NO:35** |
| EVQLVQSGAE VKKPGESLKI SCKGSGYSFT NYISWVRQMP GKGLEWMGII DPDDSYTEYS PSFQGQVTIS ADKSISTAYL QWSSLKASDT AMYYCARYEY GGFDIWGQGT LVTVSS |
| **SEQ ID NO:36** |
| SYELTQPPSV SVAPGQTARI SCSGDNIGNS YVHWYQQKPG QAPVLVIYKD NDRPSGIPER FSGSNSGNTA TLTISGTQAE DEADYYCGTY DIESYVFGGG TKLTVL |
| **SEQ ID NO:37** |
| SSYYVA |
| **SEQ ID NO:38** |
| AIYTGSGATYKASWAKG |
| **SEQ ID NO:39** |
| DGGYDYPTHAMHY |
| **SEQ ID NO:40** |
| QASQNIGSSLA |
| **SEQ ID NO:41** |
| GASKTHS |
| **SEQ ID NO:42** |
| QSTKVGS SYGNH |
| **SEQ ID NO:43** |
| QVQLVESGGG LVQPGGSLRL SCAASGFTSH SSYYVAWVRQ APGKGLEWVG AIYTGSGATY KASWAKGRFT ISKDTSKNQV VLTMTNMDPV DTATYYCASD GGYDYPTHAM HYWGQGTLVT VSS |
| **SEQ ID NO:44** |
| DVVMTQSPSS LSASVGDRVT ITCQASQNIG SSLAWYQQKP GQAPRLLIYG ASKTHSGVPS RFSGSGSGTD FTLTISSLQP EDVATYYCQS TKVGSSYGNH FGGGTKVEIK |
| **SEQ ID NO:45** |
| QVQLVESGGG LVQPGRSLRL SCAASGFTVH SSYYMAWVRQ APGKGLEWVG AIFTGSGAEY KAEWAKGRVT ISKDTSKNQV VLTMTNMDPV DTATYYCASD AGYDYPTHAM HYWGQGTLVT VSSASTKGPS VFPLAPSSKS TSGGTAALGC LVKDYFPEPV TVSWNSGALT SGVHTFPAVL QSSGLYSLSS VVTVPSSSLG TQTYICNVNH KPSNTKVDKK VEPKSCDKTH TCPPCPAPEL RRGPKVFLFP PKPKDTLMIS RTPEVTCVVV DVSHEDPEVK FNWYVDGVEV HNAKTKPREE QYNSTYRVVS VLTVLHQDWL NGKEYKCKVS NKGLPSSIEK TISKAKGQPR EPQVYTLPPS REEMTKNQVS LTCLVKGFYP SDIAVEWESN GQPENNYKTT PPVLDSDGSF FLYSKLTVDK SRWQQGNVFS CSVLHEALHA HYTRKELSLS P |
| **SEQ ID NO:46** |
| DIQMTQSPSS LSASVGDRVT ITCRASQGIS SSLAWYQQKP GKAPKLLIYG ASETESGVPS RFSGSGSGTD FTLTISSLQP EDFATYYCQN TKVGSSYGNT FGGGTKVEIK RTVAAPSVFI FPPSDEQLKS GTASVVCLLN NFYPREAKVQ WKVDNALQSG NSQESVTEQD SKDSTYSLSS TLTLSKADYE KHKVYACEVT HQGLSSPVTK SFNRGEC |
| **SEQ ID NO:47** |
| QVQLQESGPGLVKPSETLSLTCTVSGDSVSSSYWTWIRQPPGKGLEWIGYIYYSGSSN YNPSLKSRATISVDTSKNQFSLKLSSVTAADTAVYYCAREGNVDTTMIFDYWGQGTLV TVSS |
| **SEO ID NO:48** |
| AIQMTQSPSSLSASVGDRVTITCRASQGIRNDLGWYQQKPGKAPKLLIYAASSLQSGVP SRFAGRGSGTDFTLTISSLQPEDFATYYCLQDFNYPWTFGQGTKVEIK |
| **SEQ ID NO:49** |
| QVQLQESGPGLVKPSETLSLTCTVSGDSVSSSYWTWIRQPPGKGLEWIGYIYYSGSSNY NPSLKSRATISVDTSKNQFSLKLSSVTAADTAVYYCAREGNVDTTMIFDYWGQGTLVTV SSASTKGPSVFPLAPCSRSTSESTAALGCLVKDYFPEPVTVSWNSGALTSGVHTFPAVL QSSGLYSLSSVVTVPSSSLGTKTYTCNVDHKPSNTKVDKRVESKYGPPCPPCPAPEFLG GPSVFLFPPKPKDTLMISRTPEVTCVVVDVSQEDPEVQFNWYVDGVEVHNAKTKPREEQ FNSTYRVVSVLTVLHQDWLNGKEYKCKVSNKGLPSSIEKTISKAKGQPREPQVYTLPPS QEEMTKNQVSLTCLVKGFYPSDIAVEWESNGQPENNYKTTPPVLDSDGSFFLYSRLTVD |
| KSRWQEGNVFSCSVMHEALHNHYTQKSLSLSLGK |
| **SEQ ID NO:50** |
| AIQMTQSPSSLSASVGDRVTITCRASQGIRNDLGWYQQKPGKAPKLLIYAASSLQSGVP SRFAGRGSGTDFTLTISSLQPEDFATYYCLQDFNYPWTFGQGTKVEIKRTVAAPSVFIF PPSDEQLKSGTASVVCLLNNFYPREAKVQWKVDNALQSGNSQESVTEQDSKDSTYSLSS TLTLSKADYEKHKVYACEVTHQGLSSPVTKSFNRGEC |

### ASPECTS

The following are numbered aspects of the invention
1. A method for treating a patient having hematopoietic stem cell transplant-associated thrombotic microangiopathy (HSCT-TMA) who has been determined to have elevated blood levels of a biomarker selected from thrombomodulin (TM) and syndecan-1 (SYND1), or a combination thereof compared to normal reference ranges of the biomarker, the method comprising administering to the patient an anti-C5 antibody or an anti-complement factor B (CFB) antibody in an amount and with a frequency sufficient to attenuate the biomarker levels in the patient, thereby treating HSCT-TMA.
2. The method of aspect 1, wherein the patient has further been determined to have an elevated blood level of Ba and/or C5b9 compared to a normal reference range for complement factor Ba and/or C5b9.
3. The method of aspect 1 or 2, wherein the patient has further been determined to have an elevated blood level of heparan sulfate proteoglycan (HSPG) compared to a normal reference range for HSPG.
4. A method for treating a patient having HSCT-TMA comprising:
   (1) obtaining or having obtained a blood sample from the patient,
   (2) determining or having determined elevated levels of biomarker selected from TM and SYND1 or a combination thereof in the blood sample of the patient compared to normal reference ranges of the biomarkers, and
   (3) administering to the patient an anti-C5 antibody or anti-CFB antibody in an amount and with a frequency sufficient to attenuate elevated TM and SYND1 levels in the patient, thereby treating HSCT-TMA.
5. The method of aspect 4, further comprising determining or having determined elevated Ba and/or C5b9 levels in the blood sample compared to a normal reference range for Ba and/or C5b9.
6. The method of aspect 5, further comprising determining or having determined elevated HSPG levels in the blood sample compared to a normal reference range for HSPG.
7. A method of identifying a patient having HSCT-TMA that is suitable for treatment with an anti-C5 antibody or anti-CFB antibody, comprising determining levels of a biomarker selected from TM and SYND1, or a combination thereof, in a blood sample from the patient using an *in vitro* assay, wherein elevated levels of the biomarker in the blood sample compared to normal reference ranges of TM and SYND1, respectively, identifies the patient as being suitable for treatment with an anti-C5 antibody or anti-CFB antibody.
8. The method of aspect 7, further comprising determining Ba and/or C5b9 levels in the blood sample, wherein an elevated level of Ba and/or C5b9 compared to a normal reference range for Ba and/or C5b9 identifies the patient as being suitable for treatment with an anti-C5 antibody or anti-CFB antibody.
9. The method of aspect 8, further comprising determining a HSPG level in the blood sample, wherein an elevated level HSPG compared to a normal reference range for HSPG identifies the patient as being suitable for treatment with an anti-C5 antibody or anti-CFB antibody.
10. A method for monitoring responsiveness of a patient having HSCT-TMA to treatment with an anti-C5 antibody or anti-CFB antibody, the method comprising: determining a biomarker selected from TM and SYND1 or a combination thereof in a blood sample from the patient obtained during or after treatment, wherein: decreased biomarker levels in the blood sample from the patient obtained during or after treatment, as compared to the biomarker levels in a blood sample from the patient obtained prior to treatment with the anti-C5 antibody or anti-CFB antibody, indicates that the patient is responsive to treatment with the anti-C5 antibody or anti-CFB antibody.
11. The method of aspect 10, further comprising determining Ba and/or C5b9 levels in the blood sample, from the patient obtained during or after treatment, wherein: decreased Ba and/or C5b9 levels in the blood sample from the patient obtained during or after treatment, as compared to the Ba levels in a blood sample from the patient obtained prior to treatment with the anti-C5 antibody or anti-CFB antibody, indicates that the patient is responsive to treatment with the anti-C5 antibody or anti-CFB antibody.
12. The method of aspect 11, further comprising determining a HSPG level in the blood sample, from the patient obtained during or after treatment, wherein: a decreased HSPG level in the blood sample from the patient obtained during or after treatment, as compared to the HSPG level in a blood sample from the patient obtained prior to treatment with the anti-C5 antibody or anti-CFB antibody, indicates that the patient is responsive to treatment with the anti-C5 antibody or anti-CFB antibody.
13. The method of any of the preceding aspects, wherein the blood sample is plasma.
14. The method of any of the preceding aspects, wherein the level or levels are measured by a test system or kit that has received regulatory (e.g., USFDA) approval.
15. The method of any of the preceding aspects, wherein the level or levels are measured by using an immunoassay, immunochemistry, immunohistochemistry assay, nucleoprobe assay, in situ hybridization, fluorescent RNA probes, RT-PCR, microarray transcription assay, or RNA transcription assay.
16. The method of any of the preceding aspects, wherein the normal reference range for TM for a healthy patient is about 1.8 ng/mL to about 4.8 ng/mL.
17. The method of any of the preceding aspects, wherein the normal reference range for TM for a HSCT patient in the absence of TMA is about 3 ng/mL to about 9 ng/mL.
18. The method of any of the preceding aspects, wherein the elevated TM level is greater than about 10 ng/mL, 11 ng/mL, 12 ng/mL, 13 ng/mL, 14 ng/mL, 15 ng/mL, 16 ng/mL, 17 ng/mL, 18 ng/mL, 19 ng/mL, 20 ng/mL, 21 ng/mL, 22 ng/mL, 23 ng/mL, 24 ng/mL, 25 ng/mL, 26 ng/mL, 27 ng/mL, 28 ng/mL, 29 ng/mL, or 30 ng/mL.
19. The method of any of the preceding aspects, wherein the normal reference range for SYND1 for a healthy patient is about 15 ng/mL to 55 ng/mL.
20. The method of any of the preceding aspects, wherein the normal reference range for SYND1 for a HSCT patient in the absence of TMA is about 15 ng/mL to 55 ng/mL.
21. The method of any of the preceding aspects, wherein the elevated level for SYND1 is greater than about 100 ng/mL, 105 ng/mL, 110 ng/mL, 115 ng/mL, 120 ng/mL, 125 ng/mL, 130 ng/mL, 135 ng/mL, 140 ng/mL, 145 ng/mL, 150 ng/mL, 155 ng/mL, 160 ng/mL, 165 ng/mL, 170 ng/mL, 175 ng/mL, 180 ng/mL, 185 ng/mL, 190 ng/mL, 195 ng/ml, 200 mg/mL, 205 ng/mL, 210 ng/mL, 215 ng/mL, 220 ng/mL, 225 ng/mL, 230 ng/mL, 235 ng/mL, 240 ng/mL, 245 ng/mL, or 250 ng/mL.
22. The method of any of the preceding aspects, wherein the normal reference range for Ba for a healthy patient is between about 300 ng/mL and 600 ng/mL.
23. The method of any of the preceding aspects, wherein the normal reference range for Ba for a HSCT patient in the absence of TMA is about 500 ng/mL to 800 ng/mL.
24. The method of any of the preceding aspects, wherein the elevated level for Ba is greater than about 900 ng/mL, 910 ng/mL, 920 ng/mL, 930 ng/mL, 940 ng/mL, 950 ng/mL, 960 ng/mL, 970 ng/mL, 980 ng/mL, 990 ng/mL, 1000 ng/mL, 1010 ng/mL, 1020 ng/mL, 1030 ng/mL, 1040 ng/mL, 1050 ng/mL, 1060 ng/mL, 1070 ng/mL, 1080 ng/mL, 1090 ng/mL, 1100 ng/mL, 1110 ng/mL, 1120 ng/mL, 1130 ng/mL, 1140 ng/mL, 1150 ng/mL, 1160 ng/mL, 1170 ng/mL, 1180 ng/mL, 1190 ng/mL, 1200 ng/mL, 1210 ng/mL, 1220 ng/mL, 1230 ng/mL, 1240 ng/mL, 1250 ng/mL, 1260 ng/mL, 1270 ng/mL, 1280 ng/mL, 1290 ng/mL, 1300 ng/mL, 1310 ng/mL, 1320 ng/mL, 1330 ng/mL, 1340 ng/mL, 1350 ng/mL, 1360 ng/mL, 1370 ng/mL, 1380 ng/mL, 1390 ng/mL, 1400 ng/mL, 1410 ng/mL, 1420 ng/mL, 1430 ng/mL, 1440 ng/mL, 1450 ng/mL, 1460 ng/mL, 1470 ng/mL, 1480 ng/mL, 1490 ng/mL, 1500 ng/mL, 1510 ng/mL, 1520 ng/mL, 1530 ng/mL, 1540 ng/mL, 1550 ng/mL, 1560 ng/mL, 1570 ng/mL, 1580 ng/mL, 1590 ng/mL, 1600 ng/mL, 1610 ng/mL, 1620 ng/mL, 1630 ng/mL, 1640 ng/mL, 1650 ng/mL, 1660 ng/mL, 1670 ng/mL, 1680 ng/mL, 1690 ng/mL, 1700 ng/mL, 1710 ng/mL, 1720 ng/mL, 1730 ng/mL, 1740 ng/mL, 1750 ng/mL, 1760 ng/mL, 1770 ng/mL, 1780 ng/mL, 1790 ng/mL, 1800 ng/mL, 1810 ng/mL, 1820 ng/mL, 1830 ng/mL, 1840 ng/mL, 1850 ng/mL, 1860 ng/mL, 1870 ng/mL, 1880 ng/mL, 1890 ng/mL, 1900 ng/mL, 1910 ng/mL, 1920 ng/mL, 1930 ng/mL, 1940 ng/mL, 1950 ng/mL, 1960 ng/mL, 1970 ng/mL, 1980 ng/mL, 1990 ng/mL, 2000 ng/mL, 2010 ng/mL, 2020 ng/mL, 2030 ng/mL, 2040 ng/mL, 2050 ng/mL, 2060 ng/mL, 2070 ng/mL, 2080 ng/mL, 2090 ng/mL, 2100 ng/mL, 2110 ng/mL, 2120 ng/mL, 2130 ng/mL, 2140 ng/mL, 2150 ng/mL, 2160 ng/mL, 2170 ng/mL, 2180 ng/mL, 2190 ng/mL, 2200 ng/mL, 2210 ng/mL, 2220 ng/mL, 2230 ng/mL, 2240 ng/mL, 2250 ng/mL, 2260 ng/mL, 2270 ng/mL, 2280 ng/mL, 2290 ng/mL, 2300 ng/mL, 2310 ng/mL, 2320 ng/mL, 2330 ng/mL, 2340 ng/mL, 2350 ng/mL, 2360 ng/mL, 2370 ng/mL, 2380 ng/mL, 2390 ng/mL, 2400 ng/mL, 2410 ng/mL, 2420 ng/mL, 2430 ng/mL, 2440 ng/mL, 2450 ng/mL, 2460 ng/mL, 2470 ng/mL, 2480 ng/mL, 2490 ng/mL, or 2500 ng/mL.
25. The method of any one of the preceding aspects, wherein the anti-C5 antibody is a human antibody, a humanized antibody, a bispecific antibody, a chimeric antibody, a Fab, a Fab'2, a ScFv, a SMIP, an Affibody^{®}, a nanobody, or a domain antibody which inhibits C5.
26. The method of any one of the preceding aspects, wherein the anti-C5 antibody comprises CDR1, CDR2, and CDR3 heavy chain sequences as set forth in SEQ ID NOs:1, 2, and 3, respectively, and CDR1, CDR2, and CDR3 light chain sequences as set forth in SEQ ID NOs:4, 5, and 6, respectively.
27. The method of any one of the preceding aspects, wherein the anti-C5 antibody comprises a heavy chain variable region comprising SEQ ID NO:7 and a light chain variable region comprising SEQ ID NO:8.
28. The method of any one of the preceding aspects, wherein the anti-C5 antibody comprises a heavy chain comprising SEQ ID NO:10 and a light chain comprising SEQ ID NO:11.
29. The method of any one of the preceding aspects, wherein the anti-C5 antibody is SOLIRIS^{®}.
30. The method of any one of aspects 1-23, wherein the anti-C5 antibody comprises CDR1, CDR2, and CDR3 heavy chain sequences as set forth in SEQ ID NOs:19, 18, and 3, respectively, and CDR1, CDR2, and CDR3 light chain sequences as set forth in SEQ ID NOs:4, 5, and 6, respectively.
31. The method of aspect 30, wherein the anti-C5 antibody further comprises a variant human Fc constant region that binds to human neonatal Fc receptor (FcRn), wherein the variant human Fc CH3 constant region comprises Met-429-Leu and Asn-435-Ser substitutions at residues corresponding to methionine 428 and asparagine 434 of a native human IgG Fc constant region, each in EU numbering.
32. The method of aspect 30 or 31, wherein the anti-C5 antibody comprises a heavy chain variable region comprising SEQ ID NO:12 and a light chain variable region comprising SEQ ID NO:8.
33. The method of any one of aspects 30-32, wherein the anti-C5 antibody further comprises a heavy chain constant region depicted in SEQ ID NO:13.
34. The method of any one of aspects 30-33, wherein the anti-C5 antibody comprises a heavy chain polypeptide comprising the amino acid sequence depicted in SEQ ID NO:14 and a light chain polypeptide comprising the amino acid sequence depicted in SEQ ID NO:11.
35. The method of any one of aspects 30-34, wherein the anti-C5 antibody is ULTOMIRIS^{®}.
36. The method of any of the preceding aspects, wherein the anti-C5 antibody is administered intravenously.
37. The method of any one of aspects 1-29, wherein the anti-C5 antibody is administered:
   (a) once on Day 1 at a dose of 600 mg to a patient weighing ≥ 5 to < 10 kg, 600 mg to a patient weighing ≥ 10 to < 20 kg, 900 mg to a patient weighing ≥ 20 to < 30 kg, 1200 mg to a patient weighing ≥ 30 to < 40 kg, 2400 mg to a patient weighing ≥ 40 to < 60 kg, 2700 mg to a patient weighing ≥ 60 to < 100 kg, or 3000 mg to a patient weighing ≥ 100 kg;
   (b) once on Day 5 at a dose of 300 mg to a patient weighing ≥ 5 to < 10 kg, 300 mg to a patient weighing ≥ 10 to < 20 kg, 300 mg to a patient weighing ≥ 20 to < 30 kg, 300 mg to a patient weighing ≥ 30 to < 40 kg, 600 mg to a patient weighing ≥ 40 to < 60 kg, 900 mg to a patient weighing ≥ 60 to < 100 kg, or 900 mg to a patient weighing ≥ 100 kg;
   (c) once on Day 10 at a dose of 300 mg to a patient weighing ≥ 5 to < 10 kg, 300 mg to a patient weighing ≥ 10 to < 20 kg, 300 mg to a patient weighing ≥ 20 to < 30 kg, 300 mg to a patient weighing ≥ 30 to < 40 kg, 600 mg to a patient weighing ≥ 40 to < 60 kg, 900 mg to a patient weighing ≥ 60 to < 100 kg, or 900 mg to a patient weighing ≥ 100 kg; and
   (d) on Day 15 and every four weeks thereafter at a dose of 300 mg to a patient weighing ≥ 5 to < 10 kg or 600 mg to a patient weighing ≥ 10 to < 20 kg; or on Day 15 and every eight weeks thereafter at a dose of 2100 mg to a patient weighing ≥ 20 to < 30 kg, 2700 mg to a patient weighing ≥ 30 to < 40 kg, 3000 mg to a patient weighing ≥ 40 to < 60 kg, 3300 mg to a patient weighing ≥ 60 to < 100 kg, or 3600 mg to a patient weighing ≥ 100 kg.
38. The method of any one of the preceding aspects, wherein the treatment results in a reduction or cessation in microangiopathic hemolytic anemia, thrombocytopenia, endothelial injury, kidney damage, kidney failure, serositis, pulmonary hypertension, and multisystem organ failure compared to baseline.
39. The method of any one of the preceding aspects, wherein the treatment results in normalization of LDH, resolution of need for red cell and platelet transfusions, an increase in hemoglobin, and/or a disappearance of schistocytes compared to baseline.
40. The method of any one of the preceding aspects, wherein the treatment results in (a) platelet count ≥ 50,000/mm³ without transfusion support during the prior 7 days, (b) LDH <1.5 × ULN, and (c) absence of schistocytes (if there were schistocytes present at baseline), and/or (d) at least 50% reduction of proteinuria from baseline.
41. The method of any one of the preceding aspects, wherein the treatment results in results in a favorable hematological response.
42. The method of any one of the preceding aspects, wherein the treatment results in hemoglobin ≥ 8 g/dL without transfusion support.
43. The method of any one of the preceding aspects, wherein the treatment results in terminal complement inhibition.
44. The method of any one of the preceding aspects, wherein the treatment results in a reduction in adverse events.
45. The method of any one of the preceding aspects, wherein the treatment results in a change from baseline in quality of life as assessed via a Quality of Life Assessment.
46. The method of any one of the preceding aspects, wherein the Quality of Life Assessment is a Quality of Life Inventory (PedsQL) Scale or an EQ-5D-5L questionnaire).
47. The method of any one of the preceding aspects, wherein the patient is a pediatric patient.
48. The method of any one of the preceding aspects, wherein the patient is an adult patient.
49. An anti-C5 antibody, or antigen-binding fragment thereof, or an anti- complement factor B (CFB) antibody for use in treatment of a patient having hematopoietic stem cell transplant-associated thrombotic microangiopathy (HSCT-TMA) who has been determined to have elevated blood levels of a biomarker selected from thrombomodulin (TM) and syndecan-1 (SYND1), or a combination thereof compared to normal reference ranges of the biomarker, wherein the anti-C5 antibody or an anti-complement factor B (CFB) antibody is administered to the patient in an amount and with a frequency sufficient to attenuate the biomarker levels in the patient.
50. An anti-C5 antibody, or antigen-binding fragment thereof, or an anti-complement factor B (CFB) antibody for use in identifying a patient having HSCT-TMA that is suitable for treatment with an anti-C5 antibody or an anti-CFB antibody , comprising determining levels of a biomarker selected from TM and SYND1, or a combination thereof, in a blood sample from the patient using an *in vitro* assay, wherein elevated levels of the biomarker in the blood sample compared to normal reference ranges of TM and SYND1, respectively, identifies the patient as being suitable for treatment with an anti-C5 antibody or an anti-CFB antibody .
51. An anti-C5 antibody, or antigen-binding fragment thereof, or an anti-CFB antibody for use in monitoring responsiveness of a patient having HSCT-TMA to treatment with an anti-C5 antibody or an anti-CFB antibody , wherein the use comprises: determining a biomarker selected from TM and SYND1 or a combination thereof in a blood sample from the patient obtained during or after treatment, wherein: decreased biomarker levels in the blood sample from the patient obtained during or after treatment, as compared to the biomarker levels in a blood sample from the patient obtained prior to treatment with the anti-C5 antibody or an anti-CFB antibody , indicates that the patient is responsive to treatment with the anti-C5 antibody or an anti-CFB antibody .
52. Use of an anti-C5, antibody or antigen-binding fragment thereof, or an anti-CFB antibody for treatment of a patient having hematopoietic stem cell transplant-associated thrombotic microangiopathy (HSCT-TMA) who has been determined to have elevated blood levels of a biomarker selected from thrombomodulin (TM) and syndecan-1 (SYND1), or a combination thereof compared to normal reference ranges of the biomarker, wherein the anti-C5 antibody or an anti-CFB antibody is administered to the patient in an amount and with a frequency sufficient to attenuate the biomarker levels in the patient.
53. Use of an anti-C5 antibody, or antigen-binding fragment thereof, or an anti-CFB antibody in identifying a patient having HSCT-TMA that is suitable for treatment with an anti-C5 antibody or an anti-CFB antibody , comprising determining levels of a biomarker selected from TM and SYND1, or a combination thereof, in a blood sample from the patient using an *in vitro* assay, wherein elevated levels of the biomarker in the blood sample compared to normal reference ranges of TM and SYND1, respectively, identifies the patient as being suitable for treatment with an anti-C5 antibody or an anti-CFB antibody.
54. Use of an anti-C5 antibody, or antigen-binding fragment thereof, or an anti-CFB antibody in monitoring responsiveness of a patient having HSCT-TMA to treatment with an anti-C5 antibody or an anti-CFB antibody , comprising: determining a biomarker selected from TM and SYND1 or a combination thereof in a blood sample from the patient obtained during or after treatment, wherein: decreased biomarker levels in the blood sample from the patient obtained during or after treatment, as compared to the biomarker levels in a blood sample from the patient obtained prior to treatment with the anti-C5 antibody or an anti-CFB antibody , indicates that the patient is responsive to treatment with the anti-C5 antibody or an anti-CFB antibody .

## Claims

1. An anti-C5 antibody or an anti-complement factor B (CFB) antibody for use in a method for treating a patient having hematopoietic stem cell transplant-associated thrombotic microangiopathy (HSCT-TMA) who has been determined to have elevated blood levels of a biomarker selected from thrombomodulin (TM) and syndecan-1 (SYND1), or a combination thereof compared to normal reference ranges of the biomarker, the method comprising administering to the patient the anti-C5 antibody or the anti-complement factor B (CFB) antibody in an amount and with a frequency sufficient to attenuate the biomarker levels in the patient, thereby treating HSCT-TMA.

2. The anti-C5 antibody or anti-complement factor B (CFB) antibody for use according to claim 1, wherein the patient has further been determined to have an elevated blood level of Ba and/or C5b9 compared to a normal reference range for complement factor Ba and/or C5b9, and/or wherein the patient has further been determined to have an elevated blood level of heparan sulfate proteoglycan (HSPG) compared to a normal reference range for HSPG.

3. A method of identifying a patient having HSCT-TMA that is suitable for treatment with an anti-C5 antibody or anti-CFB antibody, comprising determining levels of a biomarker selected from TM and SYND1, or a combination thereof, in a blood sample from the patient using an *in vitro* assay, wherein elevated levels of the biomarker in the blood sample compared to normal reference ranges of TM and SYND1, respectively, identifies the patient as being suitable for treatment with an anti-C5 antibody or anti-CFB antibody, optionally wherein the method further comprises determining Ba and/or C5b9 levels in the blood sample, wherein an elevated level of Ba and/or C5b9 compared to a normal reference range for Ba and/or C5b9 identifies the patient as being suitable for treatment with an anti-C5 antibody or anti-CFB antibody, optionally further comprising determining a HSPG level in the blood sample, wherein an elevated level HSPG compared to a normal reference range for HSPG identifies the patient as being suitable for treatment with an anti-C5 antibody or anti-CFB antibody.

4. A method for monitoring responsiveness of a patient having HSCT-TMA to treatment with an anti-C5 antibody or anti-CFB antibody, the method comprising: determining a biomarker selected from TM and SYND1 or a combination thereof in a blood sample from the patient obtained during or after treatment, wherein: decreased biomarker levels in the blood sample from the patient obtained during or after treatment, as compared to the biomarker levels in a blood sample from the patient obtained prior to treatment with the anti-C5 antibody or anti-CFB antibody, indicates that the patient is responsive to treatment with the anti-C5 antibody or anti-CFB antibody, optionally wherein the method further comprises determining Ba and/or C5b9 levels in the blood sample, from the patient obtained during or after treatment, wherein: decreased Ba and/or C5b9 levels in the blood sample from the patient obtained during or after treatment, as compared to the Ba levels in a blood sample from the patient obtained prior to treatment with the anti-C5 antibody or anti-CFB antibody, indicates that the patient is responsive to treatment with the anti-C5 antibody or anti-CFB antibody, optionally further comprising determining a HSPG level in the blood sample, from the patient obtained during or after treatment, wherein: a decreased HSPG level in the blood sample from the patient obtained during or after treatment, as compared to the HSPG level in a blood sample from the patient obtained prior to treatment with the anti-C5 antibody or anti-CFB antibody, indicates that the patient is responsive to treatment with the anti-C5 antibody or anti-CFB antibody.

5. The anti-C5 antibody or anti-complement factor B (CFB) antibody for use according to claim 1 or 2 or the method of claim 3 or 4, wherein the blood sample is plasma.

6. The anti-C5 antibody or anti-complement factor B (CFB) antibody for use according to any one of claims 1, 2 and 5 or the method of any one of claims 3 to 5, wherein the level or levels are measured by:
(a) a test system or kit that has received regulatory (e.g., USFDA) approval; and/or
(b) using an immunoassay, immunochemistry, immunohistochemistry assay, nucleoprobe assay, in situ hybridization, fluorescent RNA probes, RT-PCR, microarray transcription assay, or RNA transcription assay.

7. The anti-C5 antibody or anti-complement factor B (CFB) antibody for use according to any one of claims 1, 2, 5 and 6 or the method of any one of claims 3 to 6, wherein:
(a) the normal reference range for TM for a healthy patient is about 1.8 ng/mL to about 4.8 ng/mL;
(b) the normal reference range for TM for a HSCT patient in the absence of TMA is about 3 ng/mL to about 9 ng/m;
(c) the elevated TM level is greater than about 10 ng/mL, 11 ng/mL, 12 ng/mL, 13 ng/mL, 14 ng/mL, 15 ng/mL, 16 ng/mL, 17 ng/mL, 18 ng/mL, 19 ng/mL, 20 ng/mL, 21 ng/mL, 22 ng/mL, 23 ng/mL, 24 ng/mL, 25 ng/mL, 26 ng/mL, 27 ng/mL, 28 ng/mL, 29 ng/mL, or 30 ng/mL;
(d) the normal reference range for SYND1 for a healthy patient is about 15 ng/mL to 55 ng/mL;
(e) the normal reference range for SYND1 for a HSCT patient in the absence of TMA is about 15 ng/mL to 55 ng/mL; and/or
(f) the elevated level for SYND1 is greater than about 100 ng/mL, 105 ng/mL, 110 ng/mL, 115 ng/mL, 120 ng/mL, 125 ng/mL, 130 ng/mL, 135 ng/mL, 140 ng/mL, 145 ng/mL, 150 ng/mL, 155 ng/mL, 160 ng/mL, 165 ng/mL, 170 ng/mL, 175 ng/mL, 180 ng/mL, 185 ng/mL, 190 ng/mL, 195 ng/ml, 200 mg/mL, 205 ng/mL, 210 ng/mL, 215 ng/mL, 220 ng/mL, 225 ng/mL, 230 ng/mL, 235 ng/mL, 240 ng/mL, 245 ng/mL, or 250 ng/mL.

8. The anti-C5 antibody or anti-complement factor B (CFB) antibody for use according to any one of claims 2 and 5 to 7 or the method of any one of claims 3 to 7, wherein the normal reference range for Ba for:
(a) a healthy patient is between about 300 ng/mL and 600 ng/mL; and/or
(b) a HSCT patient in the absence of TMA is about 500 ng/mL to 800 ng/mL.

9. The anti-C5 antibody or anti-complement factor B (CFB) antibody for use according to any one of claims 2 and 5 to 8 or the method of any one of claims 3 to 8, wherein the elevated level for Ba is greater than about 900 ng/mL, 910 ng/mL, 920 ng/mL, 930 ng/mL, 940 ng/mL, 950 ng/mL, 960 ng/mL, 970 ng/mL, 980 ng/mL, 990 ng/mL, 1000 ng/mL, 1010 ng/mL, 1020 ng/mL, 1030 ng/mL, 1040 ng/mL, 1050 ng/mL, 1060 ng/mL, 1070 ng/mL, 1080 ng/mL, 1090 ng/mL, 1100 ng/mL, 1110 ng/mL, 1120 ng/mL, 1130 ng/mL, 1140 ng/mL, 1150 ng/mL, 1160 ng/mL, 1170 ng/mL, 1180 ng/mL, 1190 ng/mL, 1200 ng/mL, 1210 ng/mL, 1220 ng/mL, 1230 ng/mL, 1240 ng/mL, 1250 ng/mL, 1260 ng/mL, 1270 ng/mL, 1280 ng/mL, 1290 ng/mL, 1300 ng/mL, 1310 ng/mL, 1320 ng/mL, 1330 ng/mL, 1340 ng/mL, 1350 ng/mL, 1360 ng/mL, 1370 ng/mL, 1380 ng/mL, 1390 ng/mL, 1400 ng/mL, 1410 ng/mL, 1420 ng/mL, 1430 ng/mL, 1440 ng/mL, 1450 ng/mL, 1460 ng/mL, 1470 ng/mL, 1480 ng/mL, 1490 ng/mL, 1500 ng/mL, 1510 ng/mL, 1520 ng/mL, 1530 ng/mL, 1540 ng/mL, 1550 ng/mL, 1560 ng/mL, 1570 ng/mL, 1580 ng/mL, 1590 ng/mL, 1600 ng/mL, 1610 ng/mL, 1620 ng/mL, 1630 ng/mL, 1640 ng/mL, 1650 ng/mL, 1660 ng/mL, 1670 ng/mL, 1680 ng/mL, 1690 ng/mL, 1700 ng/mL, 1710 ng/mL, 1720 ng/mL, 1730 ng/mL, 1740 ng/mL, 1750 ng/mL, 1760 ng/mL, 1770 ng/mL, 1780 ng/mL, 1790 ng/mL, 1800 ng/mL, 1810 ng/mL, 1820 ng/mL, 1830 ng/mL, 1840 ng/mL, 1850 ng/mL, 1860 ng/mL, 1870 ng/mL, 1880 ng/mL, 1890 ng/mL, 1900 ng/mL, 1910 ng/mL, 1920 ng/mL, 1930 ng/mL, 1940 ng/mL, 1950 ng/mL, 1960 ng/mL, 1970 ng/mL, 1980 ng/mL, 1990 ng/mL, 2000 ng/mL, 2010 ng/mL, 2020 ng/mL, 2030 ng/mL, 2040 ng/mL, 2050 ng/mL, 2060 ng/mL, 2070 ng/mL, 2080 ng/mL, 2090 ng/mL, 2100 ng/mL, 2110 ng/mL, 2120 ng/mL, 2130 ng/mL, 2140 ng/mL, 2150 ng/mL, 2160 ng/mL, 2170 ng/mL, 2180 ng/mL, 2190 ng/mL, 2200 ng/mL, 2210 ng/mL, 2220 ng/mL, 2230 ng/mL, 2240 ng/mL, 2250 ng/mL, 2260 ng/mL, 2270 ng/mL, 2280 ng/mL, 2290 ng/mL, 2300 ng/mL, 2310 ng/mL, 2320 ng/mL, 2330 ng/mL, 2340 ng/mL, 2350 ng/mL, 2360 ng/mL, 2370 ng/mL, 2380 ng/mL, 2390 ng/mL, 2400 ng/mL, 2410 ng/mL, 2420 ng/mL, 2430 ng/mL, 2440 ng/mL, 2450 ng/mL, 2460 ng/mL, 2470 ng/mL, 2480 ng/mL, 2490 ng/mL, or 2500 ng/mL.

10. The anti-C5 antibody or anti-complement factor B (CFB) antibody for use according to any one of claims 1, 2 and 5 to 9 or the method of any one of claims 3 to 9, wherein the anti-C5 antibody:
(a) is a human antibody, a humanized antibody, a bispecific antibody, a chimeric antibody, a Fab, a Fab'2, a ScFv, a SMIP, an Affibody^{®}, a nanobody, or a domain antibody which inhibits C5;
(b) comprises CDR1, CDR2, and CDR3 heavy chain sequences as set forth in SEQ ID NOs:1, 2, and 3, respectively, and CDR1, CDR2, and CDR3 light chain sequences as set forth in SEQ ID NOs:4, 5, and 6, respectively;
(c) comprises a heavy chain variable region comprising SEQ ID NO:7 and a light chain variable region comprising SEQ ID NO:8;
(d) comprises a heavy chain comprising SEQ ID NO:10 and a light chain comprising SEQ ID NO:11; and/or
(e) is SOLIRIS^{®}.

11. The anti-C5 antibody or anti-complement factor B (CFB) antibody for use according to any one of claims 1, 2 and 5 to 8 or method of any one of claims 3 to 8, wherein the anti-C5 antibody comprises CDR1, CDR2, and CDR3 heavy chain sequences as set forth in SEQ ID NOs:19, 18, and 3, respectively, and CDR1, CDR2, and CDR3 light chain sequences as set forth in SEQ ID NOs:4, 5, and 6, respectively, optionally wherein:
(a) the anti-C5 antibody further comprises a variant human Fc constant region that binds to human neonatal Fc receptor (FcRn), wherein the variant human Fc CH3 constant region comprises Met-429-Leu and Asn-435-Ser substitutions at residues corresponding to methionine 428 and asparagine 434 of a native human IgG Fc constant region, each in EU numbering;
(b) the anti-C5 antibody comprises a heavy chain variable region comprising SEQ ID NO:12 and a light chain variable region comprising SEQ ID NO:8;
(c) the anti-C5 antibody further comprises a heavy chain constant region depicted in SEQ ID NO:13;
(d) the anti-C5 antibody comprises a heavy chain polypeptide comprising the amino acid sequence depicted in SEQ ID NO:14 and a light chain polypeptide comprising the amino acid sequence depicted in SEQ ID NO:11; and/or
(e) the anti-C5 antibody is ULTOMIRIS^{®}.

12. The anti-C5 antibody or anti-complement factor B (CFB) antibody for use according to any one of claims 1, 2 and 5 to 11, wherein the anti-C5 antibody is administered intravenously.

13. The anti-C5 antibody or anti-complement factor B (CFB) antibody for use according to any one of claims 1, 2 and 5 to 10, wherein the anti-C5 antibody is administered:
(a) once on Day 1 at a dose of 600 mg to a patient weighing ≥ 5 to < 10 kg, 600 mg to a patient weighing ≥ 10 to < 20 kg, 900 mg to a patient weighing ≥ 20 to < 30 kg, 1200 mg to a patient weighing ≥ 30 to < 40 kg, 2400 mg to a patient weighing ≥ 40 to < 60 kg, 2700 mg to a patient weighing ≥ 60 to < 100 kg, or 3000 mg to a patient weighing ≥ 100 kg;
(b) once on Day 5 at a dose of 300 mg to a patient weighing ≥ 5 to < 10 kg, 300 mg to a patient weighing ≥ 10 to < 20 kg, 300 mg to a patient weighing ≥ 20 to < 30 kg, 300 mg to a patient weighing ≥ 30 to < 40 kg, 600 mg to a patient weighing ≥ 40 to < 60 kg, 900 mg to a patient weighing ≥ 60 to < 100 kg, or 900 mg to a patient weighing ≥ 100 kg;
(c) once on Day 10 at a dose of 300 mg to a patient weighing ≥ 5 to < 10 kg, 300 mg to a patient weighing ≥ 10 to < 20 kg, 300 mg to a patient weighing ≥ 20 to < 30 kg, 300 mg to a patient weighing ≥ 30 to < 40 kg, 600 mg to a patient weighing ≥ 40 to < 60 kg, 900 mg to a patient weighing ≥ 60 to < 100 kg, or 900 mg to a patient weighing ≥ 100 kg; and
(d) on Day 15 and every four weeks thereafter at a dose of 300 mg to a patient weighing ≥ 5 to < 10 kg or 600 mg to a patient weighing ≥ 10 to < 20 kg; or on Day 15 and every eight weeks thereafter at a dose of 2100 mg to a patient weighing ≥ 20 to < 30 kg, 2700 mg to a patient weighing ≥ 30 to < 40 kg, 3000 mg to a patient weighing ≥ 40 to < 60 kg, 3300 mg to a patient weighing ≥ 60 to < 100 kg, or 3600 mg to a patient weighing ≥ 100 kg.

14. The anti-C5 antibody or anti-complement factor B (CFB) antibody for use according to any one of claims 1, 2 and 5 to 13, wherein the treatment results in:
(a) a reduction or cessation in microangiopathic hemolytic anemia, thrombocytopenia, endothelial injury, kidney damage, kidney failure, serositis, pulmonary hypertension, and multisystem organ failure compared to baseline;
(b) normalization of LDH, resolution of need for red cell and platelet transfusions, an increase in hemoglobin, and/or a disappearance of schistocytes compared to baseline;
(c) platelet count ≥ 50,000/mm³ without transfusion support during the prior 7 days, LDH <1.5 × ULN, and absence of schistocytes (if there were schistocytes present at baseline), and/or at least 50% reduction of proteinuria from baseline;
(d) a favorable hematological response;
(e) hemoglobin ≥ 8 g/dL without transfusion support;
(f) terminal complement inhibition;
(g) a reduction in adverse events; and/or
(h) a change from baseline in quality of life as assessed via a Quality of Life Assessment, optionally wherein the Quality of Life Assessment is a Quality of Life Inventory (PedsQL) Scale or an EQ-5D-5L questionnaire).

15. The anti-C5 antibody or anti-complement factor B (CFB) antibody for use according to any one of claims 1, 2 and 5 to 14, wherein the patient is a pediatric patient, or the patient is an adult patient.
